# EUROPÄISCHE PATENTSCHRIFT

(11) **EP 1 257 265 B1**
(45) Veröffentlichungstag und Bekanntmachung des Hinweises auf die Patenterteilung: **21.01.2004**
(21) Anmeldenummer: 01915288.3
(22) Anmeldetag: 22.02.2001
(51) Int. Cl.: A61K 31/135, A61P 33/00

(54) **VERWENDUNG VON 2-PHENYLENDIAMINDERIVATEN ZUR BEHANDLUNG VON INFEKTIONEN**
USE OF 2-PHENYLENE DIAMINE DERIVATIVES FOR THE TREATMENT OF INFECTIONS
UTILISATION DE DERIVES DE 2-PHENYLENE DIAMINE POUR LE TRAITEMENT D'INFECTIONS

(30) Priorität: 24.02.2000 DE 10008522
(43) Veröffentlichungstag der Anmeldung: 20.11.2002
(73) Patentinhaber: BioAgency AG, 22525 Hamburg (DE)
(72) Erfinder: JOMAA, Hassan, 35392 Giessen (DE); SCHLITZER, Martin, 35287 Amöneburg-Rossdorf (DE); WIESNER, Jochen, 35394 Giessen (DE)
(74) Vertreter: Dreiss, Fuhlendorf, Steimle & Becker
(86) Internationale Anmeldenummer: PCT/EP2001/002016
(87) Internationale Veröffentlichungsnummer: WO 2001/062709

(56) Entgegenhaltungen:
- WO-A-95/35287

## Beschreibung

Die vorliegende Erfindung betrifft die Verwendung von 2-Phenylendiaminderivaten zur Herstellung von Arzneimitteln für die prophylaktische und therapeutische Behandlung von Infektionen. Sie betrifft insbesondere die Herstellung von Arzneimitteln für die Behandlung von Infektionen, die durch Parasiten hervorgerufen wurden.

Malaria ist eine der Hauptursachen für die hohe Sterblichkeitsrate in tropischen Gebieten mit 300 bis 500 Millionen klinischen Fällen und 1,5 bis 2,5 Millionen Todesfällen pro Jahr. Da die Resistenz der Parasiten gegenüber herkömmlichen Antimalariamitteln alarmierend ansteigt, werden dringend neue Mittel benötigt.

Aufgabe der vorliegenden Erfindung ist es daher, neue Mittel zur Bekämpfung von parasitären Infektionen, insbesondere Malaria bereitzustellen.

Diese Aufgabe wird in völlig überraschender Weise durch die in Anspruch 1 definierte Stoffgruppe der 2-Phenylendiaminderivate gelöst. Diese Stoffgruppe zeigt nicht nur eine antiinfektiöse Wirkung gegen ein- und mehrzellige Parasiten, sondern ebenfalls gegen Viren, Bakterien und Pilze. Im Sinne der vorliegenden Erfindung sind unter einzelligen Parasiten entsprechend der engen Definition der Parasitologie Protozoen zu verstehen.

Die erfindungsgemäß verwendeten Verbindungen sind bereits für die Krebstherapie vorgeschlagen worden, wie es in der gleichzeitig anhängigen Anmeldung desselben Anmelders offenbart ist.

Die 2-Phenylendiaminderivate entsprechen der allgemeinen Formel (I): 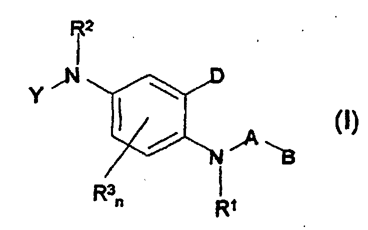 worin
n = 0 - 3;
R¹, R² = H, C₁₋₂₆-Alkyl, Aryl, Heteroaryl, C₁₋₂₆-Acyl;
R³ = H, Halogen, C₁₋₂₆-Alkyl, Aryl, Heteroaryl, Ar-C₁₋₂₆-alkyl, C₁₋₂₆-Acyl, CN, NO₂, R⁴-X-;
mit R⁴ = H, C₁₋₂₆-Alkyl, Aryl, Heteroaryl, Ar- C₁₋₂₆-alkyl, C₁₋₂₆-Acyl;
X = NH, O, S, SO₂, NHSO₂, OSO₂,
A = CH₂, CHR⁵, CR⁵R⁶, CO, CS, CONR⁴, CSNR⁴, SO₂, PO₂,
R⁵, R⁶ = unabhängig voneinander C₁₋₂₆-Alkyl, Aryl, Heteroaryl, Ar- C₁₋₂₆-alkyl, CN, NO₂, COR⁷,
R⁷ = H, C₁₋₂₆-Alkyl, Aryl, Ar- C₁₋₂₆-alkyl, C₁₋₂₆-Alkoxy, Aryloxy, Ar- C₁₋₂₆-alkoxy, NR⁸R⁹,
R⁸, R⁹ = unabhängig voneinander H, C₁₋₂₆-Alkyl, Aryl, Ar- C₁₋₂₆-alkyl, Heteroaryl,
B = C₁₋₂₆-Alkyl, Aryl, Heteroaryl, Arylcarbonyl, gesättigte Heterocyclen, substituiertes Alkyl mit 1-4 Kettenglieder, wobei als Substituenten C₁₋₉-Alkyl, Aryl, Heteroaryl, Halogen, =O, OH, NH₂, NH-CO-R¹⁰, NH-SO₂-R¹⁰, COOR¹¹, CO-NR¹²R¹³, CS-NR¹⁴R¹⁵, SO₂OR¹⁶, SO₂NR¹⁷R¹⁸, NH-CO-OR¹⁹, NH-CO-NR²⁰R²¹, NHCSNR²²R²³ auftreten können,
R¹⁰ - R²³ = unabhängig voneinander H, C₁₋₂₆-Alkyl, Aryl, Ar- C₁₋₂₆-alkyl, Heteroaryl,
D = H, C₁₋₂₆-Alkyl, Aryl, Heteroaryl, Ar- C₁₋₂₆-alkyl, -Y-R²⁴, Halogen, NO₂, CN, NH-CO-R²⁵, NH-SO₂-R²⁶ , NH-CO-OR²⁷, NH-CO-NR²⁸R²⁹, NH-CS-NR³⁰R³¹,
Y= O, NH, S, CO, CS, SO₂, COO, CONR³¹, CSNR³², SO₂NR³³,
R²⁴ - R³³ = unabhängig voneinander H, C₁₋₂₆-Alkyl, Aryl, Heteroaryl, Ar- C₁₋₂₆-alkyl bedeuten, und
Y = eine Gruppe ausgewählt aus 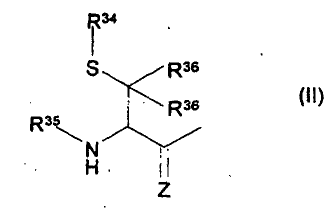 worin
Z = O, S oder zwei Wasserstoffatome,
R³⁴ = H, C₁₋₂₆-Alkyl, Aryl, Ar- C₁₋₂₆-alkyl, COOR³⁷, Arylsulfonyl,
R³⁵ = H, C₁₋₂₆-Acyl, COOR³⁸,
R³⁶ = unabhängig voneinander H, C₁₋₂₆-Alkyl,
R³⁷, R³⁸ = unabhängig voneinander C₁₋₂₆-Alkyl, Aryl, Ar- C₁₋₂₆-alkyl bedeuten,
oder 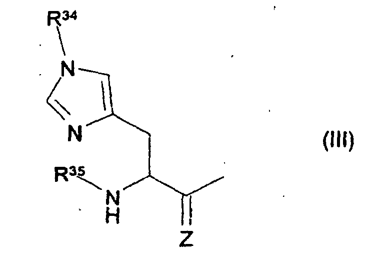 worin
Z = O, S oder zwei Wasserstoffatome,
R³⁴ = H, C₁₋₂₆-Alkyl, Aryl, Ar- C₁₋₂₆-alkyl, COOR³⁷, Arylsulfonyl,
R³⁵ = H, C₁₋₂₆-Acyl, COOR³⁸
R³⁷, R³⁸ = unabhängig voneinander C₁₋₂₆-Alkyl, Aryl, Ar- C₁₋₂₆-alkyl bedeuten,
oder 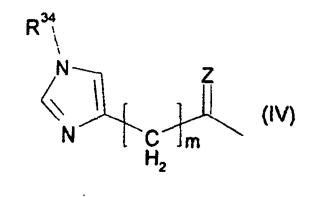 worin
Z = O, S oder zwei Wasserstoffatome,
m=0-3
R³⁴ = H, C₁₋₂₆-Alkyl, Aryl, Ar- C₁₋₂₆-alkyl, COOR³⁷, Arylsulfonyl,
R³⁷ = C₁₋₂₆-Alkyl, Aryl, Ar- C₁₋₂₆-alkyl bedeuten,
oder 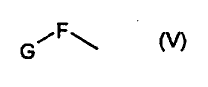 worin
F = CH₂, CO, CS, SO₂,
G = COOR³⁹, CONHOH, CONR⁴⁰R⁴¹, CSNR⁴²R⁴³, C₁₋₂₆-alkyl- oder arylsubstituiertes Alkyl mit 1-3 Kettengliedern oder Alkyl mit 1-3 Kettengliedern, das am terminalen C-Atom einen Substituenten ausgewählt aus COOR⁴⁴, CONHOH, CONR⁴⁵R⁴⁶, CSNR⁴⁷R⁴⁸, SR⁴⁹, SOR⁵⁰, SO₂R⁵¹, SO₂NR⁵²R⁵³, PO(OR⁵⁴)OR⁵⁵, PO(OR⁵⁶)NR⁵⁷₂, OSO₂R⁵⁸, O(PO)OR⁵⁹, NHSO₂R⁶⁰, NHPO₂R⁶¹, NHCOR⁶², NHCSR⁶³, NHCONR⁶⁴R⁶⁵, NHCSNR⁶⁶R⁶⁷, -S(NH)₂-R⁶⁸ oder NH(C=NR⁷⁰)NHR⁶⁹ trägt, ferner Aryl oder Heteroaryl,
R³⁹ - R⁶⁹ = unabhängig voneinander H, C₁₋₂₆-Alkyl, Aryl,
R⁷⁰ = CONH₂, SO₂NH₂ bedeuten,
oder 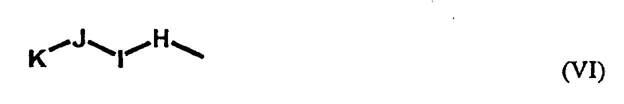 ist, worin
H = CH₂, CO, CS, CHR⁷¹, CR⁷²R⁷³, SO₂, SO, PO₂,
I = C₁₋₂₆-Alkylen, C₁₋₂₆-Alkylen, bei dem eine Methylengruppe durch O, S, oder NR⁷⁷ ersetzt ist, oder C₂₋₂₆-Alkenylen, wobei diese Alkylen- bzw. Alkenylenreste unsubstituiert oder durch Aryl, Heteroaryl, Halogen, OH, CN, C₁₋₉-Alkyloxy, Aryloxy, COOR⁷⁴, CONR⁷⁵R⁷⁶, NR⁷⁷R⁷⁸, NH(C=NR⁷⁰)NHR⁶⁹, SR⁷⁹, SO₂R⁸⁰ substituiert sind, ferner C₃₋₈-Cycloalkylen, unsubstituiert
oder durch Aryl, Heteroaryl, Halogen, OH, CN, C₁₋₉-Alkyloxy, Aryloxy, COOR⁷⁴,
CONR⁷⁵R⁷⁶, NR⁷⁷R⁷⁸, SR⁷⁹, SO₂R⁸⁰ substituiert, C₃₋₈-Cycloalkylen, worin die Alkylenkette durch O, S oder NR⁷⁷ unterbrochen ist, Arylen, unsubstituiert oder durch Aryl, Heteroaryl, Halogen, OH, CN, C₁₋₂₆-Alkoxy, Aryloxy, COOR⁷⁴, CONR⁷⁵R⁷⁶, NR⁷⁷R⁷⁸, SR⁷⁹, SO₂R⁸⁰ sub stituiert,
R⁷¹ - R⁸⁰ unabhängig voneinander H, C₁₋₂₆-Alkyl, Aryl, Ar- C₁₋₂₆-alkyl, Heteroaryl,
J = eine Bindung oder CO, COO, CONR⁸¹, CS, CSNR⁸², SO₂, S(NH)₂, SO(NH), SO₂O, SO₂NR⁸³, PO(OR⁸⁴), PO(OR⁸⁵)NR⁸⁶, NR⁸⁷CO, NR⁸⁸CS, NR⁸⁹SO₂, OSO₂, NR⁹⁰PO(OR⁸⁴), OPO(OR⁹¹), PO(OR⁸⁴)O, NR⁹²CONR⁹³, NR⁹⁴CSNR⁹⁵, NR⁹⁶SO₂NR⁹⁷, NR⁹⁸C(NR⁹⁹)NR¹⁰⁰,
R⁸¹-R¹⁰⁰ = unabhängig voneinander H, C₁₋₂₆-Alkyl, Aryl, Ar- C₁₋₂₆-alkyl, Heteroaryl,
R⁹⁹ = H, C₁₋₂₆-Alkyl, Aryl, Ar- C₁₋₂₆-alkyl, Heteroaryl, CONR¹⁰¹R¹⁰², CSNR¹⁰³R¹⁰⁴, SO₂NR¹⁰⁵R¹⁰⁶
R¹⁰¹ - R¹⁰⁶ = unabhängig voneinander H, C₁₋₂₆-Alkyl, Aryl, Ar- C₁₋₂₆-alkyl, Heteroaryl,
K = verzweigtes oder unverzweigtes C₁₁₋₂₃-Alkyl, verzweigtes oder unverzweigtes C₁₁₋₂₃-Alkenyl, welches unsubstituiert oder durch Aryl oder Heteroaryl substituiert ist, C₁₁₋₂₃-Alkinyl, Aryl, Heteroaryl, Ar- C₁₋₂₆-alkyl, wobei Aryl, Heteroaryl und Ar- C₁₋₂₆-alkyl mit weiteren Aryl-, Heteroaryl- und/oder Ar- C₁₋₂₆-alkylresten substituiert sein können, bedeuten und Salze davon.

Unter den Verbindungen, die durch ihre Wirkung besonders aufgefallen sind, gelten die folgenden Definitionen:
n = 0 - 3,
R¹, R² und R³ = H,
A = CO, SO₂,
B = Phenyl, Benzyl, Phenethyl, 4-Chlorphenylmethyl, 4-Bromphenylmethyl, 4-Nitrophenylmethyl, 4-Trifluormethylphenylmethyl, 4-Methylphenylmethyl, 4-Phenylphenyl-methyl, 1-Naphthylmethyl, 2-Naphthylmethyl, Benzoyl, 2,4,4-Trimethylpentyl, 2-Carboxyethyl, 3-Carboxypropyl, 1-(4-Methyl-1-piperazinyl)-1-(4-trifluormethylphenyl)-methyl, 1-(4-Methyl-1-piperazinyl)-1-phenylmethyl,
D = Benzoyl,
Y =
ein Rest der Formel (II),
worin
Z = O,
R³⁴ = H, Benzyloxycarbonyl, Trityl,
R³⁵ = H, Benzyloxycarbonyl, tert.-Butyloxycarbonyl,
R³⁶ = H,
oder ein Rest der Formel (III),
worin
Z = O,
R³⁴ = H, Tosyl, Benzyl, 4-Nitrobenzyl, 4-Cyanobenzyl, Benzyloxycarbonyl,
R³⁵ = H, Benzyloxycarbonyl, tert.-Butyloxycarbonyl,
oder ein Rest der Formel (IV),
worin
Z = O,
m = 0 - 3,
R³⁴ = H, Tosyl, Benzyl, 4-Nitrobenzyl, 4-Cyanobenzyl, Benzyloxycarbonyl,
oder ein Rest der Formel (V),
worin
F = CO, CH₂, SO₂,
G = COOH, COOMe, CONHOH, CH₂-COOH, CH₂COOMe, CH₂CONHOH, CH₂CONH-(C₁₄-C₂₀)-Alkyl, CH₂CH₂COOH, CH₂CH₂COOMe, CH₂CH₂CONHOH, CH₂CH₂CONH-(C₁₄-C₂₀)-Alkyl, CH₂CH₂CH₂COOH, CH₂CH₂CH₂COOMe, CH₂CH₂CH₂CONHOH, CH₂CH₂CH₂CONH-(C₁₄-C₂₀)-Alkyl, Phenyl, Naphthyl, Pyridyl, Fluorenyl, Anthracenyl,
oder ein Rest der Formel (VI),
worin
H = CO, SO₂,
I = Methylen, 1,2-Ethylen, 1,3-Trimethylen, 1,4-Tetramethylen, -CH₂-S-CH₂-, -CH₂-O-CH₂-, - CH₂-NH-CH₂-, 1,2-Ethenylen, 1,1-Ethylen, Prop-1-en-1,3-ylen, Prop-2-en-1,3-ylen, Benzylen, 2-Phenyl-1,1-ethylen, Carboxymethylen, Aminocarbonylmethylen, 2-Carboxy-1,1-ethylen, 2-Aminocarbonyl-1,1-ethylen, 3-Carboxy-1,1-propylen, 3-Aminocarbonyl-1,1-propylen, 2-Methyl-1,1-propylen, 3-Methyl-1,1-butylen, 2-Methyl-1,1-butylen, über N1 und C2 verknüpftes Pyrrolidin, 1,2-Phenylen, 1,3-Phenylen, 1,2-Naphthylen, 1,3-Naphthylen, 1,1-Cyclopentylen, 1,1-Cyclohexylen, 1,2-Cyclohexylen,
J = eine Bindung, CO, CS, SO₂, PO(OMe), PO(OH), CONH, CSNH, SO₂NH, PO(OH)O, PO(OH)NH, PO(OMe)O, PO(OMe)NH,
K = (C₁₃-C₁₉)-Alkyl, (C₁₃-C₁₉)-Alkenyl, 4-Benzyloxystyryl, 4-Styrylstyryl, 4-Phenylstyryl, 4-Cyanostyryl, 4-Nitrostyryl, Phenyl, 4-Biphenylyl, 4-Nitrophenyl, 4-Cyanophenyl, 4-Methylsulfonylphenyl, 4-Methoxyphenyl, 4-Bromphenyl, 4-Chlorphenyl, 4-Trifluormethylphenyl, 4-Formylphenyl, 4-Methoxycarbonylphenyl, 4-(1,1-Dicyano-2-vinyl)phenyl, 4-Aminophenyl, 4-Ethylphenyl, 4-Isopropylphenyl, 4-tert.-Butylphenyl, 4-Ethoxyphenyl, 4-Propoxyphenyl, 4-Butoxyphenyl, 4-Benzyloxyphenyl, 3,4-Bis-(benzyloxy)phenyl 3-Phenoxyphenyl, 4-Styrylphenyl 1-Naphthyl, 2-Naphthyl, 2-Fluorenyl, 2-(2-Phenylthiazol-4-yl), 5-(4-Nitrophenyl)thiazo-4-yl) 5-(4-Nitrophenyl)furan-2-yl), 5-(3-Nitrophenyl)furan-2-yl), 5-(2-Nitrophenyl)furan-2-yl), 5-(4-Bromphenyl)furan-2-yl), 5-(4-Chlorphenyl)furan-2-yl), 5-(3-Trifluormethylphenyl)furan-2-yl), 5-(4-Trifluormethylphenyl)furan-2-yl), 5-(2-Chlor-3-trifluormethylphenyl)furan-2-yl) 3,4-Methylendioxyphenyl, 1-Acetylindol-3-yl, 4'-Nitrobiphenylyl, 5-(4-Bromphenyl)thiophen-2-yl), 5-(4-Methylphenyl)furan-2-yl), 5-(4-Methoxyphenyl)furan-2-yl), 5-Bromthiophen-2-yl, 5-Bromfuran-2-yl, 4-Pyridyl, 3-Pyridyl, 2-Pyridyl, Chinolin-2-yl, 1-Naphthylvinyl, 2-Naphthylvinyl, 2-Fluorenyl-vinyl, 2-(2-Phenylthiazol-4-yl)vinyl, 2-[5-(4-Nitrophenyl)furan-2-yl)vinyl, 2-[5-(4-Acetoxy-methylphenyl)furan-2-yl)vinyl, 2-[5-(3-Trifluormethylphenyl)-furan-2-yl)vinyl, 4-Benzyloxystyryl, 3,4-Dibenzyloxystyryl, 3-Methoxy-4-(4-nitrobenzyloxy)styryl, 2-Methylindol-3-ylvinyl, 1-Acetylindol-3-ylvinyl, 3,4-Methylendioxystyryl, 4-(1,1 Dicyano-2-vinyl)styryl, bedeuten, und Salze davon.

Insbesondere eignen sich Verbindungen der Formel (I), für die gilt:
n=0-3,
R¹, R² = H
R³ = H,
A = CO, SO₂,
B = Phenyl, Benzyl, Phenethyl, 4-Chlorphenylmethyl, 4-Methylphenylmethyl, 4-Bromphenylmethyl, 4-Nitrophenylmethyl, 4-Trifluormethylphenylmethyl, 4-Phenylphenyl-methyl, 1-Naphthylmethyl, 2-Naphthylmethyl, Benzoyl, 2,4,4-Trimethylpentyl, 2-Carboxyethyl, 3-Carboxypropyl, 1-(4-Methyl-1-piperazinyl)-1-(4-trifluormethylphenyl)-methyl, 1-(4-Methyl-1-piperazinyl)-1-phenylmethyl,
D = Benzoyl,
Y =
ein Rest der Formel (IV)
worin
Z = O,
m = 0 - 3,
R³⁴ = H, Benzyl, 4-Nitrobenzyl, 4-Cyanobenzyl,
oder ein Rest der Formel (V)
worin
F = CO, CH₂, SO₂,
G = CH₂CH₂CONH-(C₁₄-C₁₈)-Alkyl, CH₂CH₂CH₂CONH-(C₁₄-C₁₈)-Alkyl, Phenyl, Naphthyl, Pyridyl, Fluorenyl, Anthracenyl
oder ein Rest der Formel (VI)
H=CO,
I = Methylen, 1,2-Ethylen, 1,3-Trimethylen, 1,4-Tetramethylen, -CH₂-S-CH₂-, -CH₂-O-CH₂-, - CH₂-NH-CH₂-, 1,2-Ethenylen, 1,1-Ethylen, Prop-1-en-1,3-ylen, Prop-2-en-1,3-ylen, Benzylen, 2-Phenyl-1,1-ethylen, 2-Methyl-1,1-propylen, 3-Methyl-1,1-butylen, 2-Methyl-1,1-butylen, über N 1 und C2 verknüpftes Pyrrolidin,
J = PO(OMe), PO(OH), CONH, CSNH, SO₂NH, PO(OH)O, PO(OH)NH, PO(OMe)O, PO(OMe)NH,
K = (C₁₃-C₁₉)-Alkyl, (C₁₃-C₁₉)-Alkenyl, 4-Benzyloxystyryl, 4-Phenylstyryl, 4-Cyanostyryl, 4-Nitrostyryl, Phenyl, 4-Biphenylyl, 4-Nitrophenyl, 4-Cyanophenyl, 4-Methoxyphenyl, 1-Naphthylvinyl, 2-Naphthylvinyl, 2-Fluorenylvinyl, 2-(2-Phenylthiazol-4-yl)vinyl, 2-[5-(4-Nitrophenyl)furan-2-yl)vinyl, 2-[5-(4-Acetoxymethylphenyl)furan-2-yl)vinyl, 2-[5-(3-Trifluormethylphenyl)furan-2-yl)vinyl, 3,4-Dibenzyloxystyryl, 3-Methoxy-4-(4-nitrobenzyloxy)styryl, 3,4-Methylendioxystyryl, 4-(1,1 Dicyano-2-vinyl)styryl bedeuten, und Salze davon.

Besonders bevorzugt sind Einzelverbindungen, die den nachfolgenden Strukturformeln entsprechen:

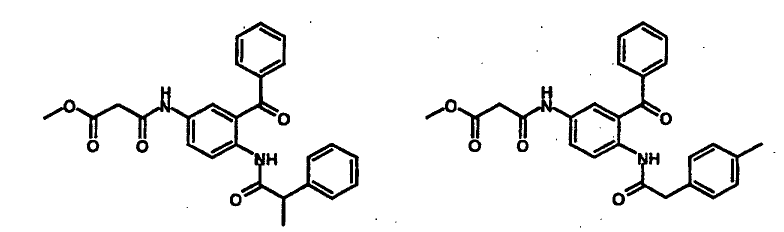

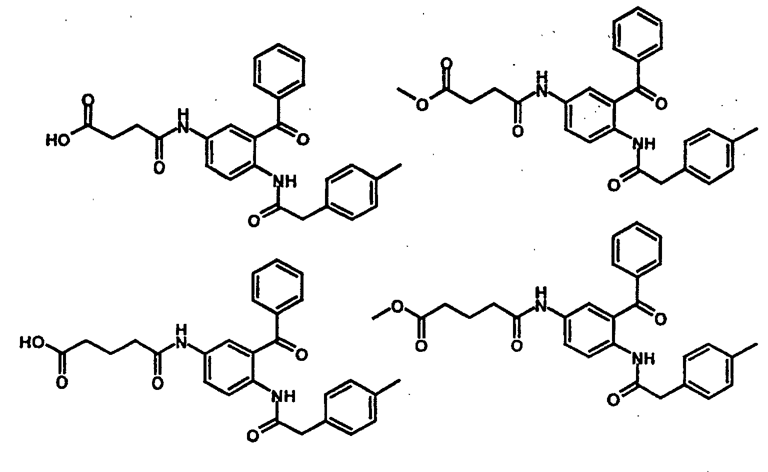

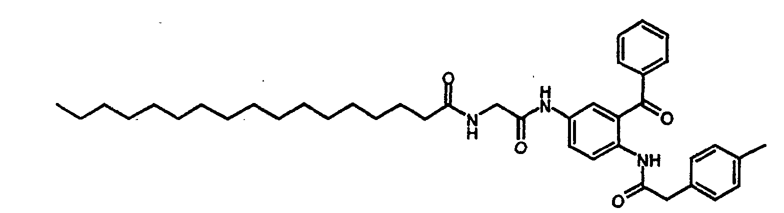

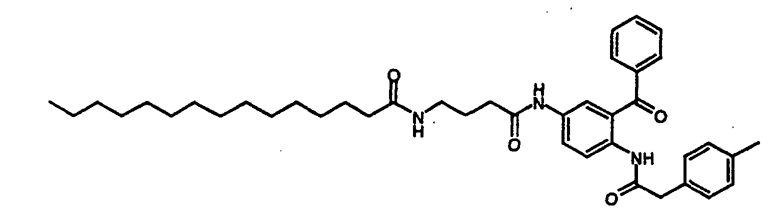

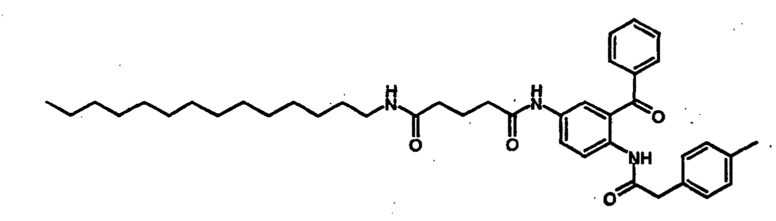

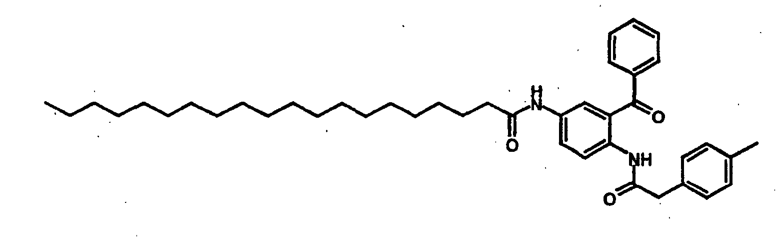

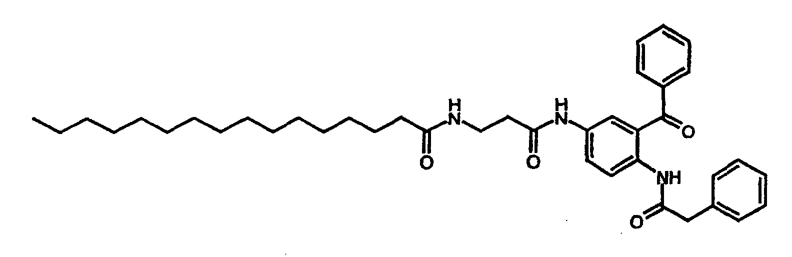

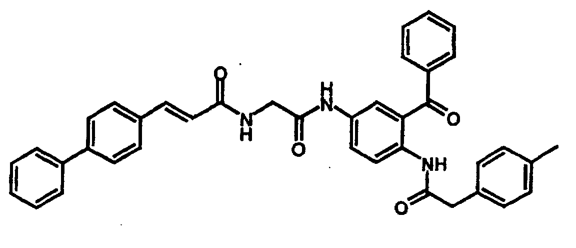

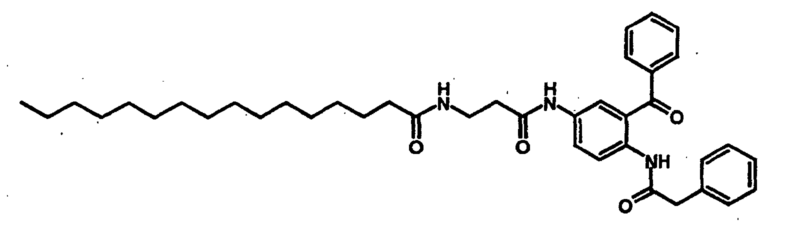

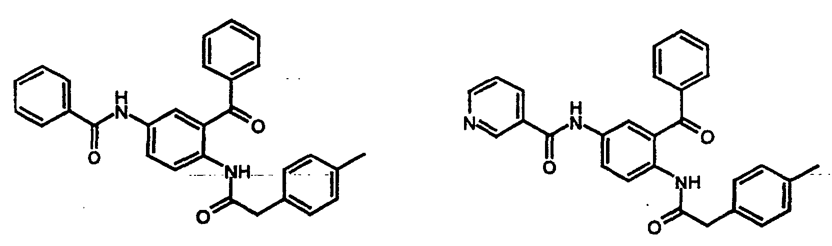

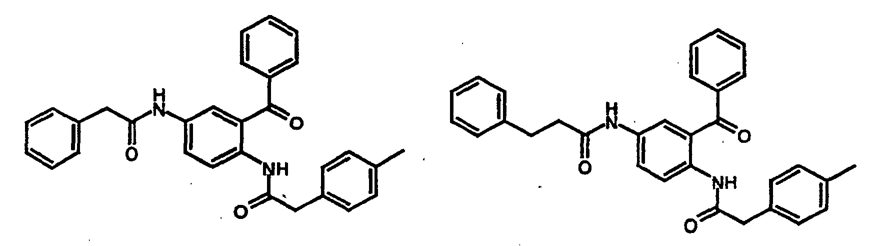

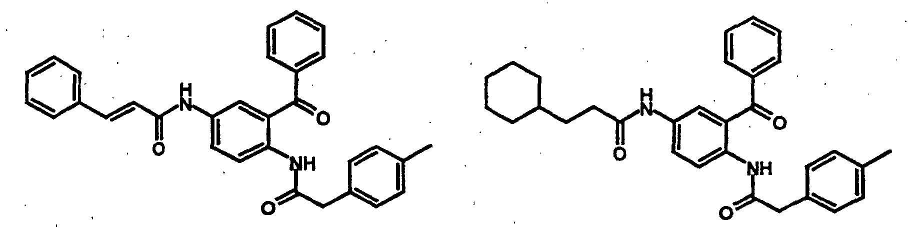

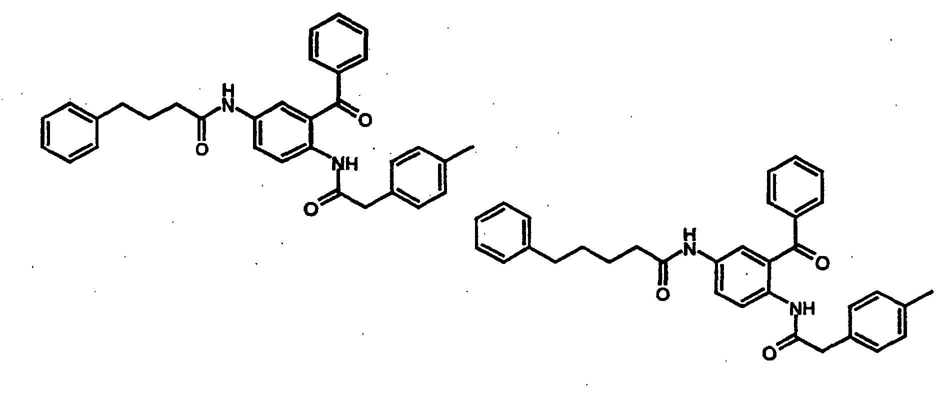

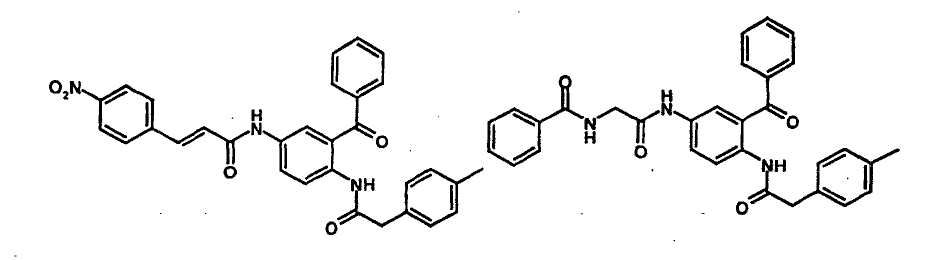

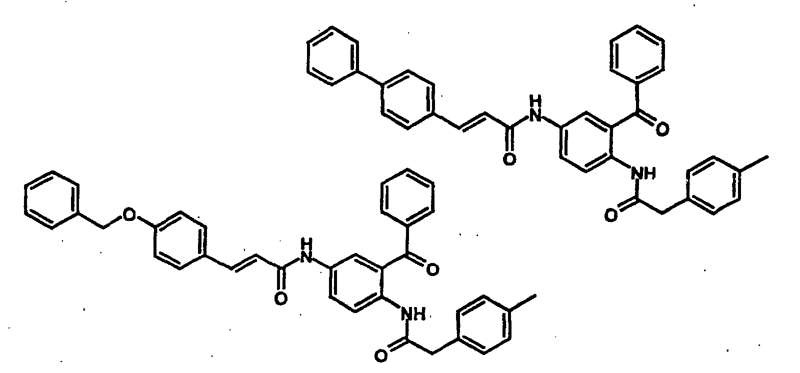

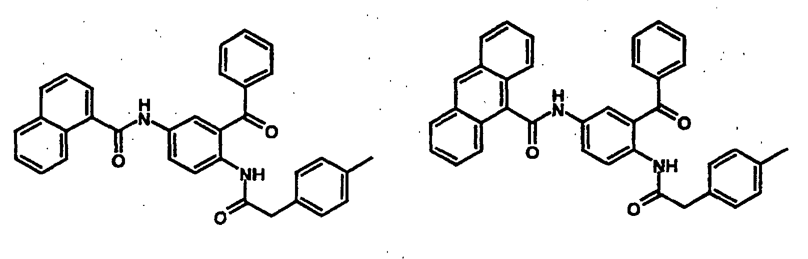

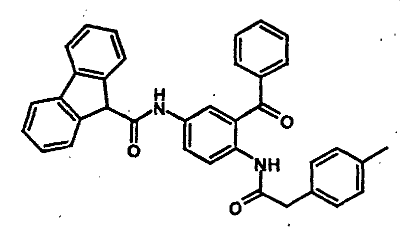

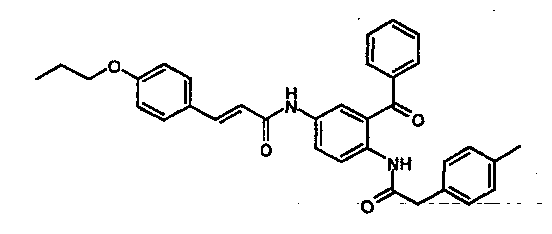

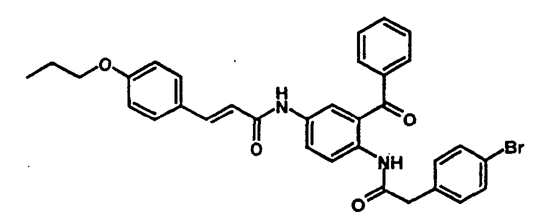

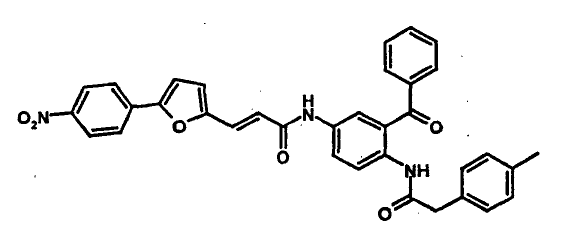

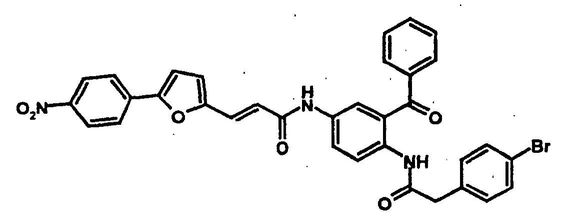

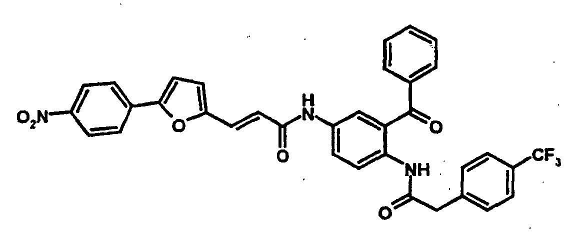

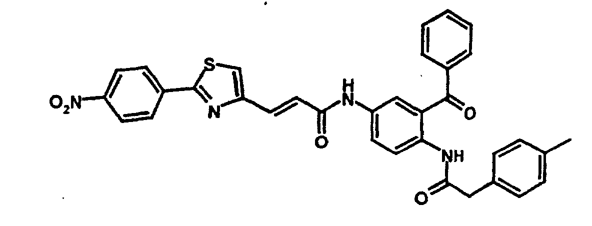

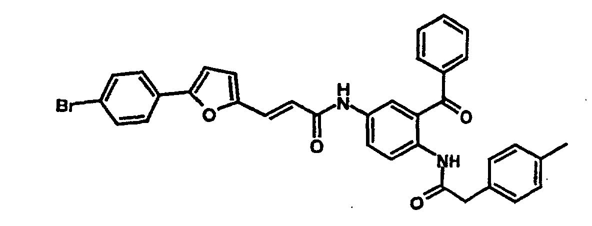

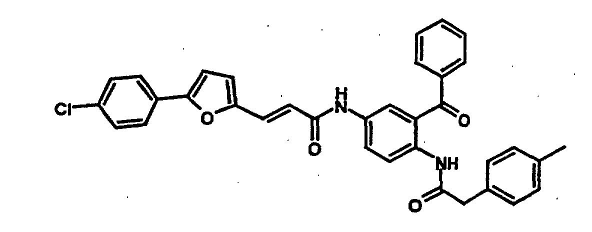

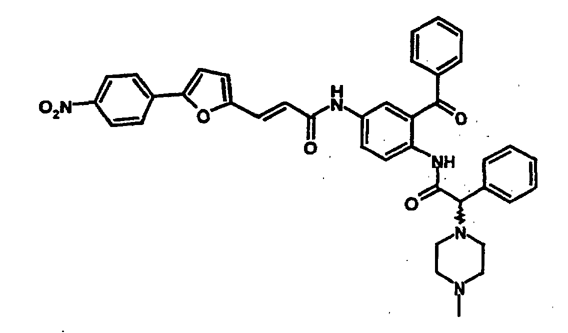

Von diesen Verbindungen sind N-[3-[3-Benzoyl-4-[2-(4-methylphenyl)-acetyl]amino]-phenylamino]-4-phenylzimtsäureamid und N-[2-[3-Benzoyl-4-[[2-(4-methylphenyl)acetyl]-amino]phenyl]- 4-benzyloxyzimtsäureamid ganz besonders bevorzugt.

In den vorstehenden und in den folgenden Formeln und Definitionen bedeuten Acyl insbesondere Alkanoyl, auch durch Aryl substituiertes Alkanoyl. Bevorzugt sind Acylgruppen mit 1 bis 5 Kohlenstoffatomen. Alkyl, auch in abgeleiteten Begriffen, wie Alkoxy, Alkylen, Alkenyl und Alkinyl ist geradkettig oder verzweigtkettig, enthält, soweit nicht anders angegeben, insbesondere 1 bis 8 C-Atome und ist unsubstituiert oder z.B. durch CN, NH₂, NO₂, COOH, CONH₂ und Alkoxycarbonyl substituiert. Aryl bedeutet vorwiegend Phenyl, durch z.B. Halogen, Alkyl, Trifluormethyl, Cyano, Aryl, Alkoxy, Hydroxy, Benzyloxy, Phenyl, Styryl, Acyl, NO₂, COOH, Alkylsulfonyl, SO₂NH₂ substituiertes Phenyl, Naphthyl, durch z.B. Halogen, Alkyl, Aryl, Alkoxy, Acyl, NO₂, COOH, SO₂NH₂ substituiertes Naphthyl, ferner z.B. auch Fluorenyl und Anthracenyl. Für Arylen gelten sinngemäß die gleichen Bedeutungen. Heteroaryl ist z.B. ein sechsgliedriger Aromat oder ein fünfgliedriger Aromat, der 1 bis 4 Heteroatome enthält, wobei unter Heteroatomen Stickstoff, Sauerstoff und Schwefel zu verstehen sind, beispielsweise Pyridyl, Furanyl, Thiazolyl, ferner z.B. auch Indolyl. Heteroaryl ist unsubstituiert oder wie Aryl und insbesondere auch mit Aryl substituiert. Aralkyl bedeutet Alkyl, das durch Aryl mono- oder polysubstituiert, insbesondere mono- bis trisubstituiert, ist. Bei Cycloalkylen, worin die Alkylenkette durch O, S oder NR77 unterbrochen ist, handelt es sich z.B. um über N1 und C2 verknüpftes Pyrrolidin, Halogen bedeutet Fluor, Chlor, Brom und Iod.

Die Verbindungen sind insbesondere für die therapeutische und prophylaktischen Behandlung von Infektionen bei Mensch und Tier geeignet, die durch Viren, Bakterien und Pilze ein- und mehrzellige Parasiten hervorgerufen werden. Sie zeigen eine besonders starke zytotoxische Wirksamkeit gegenüber ein- und mehrzelligen Parasiten, insbesondere gegenüber den Erregern der Malaria und der Schlafkrankheit.

Die Verbindungen sind vorzugsweise gegen einzellige Parasiten (Protozoen) einsetzbar, insbesondere gegen Erreger der Malaria und der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose.

Sie sind daher insbesondere als Malariaprophylaxe und als Prophylaxe der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose geeignet.

Die erfindungsgemäßen Verbindungen, hierzu gehören im allgemeinen auch ihre pharmazeutisch verträglichen Salze oder aber Verbindungen, die bei Applikation die erfindungsgemäßen Verbindungen als Stoffwechselprodukte oder Abbauprodukte bereitstellen, auch "Prodrugs" genannt, können für die Verabreichung in irgendeiner geeigneten Weise analog zu bekannten antiinfektiös wirkenden Mitteln (gemischt mit einem nicht toxischen pharmazeutisch akzeptablen Träger) zubereitet werden.

Die pharmazeutisch wirksamen Mittel können in Form von pharmazeutischen Zubereitungen in Dosierungseinheiten zubereitet werden. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füllund Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die Wirkstoffe der Formel (I) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-%, der Gesamtmischung vorhanden sein.

Die genannten Formulierungsformen können neben weiteren pharmazeutischen Wirkstoffen auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die Verbindungen können mit bisher beschriebenen Substanzen mit antiinfektiösen Eigenschaften verwendet werden. Hierzu gehören insbesondere Verbindungen, die bereits in der Therapie Anwendung gefunden haben oder noch angewendet werden. Hierzu sind insbesondere geeignet Stoffe, die in der in der Roten Liste oder in Simon/Stille, Antibiotika-Therapie in Klinik und Praxis, 9.Auflage 1998 Schattauer Verlag, oder unter http:/www.customs.treas.gov/imp-exp/rulings/harmoniz/hrm129.html im Internet mitaufgeführt. Insbesondere können die Derivate mit Penicillin, Benzylpenicillin (Penicillin G), Phenoxypenicillin, Isoxazolylpenicillin, Aminopenicillin, Ampicillin, Amoxixillin, Bacampicillin, Carboxypenicillin, Ticarcillin, Temocillin, Acyalaminopenicillin, Azlocillin, Mezlocillin, Piperacillin, Apalcillin, Mecillinam, Cephalosporine, Cefazolin-Gruppe, Cefuroxim-Gruppe, Cefoxitin-Gruppe, Cefoxitin, Cefotetan, Cefmetazol, Latamoxef, Flomoxef, Cefotaxim-Guppe, Cefozidim, Ceftazidim-Gruppe, Ceftazidim, Cefpirom, Cefepim, übrige Cephalosporine, Cefsulodin, Cefoperazon, Oralcephalosporine der Cefalexin-Gruppe, Loracarbef, Cefprozil, neue Oralcephalosporine mit erweitertem Spektrum, Cefixim, Cefpodoxim-Proxetil, Cefuroxim-Axetil, Cefetamet, Cefotiam-Hexetil, Cefdinir, Ceftibuten, andere β-Lactam-Antibiotika, Carbapenem, Imipenem /Cilastatin, Meropenem, Biapenem, Aztreonam, β-Lactamase-Hemmer, Clavulansäure/Amoxicillin, Clavulansäure/Ticarcillin, Sulbactam/Ampicillin, Tazobactam/Piperacillin, Tetracycline, Oxytetracyclin, Rolitetraxyxlin, Doxycyclin, Minocyclin, Chloramphenicol, Aminoglykoside, Gentamicin, Tobramycin, Netilmicin, Amikacin, Spectinomyxin, Makrolide, Erythromycin, Clarithromycin, Roxithromycin, Azithromycin, Dirithromycin, Spiramycin, Josamycin, Lincosamide, Clindamycin, Fusidinsäure, Glykopeptid-Antibiotika, Vancomycin, Tecoplanin, Pristinamycin-Derivate, Fosfomycin, Antimikrobielle Folsäureantagonisten, Sulfonamide, Co-Trimoxazol, Trimethoprim, andere Diaminopyrimidin-Sulfonamid-Kombinationen, Nitrofurane, Nitrofurantoin, Nitrofurazon, Gyrase-Hemmer (Chinolone), Norfloxacin, Ciprofloxacin, Ofloxacin, Sparfloxacin, Enoxacin, Fleroxacin, Pefloxacin, Lomefloxacin, Bay Y3118, Nitroimidazole, antimykobakterielle Mittel, Isoniazid, Rifampicin, Rifabutin, Ethambutol, Pyrazinamid, Streptomycin, Capreomycin, Prothionamid, Terizidon, Dapson, Clofazimin, Lokalantibiotika, Bacitracin, Tyrothricin, Polymyxine, Neomycin, Kanamycin, Paromomycin, Mupirocin, antivirale Mittel, Acyclovir, Ganciclovir, Azidothymidin, Didanosin, Zalcitabin, Thiacytidin, Stavudin, Ribavirin, Idoxuridin, Trifluridin, Foscarnet, Amantadin, Interferone, Tibol-Derivate, Proteinase-Inhibitoren, Antimykotika, Polyene, Amphothericin B, Nystatin, Natamycin, Azole, Azole zur septischen Therapie, Miconazol, Ketoconazol, Itraconazol, Fluconazol, UK-109.496, Azole für lokale Anwendung, Clotrimazol, Econazol, Isoconazol, Oxiconazol, Bifonazol, Flucytosin, Griseofulvin, Ciclopiroxolamin, Tolnaftat, Naftifin, Terbinafin, Amorolfin, Antrachinone, Betulinic acid, Semianthrachinone, Xanthone, Naphtoquinone, Aryaminoalkohole, Chinin, Quinidine, Mefloquin, Halofantrin, Chloroquin, Amodiaquin, Acridin, Benzonaphthyridin, Mepacrin, Pyronaridin, Dapson, Sulfonamide, Sulfadoxin, Sulfalene, Trimethoprim, Proguanil, Chlorproguanil, Diaminopyrimidine, Pyrimethamin, Primaquin, Aminoquinoline, WR 238,605, Tetracyclin, Doxycyclin, Clindamycin, Norfloxacin, Ciprofloxacin, Ofloxacin, Artemisinin, Dihydroartemisinin, 10b Artemether, Arteether, Atrtesunat, Atovaquon, Suramin, Melarsoprol, Nifurtmox, Stibogluconat-Natrium, Pentamidin, Amphotericin B, Metronidazol, clioquinol, Mebendazol, Niclosamid, Praziquantel, Pyrantel, Tiabendazol, Diethylcarbamazin, Ivermectin, Bithionol, Oxamniquin, Metrifonat, Piperazin, Embonat kombiniert werden.

Ferner können die Verbindungen in den pharmazeutischen Mitteln in Kombination mit Sulfonamid, Sulfadoxin, Artemisinin, Atovaquon, Chinin, Chloroquin, Hydroxychloroquin, Mefloquin, Halofantrin, Pyrimethamin, Armesin, Tetracycline, Doxycyclin, Proguanil, Metronidazol, Praziquantil, Niclosamid, Mebendazol, Pyrantel, Tiabendazol, Diethylcarbazin, Piperazin, Pyrivinum, Metrifonat, Oxamniquin, Bithionol oder Suramin oder mehreren dieser Substanzen vorliegen.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesaintmengen von etwa 0,05 bis etwa 600, vorzugsweise 0,5 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 200, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Die erfindungsgemäßen Verbindungen können in den üblichen Konzentrationen und Zubereitungen bei Tieren zusammen mit dem Futter bzw. mit Futterzubereitungen oder mit dem Trinkwasser gegeben werden.

Die erfindungsgemäßen Verbindungen werden in an sich bekannter Weise hergestellt, beispielsweise, indem
(a) ein 2-Acyl-4-nitroanilin mit einem geeigneten Acylchlorid in inertem Lösungsmittel bei erhöhter Temperatur acyliert wird,
(b) das unter (a) erhaltene 4-Nitroanilid mit Zinn-II-chlorid oder Palladium/Wasserstoff zur entsprechenden Aminoverbindung reduziert wird,
(c) die unter (b) erhaltene Aminoverbindung mit geeigneten substituierten Carbonsäuren, substituierten Carbonsäureanhydriden oder N-substituierten Aminosäuren acyliert wird, wobei N-Acylaminosäuren in der Regel mittels der gemischten Anhydrid-Methode aktiviert werden; und
(d) falls in (c) geschützte Aminosäurederivate verwendet werden, ggf. vorhandene Schutzgruppen unter Verwendung von Standardtechniken der Peptidchemie abgespalten werden.

Die Herstellung der Verbindungen ist in den Schemata 1-4 exemplarisch dargestellt:

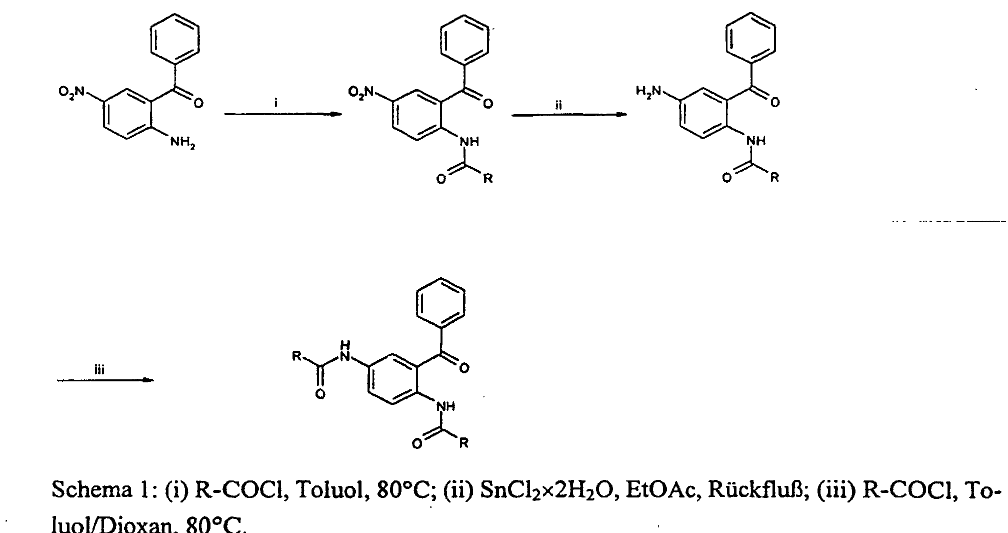

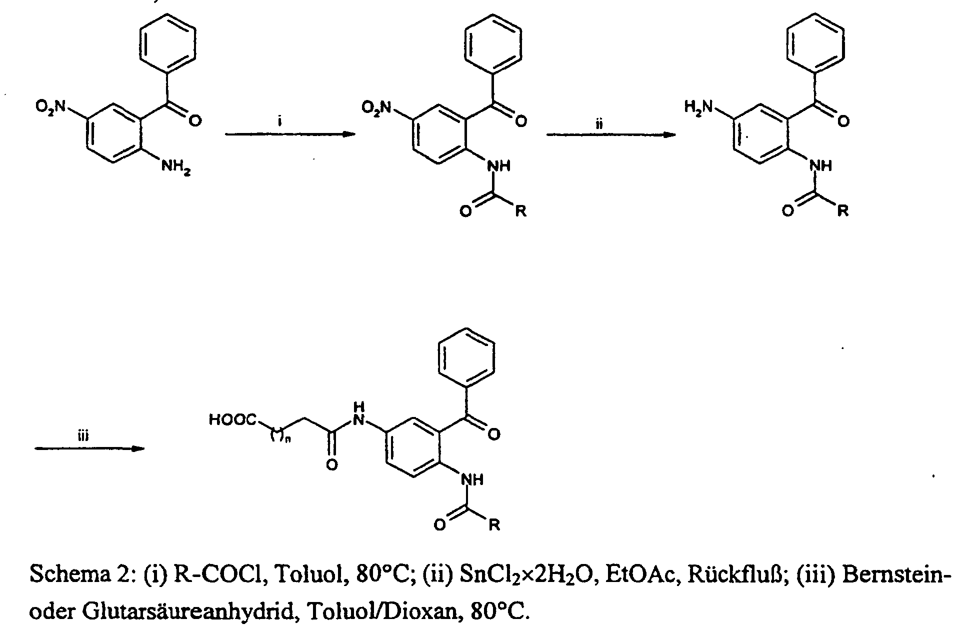

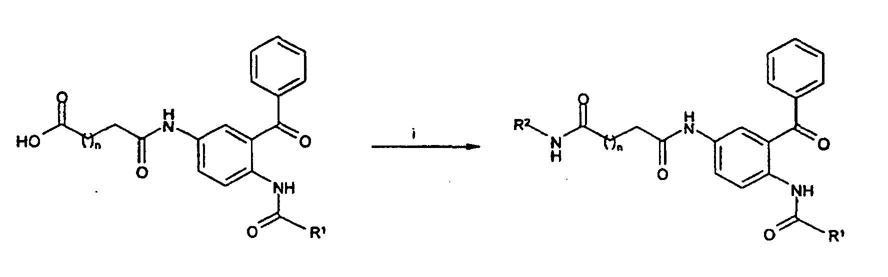

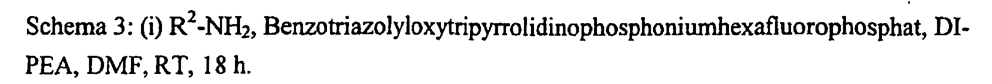

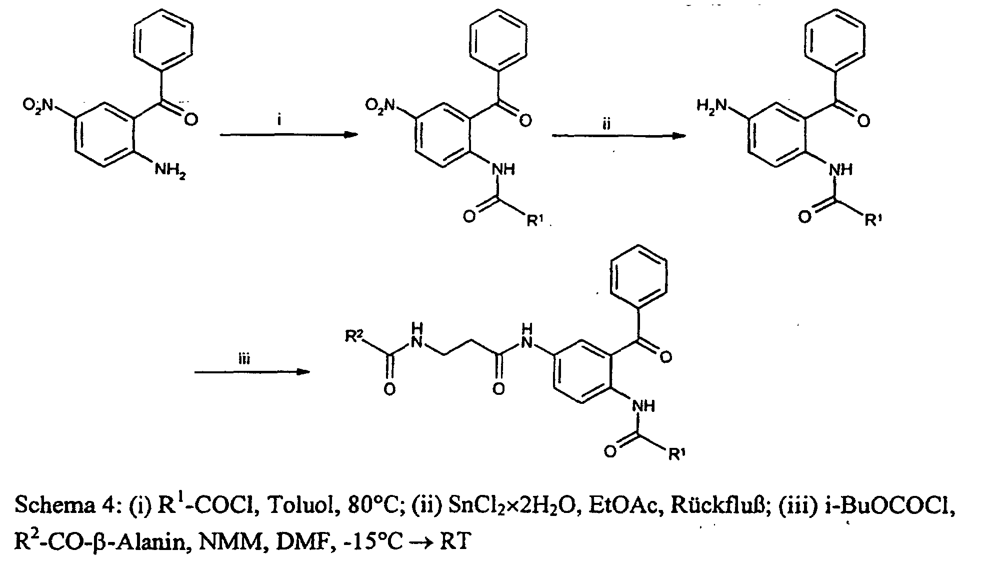

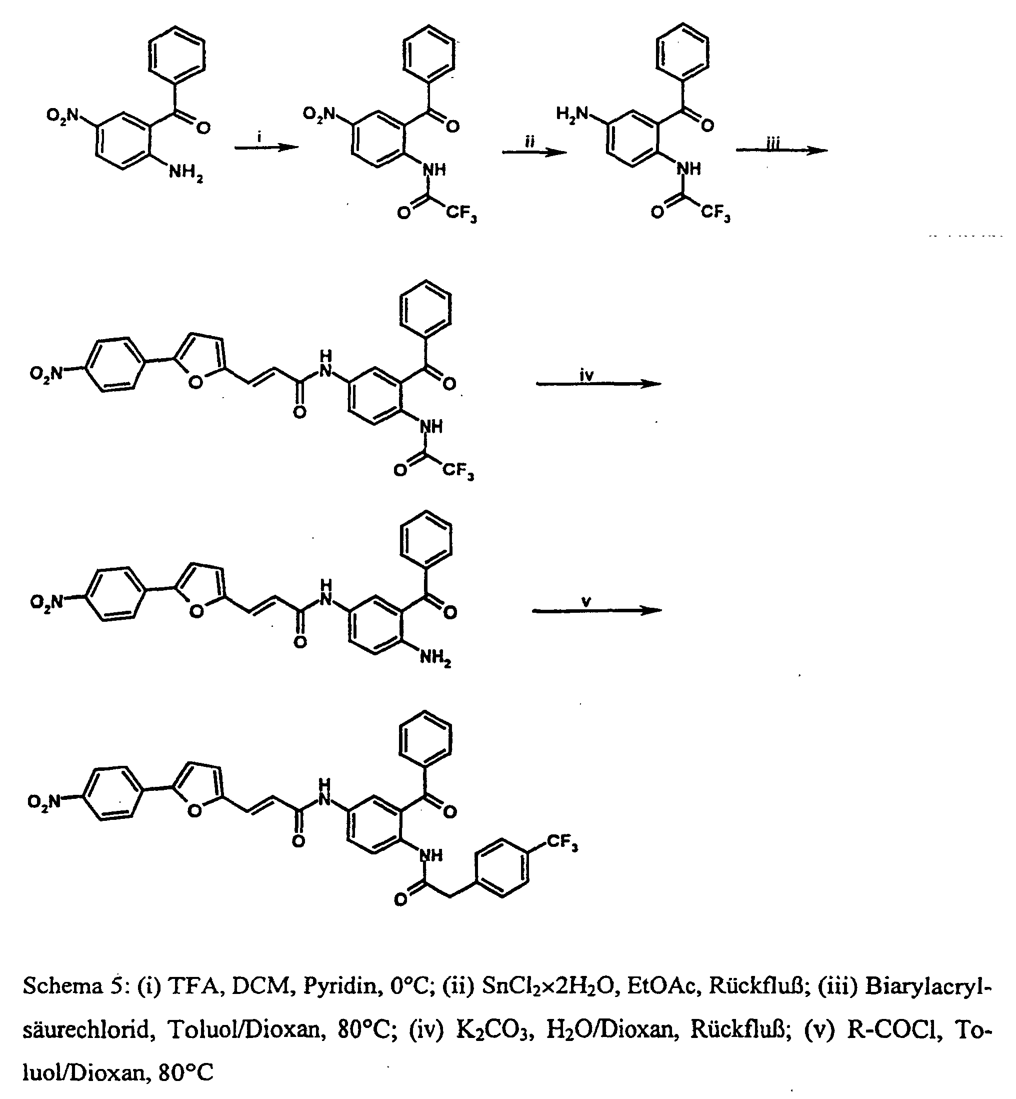

### Allgemeine Vorschrift 1:

Ein geeignetes 2-Acyl-4-nitroanilin wird in einer ausreichenden Menge Toluol - evtl. unter Erwärmen - gelöst. Anschließend wird eine äquimolare Menge eines geeigneten Carbonsäurechlorids hinzugegeben und die Mischung 2 h auf 80°C erwärmt. Anschließend wird die Reaktionsmischung eingeengt, worauf in einigen Fällen spontane Kristallisation auftritt. Die Kristalle werden isoliert und im Vakuum getrocknet. Tritt keine spontane Kristallisation auf, wird das Lösungsmittel vollständig abdestilliert und der Rückstand durch Säulenchromatographie an Kieselgel gereinigt.

### Allgemeine Vorschrift 2:

Eine Lösung der nach Vorschrift 1 erhaltenen Verbindung in Ethanol oder Ethylacetat (5 ml/mmol) wird mit Zinn-(II)-chlorid-Dihydrat (5 Äquivalente 1.125 g/mmol) 2 h zum Sieden erhitzt. Die abgekühlte Reaktionslösung wird mit Wasser verdünnt, mit gesättigter Nartiumhydrogencarbonat-Lösung auf pH 7-8 gebracht und mit Ethylacetat (3 × 100-200 ml) extrahiert. Die vereinigten organischen Extrakte werden mit gesättigter Natriumchlorid-Lösung gewaschen, über Natriumsulfat getrocknet und am Rotationsverdampfer vollständig vom Lösungsmittel befreit. In der Regel verbleibt ein Feststoff oder ein Öl, das oft innerhalb einiger Tage durchkristallisiert.

### Allgemeine Vorschrift 3:

Eine Lösung von einem Äquivalent eines Säurechlorids in Dioxan wird zu eine Lösung einer nach Vorschrift 2 erhaltenen Verbindung in Toluol/Dioxan gegeben und das Gemisch 1-2 h auf 80°C erwärmt. Anschließend wird im Vakuum eingeengt und der anfallende Feststoff isoliert.

### Allgemeine Vorschrift 4:

Eine Lösung von einem Äquivalent eines Säureanhydrids in Dioxan wird zu eine Lösung einer nach Vorschrift 2 erhaltenen Verbindung in Toluol/Dioxan gegeben und das Gemisch 1-2 h auf 80°C erwärmt. Anschließend wird im Vakuum eingeengt und der anfallende Feststoff isoliert.

### Allgemeine Vorschrift 5:

Eine Lösung äquimolarer Mengen eines Amins und einer Carbonsäure in DMF wird mit einem Äquivalent 1-Benzotriazolyloxy-tripyrrolidinophosphoniumhexafluorophosphat und 3 Äquivalenten Diisopropylethylamin versetzt und 18 h bei Zimmertemperatur gerührt. Anschließend wird mit Kochsalzlösung verdünnt und mit Ethylacetat extrahiert. Die Extrakte werden mit 2N Citronensäure, ges. Natriumhydrogencarbonat-Lösung und Kochsalzlösung gewaschen. Das nach dem Abdestillieren des Lösungsmittels verbleibende Produkt wird wie angegeben gereinigt.

### Allgemeine Vorschrift 6:

Eine geeignete N-Acylaminosäure wird unter Argon in einer ausreichenden Menge getrocketem DMF gelöst und nach Zugabe von 2.28 Äquivalenten N-Methyl- (NMM: 0.25 ml/mmol Aminosäure) auf -15°C abgekühlt. Anschließend wird ein Äquivalent Chlorameisensäureisobutylester (0.13 ml/mmol Aminosäure) zugegeben. Nach fünf Minuten wird zu dieser Mischung eine Lösung eines Äquivalentes einer nach Vorschrift 2 erhaltenen Verbindung, in einer ausreichenden Menge getrocknetem DMF gelöst, gegeben. Die Reaktionslösung wird mehrere Stunden gerührt wobei sie langsam Raumtemperatur erreicht. Anschließend wird der Ansatz in eine gerührte gesättigte Kochsalzlösung (400 - 800 ml) gegossen. Die wäßrige Lösung wird mit Ethylacetat dreimal extrahiert. Die vereinigten Extrakte werden mit 2 N Citronensäure, gesättigter Natriumhydrogencarbonat-Lösung und gesättigter Natriumchlorid-

Lösung gewaschen und über Magnesiumsulfat getrocknet. Der nach dem Entfernen des Lösungsmittels am Rotationsverdampfer verbleibende Rückstand wird durch Säulenchromatographie an Kieselgel gereinigt.

### Beispiel 1: 2-[N-[3-[3-Benzoyl-4-(2-phenylpropionyl)amino]phenylamino]carbamoyl]-essigsäuremethylester

### Stufe 1: N-(2-Benzoyl-4-nitrophenyl)-2-phenylpropionsäureamid

Gemäß Allgemeiner Vorschrift 1 aus 2-Amino-5-nitrobenzophenon (1.2 g, 5 mmol) und 2-Phenylpropansäurechlorid (0.842 g, 5 mmol). Reinigung: Umkristallisation aus Ethanol.

Ausbeute: 1.098 g (59%)

¹H-NMR (CDCl₃): δ = 1.55 (d, J=7 Hz, 3H), 3.74 (q, J=7 Hz, 1H), 7.19 (m, 1H), 7.30 (m, 2H), 7.34 (m, 2H), 7.45 (m,2H), 7.58 (m, 3H), 8.30 (m, 1H), 8.35 (m, 1H), 8.85 (m, 1H), 11.11 (s, 1H).

### Stufe 2: N-(4-Amino-2-benzoylphenyl)-2-phenylpropionsäureamid

Gemäß Allgemeiner Vorschrift 2 aus N-(2-Benzoyl-4-nitrophenyl)-2-phenylpropionsäureamid (1.020 g, 2.75 mmol).

Ausbeute: 0.937 g (99%)

¹H-NMR (CDCl₃): δ = 1.50 (d, J=7 Hz, 3H), 3.63 (q, J=7 Hz, 1H), 6.69 (m, 1H), 6.69 (m 1H), 7.17 (m, 1H), 7.25 (m, 2H), 7.31 (m, 2H), 7.38 (m, 2H), 7.51 (m, 1H), 7.59 (m, 2 H), 8.25 (m, 1H), 10.21 (s, 1H).

### Stufe 3: 2-[N-[3-[3-Benzoyl-4-(2-phenylpropionyl)amino]phenylamino]carbamoyl]-essigsäuremethylester

C₂₆H₂₄N₂O₅ (444.49 gmol⁻¹)

Gemäß allgemeiner Vorschrift 3 aus N-(4-Amino-2-benzoylphenyl)-2-(phenyl)propionsäureamid (0.688 g, 2 mmol) und Malonsäuremethylester-chlorid (0.24 ml, 2.2 mmol). Säulenchromatographie mit Ethylacetat Hexan 2:3.

Ausbeute: 0.66 g (74%), gelber Feststoff.

IR (KBr): ν = 3305, 2920, 1745, 1665, 1560 cm⁻¹. ¹H-NMR (CDCl₃): δ = 1.61 (m, 3H), 3.41 (s, 2H), 3.74 (m, 1H), 3.77 (s, 3H), 7.25 (m, 1H), 7.34 (m, 2H), 7.39 (m, 2H), 7.47 (m, 2H), 7.56 (m, 2H), 7.67 (m, 2H), 7.90 (m, 1H), 8.69 (m, 1H), 9.18 (s, 1H), 10.69 (s, 1H)

### Beispiel 2: 2-[N-[3-[3-Benzoyl-4-[[2-(4-methylphenyl)acetyl]amino]phenylamino]-carbamoyl]essigsäuremethylester

### Stufe 1: N-(2-Benzoyl-4-nitrophenyl)-2-(4-methylphenyl)acetamid

Gemäß Allgemeiner Vorschrift 1 aus 2-Amino-5-nitrobenzophenon (1.2 g, 5 mmol) und 2-(4-Methylphenyl)acetylchlorid (0.843 g, 5 mmol). Reinigung: Umkristallisation aus Ethanol. Ausbeute 1.75 g (93%).

¹H-NMR (CDCl₃): δ =2.33 (s, 3H), 3.74 (s, 2H), 7.17 (m, 2H), 7.24 (m, 2H), 7.51 (m, 2H), 7.65 (m, 3H), 8.37 (m, 1H), 8.41 (m, 1H), 8.88 (d, J=9 Hz, 1H), 11.05 (s, 1H).

### Stufe 2: N-(4-Amino-2-benzoylphenyl)-2-(4-methylphenyl)acetamid

Gemäß Allgemeiner Vorschrift 2 aus N-(2-Benzoyl-4-nitrophenyl)-2-(4-methylphenyl)acetamid (1.75 g, 4.7 mmol).

Ausbeute: 1.053 g (65%).

### Stufe 3: 2-[N-[3-[3-Benzoyl-4-[[2-(4-methylphenyl)acetyl]amino]phenylamino]carbamoyl]-essigsäuremethylester

C₂₆H₂₄N₂O₅ (444.49 gmol⁻¹)

Gemäß allgemeiner Vorschrift 3 N-(4-Amino-2-benzoylphenyl)-2-(4-methylphenyl)acetamid (0.688 g, 2 mmol) und Malonsäuremethylester-chlorid (0.24 ml, 2.2 mmol). Umkristallisation aus Toluol.

Ausbeute: 0.46 g (50%), gelber Feststoff.

IR (KBr): ν = 3295, 2955, 1740, 1690, 1660, 1560 cm⁻¹. ¹H-NMR (CDCl₃): δ = 2.22 (s, 3H), 3.32 (s, 2H), 3.40 (s, 2H), 3.62 (s, 3H), 6.95 (m, 2H), 7.01 (m, 2H), 7.45 (m, 2H), 7.54 (m, 1H), 7.62 (m, 4H), 7.73 (m, 1H), 10.0 (s, 1H), 10.22 (s, 1H). ¹³C-NMR (CDCl₃): δ = 16.7, 38.4, 39.5, 48.0, 116.5, 118.4, 120.4, 124.3, 124.9, 125.0, 125.6, 127.1, 128.1, 128.3, 128.7, 131.1131.6, 133.3, 160.1, 164.0, 165.2, 191.1.

### Beispiel 3: 3-[N-[3-[3-Benzoyl-4-[[2-(4-methylphenyl)acetyl]amino]phenylamino]-carbamoyl]propionsäure

### Stufen 1 und 2: s. Beispiel 2

### Stufe 3: 3-[N-[3-[3-Benzoyl-4-[[2-(4-methylphenyl)acetyl]amino]phenylamino]carbamoyl]-propionsäure

C₂₆H₂₄N₂O₅ (444.49 gmol⁻¹)

Gemäß allgemeiner Vorschrift 4 aus N-(4-Amino-2-benzoylphenyl)-2-(4-methylphenyl)acetamid (0.688 g, 2 mmol) und Bernsteinsäureanhydrid (0.200 g, 2 mmol). Umkristallisation aus Toluol.

Ausbeute: 0.880 g (99%), hellgelber Feststoff.

IR (KBr): ν = 3330, 2900-2600, 1725, 1655, 1560 cm⁻¹. ¹H-NMR (DMSO-d₆): δ = 2.19 (s, 3H), 2.43 (m, 4H), 3.51 (s, 2H), 6.93 (m, 2H), 6.98 (m, 2H), 7.42 (m, 2H), 7.50 (m, 1H), 7.59 (m, 4H), 7.70 (m, 1H), 9.90 (s, 1H), 9.93 (s, 1H). ¹³C-NMR (DMSO-d₆): δ = 20.4, 28.6, 30.9, 42.1, 120.0, 121.9, 124.0, 128.0, 128.6, 128.7, 129.3 131.3, 132.1, 132.4, 135.2, 135.3, 137.0, 168.8, 170.0, 173.4, 194.9.

### Beispiel 4: 3-[N-[3-[3-Benzoyl-4-[[2-(4-methylphenyl)acetyl]amino]phenylamino]-carbamoyl]propionsäuremethylester

### Stufen 1 und 2: s. Beispiel 2

### Stufe 3: 3-[N-[3-[3-Benzoyl-4-[[2-(4-methylphenyl)acetyl]amino]phenylamino]carbamoyl]-propionsäure

C₂₇H₂₆N₂O₅ (458.52 gmol⁻¹)

Gemäß allgemeiner Vorschrift 3 aus N-(4-Amino-2-benzoylphenyl)-2-(4-methylphenyl)acetamid (0.344 g, 1 mmol) und Bemsteinsäuremethylester-chlorid (0.20 ml, 1 mmol). Umkristallisation aus Toluol.

Ausbeute: 0.348 g (76%), hellgelber Feststoff.

IR (KBr): ν = 3375, 2950, 2930, 1735, 1715, 1690, 1635, 1550 cm⁻¹. ¹H-NMR (CDCl₃): δ = 2.33 (s, 3H), 2.57 (m, 2H), 2.69 (m, 2H), 3.65 (s, 3H), 3.66 (s, 2H), 7.15 (m, 2H), 7.22 (m, 2H), 7.48 (m, 3H), 7.57 (m, 1H), 7.67 (m, 2H), 7.75 (m, 1H), 7.80 (m, 1H), 8.46 (m, 1H), 10.44 (s, 1H). ¹³C-NMR (CDCl₃): δ = 21.1, 29.1, 31.8, 45.0, 52.0, 122.4, 124.2, 124.4, 125.1, 128.3, 129.3, 129.6, 130.0 131.2, 132.4, 132.7, 135.2, 136.1, 137.0, 138.0, 169.7, 170.3, 173.6, 198.5.

### Beispiel 5: 4-[N-[3-[3-Benzoyl-4-[[2-(4-methylphenyl)acetyl]amino]phenylamino]-carbamoyl]buttersäure

### Stufen 1 und 2: s. Beispiel 2

### Stufe 3: 4-[N-[3-[3-Benzoyl-4-[[2-(4-methylphenyl)acetyl]amino]phenylamino]carbamoyl]-buttersäure

C₂₇H₂₆N₂O₅ (458.52 gmol⁻¹)

Gemäß allgemeiner Vorschrift 6 aus N-(4-Amino-2-benzoylphenyl)-2-(4-methylphenyl)-acetamid (0.688 g, 2 mmol) und Glutarsäureanhydrid (0.228 g, 2 mmol). Umkristallisation aus Toluol.

Ausbeute: 0.740 g (81%), hellgelber Feststoff.

IR (KBr): ν = 3285, 2900-2600, 1735, 1690, 1660 cm⁻¹. ¹H-NMR (DMSO-d₆): δ = 1.76 (m, 2H), 2.22 (m, 5H), 2.28 (m, 2H), 3.31 (s, 2H), 6.95 (m, 2H), 7.01 (m, 2H), 7.45 (m, 2H), 7.51-(m, 1H), 7.62 (m, 4H), 7.75 (m, 1H), 9.91 (s, 1H), 9.93 (s, 1H), 11.91 (s, 1H). ¹³C-NMR
(DMSO-d₆): δ = 20.2, 20.4, 32.8, 35.2, 42.5, 120.1, 122.0, 124.0, 128.0, 128.6, 128.7, 129.3 131.3, 132.1,132.4, 135.2, 135.3, 137.0,168.8, 170.6, 173.7, 194.3.

### Beispiel 6: 4-[N-[3-[3-Benzoyl-4-[[2-(4-methylphenyl)acetyl]amino]phenylamino]-carbamoyl]buttersäuremethylester

### Stufen 1 und 2: s. Beispiel 2

### Stufe 3: 4-[N-[3-[3-Benzoyl-4-[[2-(4-methylphenyl)acetyl]amino]phenylamino]carbamoyl]-buttersäuremethylester

C₂₈H₂₈N₂O₅ (472.55 gmol⁻¹)

Gemäß allgemeiner Vorschrift 3 aus N-(4-Amino-2-benzoylphenyl)-2-(4-methylphenyl)-acetamid (0.344 g, 1 mmol) und Glutarsäuremethylester-chlorid (0.17 ml, 1 mmol). Umkristallisation aus Toluol.

Ausbeute: 0.310 g (65%), hellgelber Feststoff.

IR (KBr): ν = 3300, 3050, 2950, 1740, 1660, 1560 cm⁻¹. ¹H-NMR (CDCl₃): δ = 1.90 (m, 2H), 2.25 (s, 3H), 2.27 (m, 4H), 3.56 (s, 3H), 3.59 (s, 2H), 7.08 (m, 2H), 7.15 (m, 2H), 7.40 (m, 3H), 7.50 (m, 1H), 7.61 (m, 3H), 7.78 (m, 1H), 8.38 (m, 1H), 10.38 (s, 1H). ¹³C-NMR (CDCl₃): δ = 20.5, 21.1, 32.9 36.1, 45.0, 51.6, 122.4, 124.3, 124.4, 125.1, 128.3, 129.3, 129.6, 130.0 131.1, 132.6, 132.7, 136.0, 137.0, 138.0, 170.3, 170.5, 173.7, 198.5.

### Beispiel 7: N-[2-[3-Benzoyl-4-[[2-(4-methylphenyl)acetyl]amino]phenylamino]-2-oxoethyl]-heptadecansäureamid

### Stufen 1 und 2: s. Beispiel 2

### Stufe 3: N-[2-[3-Benzoyl-4-[[2-(4-methylphenyl)acetyl]amino]phenylamino]-2-oxoethyl]-heptadecansäureamid

C₄₁H₅₅N₃O₄ (653.91 gmol⁻¹)

Gemäß Allgemeiner Vorschrift 6 aus N-(4-Amino-2-benzoylphenyl)-2-(4-methylphenyl)acetamid (0.344 g, 1 mmol) und N-Heptadecanoylglycin (0.328 g, 1 mmol). Säulenchromatographie mit Ethylacetat n-Hexan 3 : 2.

Ausbeute: 0.415 g (63%), gelber Feststoff, Fp.: 46°C.

Ber.: C 75.31, H 8.48, N 6.43; Gef.: C, 75.34 H, 8.79 N 6.56.

IR (KBr): ν = 3305, 2920, 2850, 1645, 1555 cm⁻¹. ¹H-NMR (CDCl₃): δ = 0.80 (t, J=7 Hz, 3H), 1.21 (m, 26H), 1.47 (m, 2H), 2.08 (m, 2H), 2.26 (s, 3H), 3.61 (s, 2H), 3.99 (d, J=5 Hz, 2H), 6.44 (m, 1H), 7.10 (m, 2H), 7.18 (m, 3H), 7.39 (m, 2H), 7.50 (m, 2H), 7.61 (m, 2H), 7.78 (m, 1H), 8.43 (m, 1H), 8.95 (s, 1H), 10.42 (s, 1H). ¹³C-NMR (CDCl₃): δ = 14.1, 21.1, 22.7, 24.8, 25.5, 29.1, 29.2, 29.3, 29.4, 29.5, 29.6, 29.7, 29.8, 31.9, 33.7, 36.3, 44.4, 45.0, 122.4, 124.1, 124.2, 125.1, 128.3, 129.3, 129.6, 130.0, 131.1, 132.3, 132.7, 136.3, 137.0, 138.0, 167.1 170.3, 174.4, 198.4. MS: m/z = 652 (100, M⁺), 344 (93), 212 (94).

### Beispiel 8: N-[4-[3-Benzoyl-4-[[2-(4-methylphenyl)acetyl]amino]phenylamino]-4-oxobutyl]-pentadecansäureamid

### Stufen 1 und 2: s. Beispiel 2

### Stufe 3: N-[4-[3-Benzoyl-4-[[2-(4-methylphenyl)acetyl]amino]phenylamino]-4-oxobutyl]-pentadecansäureamid

C₄₁H₅₅N₃O₄ (653.91 gmol⁻¹)

Gemäß Allgemeiner Vorschrift 6 aus N-(4-Amino-2-benzoylphenyl)-2-(4-methylphenyl)-acetamid (0.688 g, 2 mmol) und N-Pentadecanoyl-γ-aminobuttersäure (0.712 g, 2 mmol). Säulenchromatographie mit Ethylacetat n-Hexan 3 : 2.

Ausbeute: 0.310 g (47%), gelber Feststoff, Fp.: 115°C.

Ber.: C 75.31, H 8.48, N 6.43; Gef.: C, 74.91 H, 8.06 N 6.62.

IR (KBr): ν = 3295, 2920, 2850, 1645, 1550 cm⁻¹. ¹H-NMR (CDCl₃): δ = 0.81 (t, J=7 Hz, 3H), 1.20 (m, 22H), 1.50 (m, 2H), 1.75 (m, 2H), 2.07 (m, 2H), 2.23 (m,2H), 2.26 (s, 3H), 3.24 (m, 2H), 3.60 (s, 2H), 5.80 (m, 1H), 7.10 (m, 2H), 7.17 (m, 3H), 7.39 (m, 2H), 7.50 (m, 2H), 7.65 (m, 2H), 7.96 (m, 1H), 8.43 (m, 1H), 9.20 (s, 1H), 10.46 (s, 1H). ¹³C-NMR (CDCl₃): δ = 14.1, 21.1, 22.7, 25.8, 26.6, 29.2, 29.3, 29.4, 29.5, 29.6, 29.63, 29.7, 31.9, 34.6, 36.8, 38.5, 45.1, 122.2, 124.1, 124.4, 125.2, 128.3, 129.3, 129.6, 130.1, 131.3, 132.6, 133.1, 136.0, 136.9, 138.1, 170.2, 171.2, 174.7, 198.8. MS: m/z = 652 (18, M⁺), 140 (64), 127 (100).

### Beispiel 9: 4-[N-[3-[3-Benzoyl-4-[[2-(4-methylphenyl)acetyl]amino]phenylamino]-carbamoyl]buttersäure-N-tetradecylamid

C₄₁H₅₅N₃O₄ (653.91 gmol⁻¹)

Gemäß Allgemeiner Vorschrift 5 aus Tetradecylamin (0.220 g, 1.0 mmol) 3-[N-[3-[3-Benzoyl-4-[[2-(4-methylphenyl)acetyl]amino]phenylamino]carbamoyl]buttersäure (0.460 g, 1.0 mmol). Reinigung: Säulenchromatographie an Kieselgel mit EtAc : n-Hexan 3 :2. Ausbeute: 0.325 g (50%), gelber Feststoff, Fp.: 109°C.

Ber.: C 75.31, H 8.48, N 6.43; Gef.: C, 75.04 H, 8.23 N 6.72.

IR (KBr): ν = 3300, 3060, 2925, 2855, 1655, 1550 cm⁻¹. ¹H-NMR (CDCl₃): δ = 0.81 (t, J=7 Hz, 3H), 1.18 (m, 20H), 1.39 (m, 2H), 1.90 (m, 2H), 2.18 (m, 2H), 2.27 (s, 3H), 2.32 (m, 2H), 3.13 (m, 2H), 3.60 (s, 2H), 5.55 (m, 1H), 7.10 (m, 2H), 7.18 (m, 4H), 7.41 (m, 2H), 7.51 (m, 2H), 7.64 (m, 2H), 7.83 (m, 1H), 8.43 (m, 1H), 10.42 (s, 1H). ¹³C-NMR (CDCl₃): δ = 14.1, 21.1, 22.7, 26.9, 29.2, 29.3, 29.5, 29.56, 29.6, 31.9, 35.0, 36.3, 39.7, 45.0, 122.3, 124.3, 125.1, 128.3, 129.3, 129.6, 130.0, 132.7, 132.8, 138.1, 170.2, 171.1, 172.7, 198.7.

### Beispiel 10: N-[3-Benzoyl-4-[[2-(4-methylphenyl)acetyl]amino]phenyl]eicosansäureamid

### Stufen 1 und 2: s. Beispiel 2

### Stufe 3: N-[3-Benzoyl-4-[[2-(4-methylphenyl)acetyl]amino]phenyl]eicosansäureamid

C₄₂H₅₉N₂O₃ (638.94 gmol⁻¹)

Gemäß Allgemeiner Vorschrift 6 aus N-(4-Amino-2-benzoylphenyl)-2-(4-methylphenyl)acetamid (0.344 g, 1 mmol) und Eicosansäure (0.312 g, 1 mmol). Säulenchromatographie mit Ethylacetat n-Hexan 2 : 3.

Ausbeute: 0.476 g (75%), gelber Feststoff, Fp.: 102°C.

Ber.: C 78.95, H 9.15, N 4.38; Gef.: C, 78.62 H, 8.78 N 4.63.

IR (KBr): ν = 3290, 2920, 2850, 1685, 1655, 1550 cm⁻¹. ¹H-NMR (CDCl₃): δ = 0.81 (t, J=7 Hz, 3H), 1.20 (m, 32H), 1.58 (m, 2H), 2.20 (m, 2H), 2.27 (s, 3H), 3.61 (s, 2H), 7.09 (m, 2H), 7.18 (m, 3H), 7.41 (m, 2H), 7.50 (m, 1H), 7.64 (m, 2H), 7.79 (m, 1H), 8.43 (m, 1H), 10.41 (s, 1H). ¹³C-NMR (CDCl₃): δ = 14.1, 19.0, 21.1, 22.7, 25.8, 29.2, 29.3, 29.4, 29.44, 29.5, 29.6, 29.7, 31.9, 37.6, 45.0, 122.4, 124.3, 124.4, 125.1, 128.3, 129.3, 129.6, 130.0, 132.4, 132.7, 136.2, 137.0, 138.0, 170.3, 171.4, 198.6. MS: m/z = 638 (100, M⁺), 506 (68).

### Beispiel 11: N-[3-[3-Benzoyl-4-[(2-phenylacetyl)amino]phenylamino]-3-oxopropyl]-hexadecansäureamid

### Stufe 1: N-(2-Benzoyl-4-nitrophenyl)-2-phenylacetamid

Gemäß Allgemeiner Vorschrift 1 aus 2-Amino-5-nitrobenzophenon (1.2 g, 5 mmol) und Phenylacetylchlorid (0.8 ml, 5 mmol).

Ausbeute 1.7 g (94%).

¹H-NMR (CDCl₃): δ = 3.79 (s, 2H), 7.37 (m, 2H), 7.52 (m, 3H), 7.64 (m, 3H), 8.15 (m, 1H), 8.25 (m, 1H), 8.41 (m, 1H), 8.46 (m, 1H), 8.98 (m, 1H), 11.08 (s, 1H).

### Stufe 2: N-(4-Amino-2-benzoylphenyl)-2-phenylacetamid

Gemäß Allgemeiner Vorschrift 2 aus N-(2-Benzoyl-4-nitrophenyl)-2-phenylacetamid (1.7 g, 4.7 mmol).

Ausbeute 1.38 g (89%).

¹H-NMR (CDCl₃): δ = 3.53 (s, 2H), 3.61 (s, 2H), 6.68 (m, 1H), 6.79 (m, 1H), 7.18-7.30 (m, 5H), 7.3 6-7.40 (m, 2H), 7.51 (m, 1H), 7.62 (m, 2H), 8.21 (m, 1H), 10.05 (s, 1H).

### Stufe 3: N-[3-[3-Benzoyl-4-[(2-phenylacetyl)amino]phenylamino]-3-oxopropyl]hexadecansäureamid

C₄₀H₅₃N₃O₄ (639.89 gmol⁻¹)

Gemäß Allgemeiner Vorschrift 6 aus N-Hexadodecanoyl-β-alanin (0.490 g, 1.5 mmol) und N-(4-Amino-2-benzoylphenyl)-2-phenylacetamid (0.495 g, 1.5 mmol). Reinigung: Säulenchromatographie an Kieselgel mit 1. Ethylacetat n-Hexan 2:3 und 2. Ethylacetat als Fließmittel.

Ausbeute: 0.85 g (88%), gelber Feststoff, Fp.: 117°C.

Ber.: C 75.08, H 8.35, N 6.57; Gef.: C, 74.77 H, 8.38 N 6.89.

IR (KBr): ν = 3310, 2920, 2850, 1640, 1550 cm⁻¹. ¹H-NMR (CDCl₃): δ = 0.81 (t, J=7 Hz, 3H), 1.17 (m, 24H), 1.45 (m, 2H), 2.02 (t, J=7 Hz, 2H), 2.46 (m, 2H), 3.44 (m, 2H), 3.65 (s, 2H), 6.12 (m, 1H), 7.23 (m, 1H), 7.29 (m, 3H), 7.39 (m, 2H), 7.48-7.57 (m, 2H), 7.62 (m, 2H), 7.77 (m, 1H), 8.43 (m, 2H), 10.45 (s, 1H). ¹³C-NMR (CDCl₃): δ = 14.1, 22.7, 25.6, 29.2, 29.3, 29.4, 29.5, 29.6, 29.62, 29.7, 31.9, 35.2, 36.7, 36.9, 45.4, 122.4, 124.2, 124.3, 125.3, 127.4, 128.3, 128.9, 129.4, 130.0, 132.6, 132.7, 134.2, 136.2, 138.0, 169.7, 170.0, 174.0, 198.6. MS: m/z = 639 (100, M⁺), 330 (69), 312 (67), 212 (73).

### Beispiel 12: N-[2-[3-Benzoyl-4-[[2-(4-methylphenyl)acetyl]amino]phenylamino]-2-oxoethyl]-4-phenylzimtsäureamid

### Stufen 1 und 2: s. Beispiel 2

### Stufe 3: N-[2-[3-Benzoyl-4-[[2-(4-methylphenyl)acetyl]amino]phenylamino]-2-oxoethyl]-4-phenylzimtsäureamid

C₄₀H₃₅N₃O₄ (607.72 gmol⁻¹)

Gemäß Allgemeiner Vorschrift 6 aus N-(4-Phenylcinnamoyl)glycin (0.282 g, 1.0 mmol) und N-(4-Amino-2-benzoylphenyl)-2-(4-methylphenyl)acetamid (0.344 g, 1.0 mmol). Reinigung: Säulenchromatographie an Kieselgel mit 1. Ethylacetat n-Hexan 3:2, 2. Ethylacetat.

Ausbeute: 0.476 g (78%), gelber Feststoff, Fp.: 223°C.

Ber.: C 77.08, H, 5.47 N, 6.92; Gef.: C 76.75, H 5.95, N, 6.66.

IR (KBr): ν = 3275, 3030, 2925, 1655, 1610, 1510, cm⁻¹. ¹H-NMR (DMSO-d₆): δ = 2.22 (s, 3H), 3.33 (s, 2H), 3.98 (m, 2H), 6.77 (d, J=16 Hz, 1H), 6.98 (m, 2H), 7.02 (m, 2H), 7.18 (d, J=16 Hz, 1H), 7.35 (m, 2H), 7.45 (m, 6H), 7.68 (m, 10H), 7.75 (m, 2H), 8.32 (m, 1H), 10.0 (s, 1H), 10.10 (s, 1H). ¹³C-NMR (DMSO-d₆): δ = 20.9, 42.5, 43.0, 119.1, 120.8, 122.2, 122.7, 124.5, 126.8, 126.9, 127.2, 127.4, 128.0, 128.4, 128.5, 128.8, 129.1, 129.2, 129.3, 129.8, 132.1, 132.5, 132.8, 134.3, 134.7, 135.3, 135.7, 137.5, 138.8, 139.7, 139.8, 140.5, 141.4, 165.7, 168.1, 169.3, 195.4.

### Beispiel 13: N-[3-[3-Benzoyl-4-[(2-phenylacetyl)amino]phenylamino]-3-oxopropyl]-4-benzyl-oxyzimtsäureamid

### Stufen 1 und 2: s. Beispiel 11

### Stufe 3: N-[3-[3-Benzoyl-4-[(2-phenylacetyl)amino]phenylamino]-3-oxopropyl]-4-benzyloxyzimtsäureamid

C₄₀H₃₅N₃O₅ (637.74 gmol⁻¹)

Gemäß Allgemeiner Vorschrift 6 aus N-(4-Benzyloxycinnamoyl)-β-alanin (0.44 g, 1.35 mmol) und N-(4-Amino-2-benzoylphenyl)-2-phenylacetamid (0.445 g, 1.35 mmol). Reinigung: Säulenchromatographie an Kieselgel mit 1. Ethylacetat n-Hexan 3:2 2. Ethylacetat als Fließmittel.

Ausbeute: 0.8 g (93%), gelber Feststoff, Fp.: 138°C.

Ber.: C 75.34, H, 5.53 N, 6.59; Gef.: C 74.95, H 5.72, N, 6.22.

IR (KBr): ν = 3300, 3090, 2925, 1710, 1685, 1655, 1615, 1610, 1510, cm⁻¹. ¹H-NMR
(CDCl₃): δ = 2.54 (m, 2H), 3.55 (m, 2H), 3.64 (s, 2H), 4.99 (s, 2H), 6.13 (d, J=16 Hz, 1H), 6.43 (m, 1H), 6.85 (m, 2H), 7.21-7.46 (m, 17H), 7.58 (m, 2H), 7.76 (m, 1H), 8.43 (m, 1H), 8.58 (s, 1H), 10.45 (s, 1H). ¹³C-NMR (CDCl₃): δ = 37.7, 37.1, 45.5, 70.1, 109.4, 115.2117.8, 122.5, 124.3, 124.6, 125.5, 127.4, 127.5, 128.2, 128.4, 128.7, 129.0, 129.5, 130.1, 132.7, 132.8, 134.3, 136.3, 136.6, 138.1, 141.2, 160.3, 167.2, 170.0, 170.1, 198.7. MS: m/z = 637 (0.2, M⁺), 384 (14), 312 (21), 311 (22), 253 (27), 91 (100).

### Beispiel 14: N-[3-Benzoyl-4-[2-(4-methylphenyl)acetyl]amino]phenyl]nicotinsäureamid

### Stufen 1 und 2: s. Beispiel 2

### Stufe 3: N-[3-Benzoyl-4-[2-(4-methylphenyl)acetyl]amino]phenyl]nicotinsäureamid

C₂₈H₂₃N₃O₃ (449.51 gmol⁻¹)

Gemäß Allgemeiner Vorschrift 3 aus Nicotinsäurechlorid (0.142 g, 1.0 mmol) und N-(4-Amino-2-benzoylphenyl)-2-(4-methylphenyl)acetamid (0.344 g, 1.0 mmol). Reinigung: Säulenchromatographie an Kieselgel mit Ethylacetat n-Hexan 3:2.

Ausbeute: 0.098 g (22%), gelber Feststoff, Fp.: 198°C.

Ber.: C 74.82, H, 5.16 N, 9.35; Gef.: C 74.66, H 5.39, N, 9.05.

IR (KBr): ν = 3400, 2925, 1675, 1635, 1595, 1555 cm⁻¹. ¹H-NMR (CDCl₃): δ = 2.26 (s, 3H), 3.60 (s, 2H), 7.08 (m, 2H), 7.15 (m, 2H), 7.30 (m, 1H), 7.41 (m, 2H), 7.51 (m, 1H), 7.58 (m, 1H), 7.64 (m, 2H), 7.91 (m, 1H), 8.14 (m, 1H), 8.47 (m, 1H), 8.65 (s, 1H), 8.97 (s, 1H), 10.44 (s, 1H). ¹³C-NMR (CDCl₃): δ = 21.1, 45.1, 12.6, 123.8, 124.4, 125.1, 125.9, 128.4, 129.3, 129.7, 130.0, 130.3, 131.0, 132.0, 132.8, 135.2, 136.8, 137.1, 138.0, 147.9, 152.6, 163.9, 170.5, 198.4. MS: m/z = 449 (75, M⁺), 317 (100).

### Beispiel 15: N-[3-Benzoyl-4-[2-(4-methylphenyl)acetyl]amino]phenyl]benzoesäureamid

### Stufen 1 und 2: s. Beispiel 2

### Stufe 3: N-[3-Benzoyl-4-[2-(4-methylphenyl)acetyl]amino]phenyl]benzoesäureamid

C₂₉H₂₄N₂O₃ (448.53 gmol⁻¹)

Gemäß Allgemeiner Vorschrift 3 aus Benzoesäurechlorid (0.14 g, 1.0 mmol) und N-(4-Amino-2-benzoylphenyl)-2-(4-methylphenyl)acetamid (0.344 g, 1.0 mmol). Reinigung: Umkristallisation aus Toluol.

Ausbeute: 0.365 g (81%), gelber Feststoff, Fp.: 212°C.

Ber.: C 77.66, H, 5.36 N, 6.25; Gef.: C 77.40, H 5.29, N, 6.38.

IR (KBr): ν = 3420, 1650, 1620, 1550, 1500 cm⁻¹. ¹H-NMR (CDCl₃): δ = 2.34 (s, 3H), 3.66 (s, 2H), 7.14 (m, 2H), 7.22 (m, 2H), 7.40-7.50 (m, 5H), 7.57 (m, 1H), 7.62 (m, 1H), 7.70 (m, 2H), 7.78 (m, 2H), 7.90 (m, 1H), 7.98 (m, 1H), 8.55 (m, 1H), 10.52 (s, 1H). MS: m/z = 448 (96, M⁺), 316 (100).

### Beispiel 16: N-[3-Benzoyl-4-[2-(4-methylphenyl)acetyl]amino]phenyl]-2-phenylessigsäuresäureamid

### Stufen 1 und 2: s. Beispiel 2

### Stufe 3: N-[3-Benzoyl-4-[2-(4-methylphenyl)acetyl]amino]phenyl]-2-phenylessigsäuresäureamid

C₃₀H₂₆N₂O₃ (462.58 gmol⁻¹)

Gemäß Allgemeiner Vorschrift 3 aus Phenylessigsäurechlorid (0.2 ml, 1.5 mmol) und N-(4-Amino-2-benzoylphenyl)-2-(4-methylphenyl)acetamid (0.516 g, 1.5 mmol). Reinigung: Umkristallisation aus Toluol.

Ausbeute: 0.471 g (68%), gelber Feststoff, Fp.: 134°C.

Ber.: C 77.90, H, 5.67 N, 6.06; Gef.: C 77.51, H 5.50, N, 6.48.

IR (KBr): ν = 3290, 1700, 1670, 1630 cm⁻¹. ¹H-NMR (CDCl₃): δ = 2.30 (s, 3H), 3.61 (s, 2H), 3.64 (s, 2H), 7.13 (m, 2H), 7.20 (m, 2H), 7.25-7.45 (m, 6H), 7.57 (m, 1H), 7.43 (m, 2H), 7.55 (m, 1H), 7.65 (m, 2H), 7.87 (m, 1H), 8.43 (m, 1H), 10.44 (s, 1 H). MS: m/z = 462 (100, M⁺), 330 (79).

### Beispiel 17: N-[3-Benzoyl-4-[2-(4-methylphenyl)acetyl]amino]phenyl]-3-phenylpropionsäureamid

### Stufen 1 und 2: s. Beispiel 2

### Stufe 3: N-[3-Benzoyl-4-[2-(4-methylphenyl)acetyl]amino]phenyl]-3-phenylpropionsäureamid

C₃₁H₂₈N₂O₃ (476.58 gmol⁻¹)

Gemäß Allgemeiner Vorschrift 3 aus 3-Phenylpropionsäurechlorid (0.17 ml, 1.0 mmol) und N-(4-Amino-2-benzoylphenyl)-2-(4-methylphenyl)acetamid (0.344 g, 1.0 mmol). Reinigung: Umkristallisation aus Toluol.

Ausbeute: 0.070 g (15%), gelber Feststoff, Fp.: 59°C.

Ber.: C 78.18, H, 5.92 N, 5.88; Gef.: C 78.22, H 5.91, N, 5.78.

IR (KBr): ν = 3420, 3270, 1655, 1595, 1555, 1505 cm⁻¹. ¹H-NMR (CDCl₃): δ = 2.32 (s, 3H), 2.56 (t, J=8 Hz, 2H), 2.96 (t, J=8 Hz, 2H), 3.66 (s, 2H), 7.14 (m, 5H), 7.22 (m, 5H), 7.35 (m, 1H), 7.47 (m, 2H), 7.58 (m, 1H), 7.66 (m, 2H), 7.75 (m, 1H), 8.44 (m, 1H), 10.47 (s, 1H). ¹³C-NMR (CDCl₃): δ = 21.1, 31,5, 39.3, 45.0. 122.4, 124.3, 124.6, 125.4, 126.4, 128.3, 128.4, 128.6, 129.3, 129.6, 130.0, 131.1, 132.2, 132.7, 136.0, 137.0, 138.0, 140.4, 170.3, 170.4, 198.5. MS: m/z = 476 (57, M⁺), 458 (100).

### Beispiel 18: N-[3-Benzoyl-4-[2-(4-methylphenyl)acetyl]amino]phenyl]-3-cyclohexylpropionsäureamid

### Stufen 1 und 2: s. Beispiel 2

### Stufe 3: N-[3-Benzoyl-4-[2-(4-methylphenyl)acetyl]amino]phenyl]-3-cyclohexylpropionsäureamid

C₃₁H₃₄N₂O₃ (482.63 gmol⁻¹)

Gemäß Allgemeiner Vorschrift 3 aus 3-Cyclohexylpropionsäurechlorid (0.17 ml, 1.0 mmol) und N-(4-Amino-2-benzoylphenyl)-2-(4-methylphenyl)acetamid (0.344 g, 1.0 mmol). Reinigung: Umkristallisation aus Toluol.

Ausbeute: g (%), gelber Feststoff, Fp.: 55°C.

Ber.: C 77.15, H, 7.10 N, 5.80; Gef.: C 77.07, H 6.91, N, 5.55.

IR (KBr): ν = 3285, 2925, 2850, 1660, 1550, 1510 cm⁻¹. ¹H-NMR (CDCl₃): δ = 1.14-1.25 (m, 4H), 1.55 (m, 2H), 1.67 (m, 6H), 2.27 (m, 2H), 2.34 (m, 4H), 3.66 (s, 2H). MS: m/z = 482 (100, M⁺), 350 (57).

### Beispiel 19: N-[3-Benzoyl-4-[2-(4-methylphenyl)acetyl]amino]phenyl]-4-phenylbuttersäureamid

### Stufen 1 und 2: s. Beispiel 2

### Stufe 3: N-[3-Benzoyl-4-[2-(4-methylphenyl)acetyl]amino]phenyl]-4-phenylbuttersäureamid

C₃₂H₃₀N₂O₃ (490.61 gmol⁻¹)

Gemäß Allgemeiner Vorschrift 3 aus 4-Phenylbuttersäurechlorid (0.185 mg, 1.0 mmol) und N-(4-Amino-2-benzoylphenyl)-2-(4-methylphenyl)acetamid (0.344 g, 1.0 mmol). Reinigung: Säulenchromatographie Ethylacetat n-Hexan 2:3.

Ausbeute: 0.405 g (83%), gelber Feststoff, Fp.: 117°C.

Ber.: C 78.34, H, 6.16 N, 5.71; Gef.: C 78.42, H 5.84, N, 6.02.

IR (KBr): ν = 3430, 3290, 2945, 1700, 1645 cm⁻¹. ¹H-NMR (CDCl₃): δ = 1.94 (m, 2H), 2.20 (m, 2H), 2.26 (s, 3H), 2.60 (m, 2H), 3.60 (s, 2H), 7.10 (m, 5H), 7.17 (m, 5H), 7.41 (m, 3H), 7.53 (m, 1H), 7.61 (m, 2H), 7.72 (m, 1H), 8.44 (m, 1H), 10.41 (s, 1H). MS: m/z = 490 (100, M⁺), 358 (52).

### Beispiel 20: N-[3-Benzoyl-4-[2-(4-methylphenyl)acetyl]amino]phenyl]-5-phenylvaleriansäureamid

### Stufen 1 und 2: s. Beispiel 2

### Stufe 3: N-[3-Benzoyl-4-[2-(4-methylphenyl)acetyl]amino]phenyl]-5-phenylvaleriansäureamid

C₃₁H₂₈N₂O₃ (504.63 gmol⁻¹)

Gemäß Allgemeiner Vorschrift 3 aus 5-Phenylvaleriansäurechlorid (0.200 mg, 1.0 mmol) und N-(4-Amino-2-benzoylphenyl)-2-(4-methylphenyl)acetamid (0.344 g, 1.0 mmol). Reinigung: Säulenchromatographie Ethylacetat n-Hexan 2:3.

Ausbeute: 0.245 g (49%), gelber Feststoff.

¹H-NMR (CDCl₃): δ = 1.61 (m, 4H), 2.21 (m, 2H), 2.26 (s, 3H), 2.55 (m, 2H), 3.60 (s, 2H), 7.09 (m, 5H), 7.18 (m, 5H), 7.40 (m, 3H), 7.51 (m, 1H), 7.62 (m, 2H), 7.76 (m, 1H), 8.41 (m, 1H), 10.41 (s, 1H).

### Beispiel 21: N-[3-Benzoyl-4-[2-(4-methylphenyl)acetyl]amino]phenyl]zimtsäureamid

### Stufen 1 und 2: s. Beispiel 2

### Stufe 3: N-[3-[3-Benzoyl-4- [2-(4-methylphenyl)acetyl]amino]phenylamino]zimtsäureamid

C₃₁H₂₆N₂O₃ (474.56 gmol⁻¹)

Gemäß Allgemeiner Vorschrift 3 aus Zimtsäurechlorid (0.210 g, 1.0 mmol) und N-(4-Amino-2-benzoylphenyl)-2-(4-methylphenyl)acetamid (0.270 g, 1.0 mmol). Reinigung: Umkristallistion aus Toluol.

Ausbeute: 0.214 g (45%), gelber Feststoff, Fp.: 87°C.

Ber.: C 78.46, H, 5.52 N, 5.90; Gef.: C 78.82, H 5.54, N, 5.86.

IR (KBr): ν = 3440, 3260, 3085, 1665, 1635, 1505 cm⁻¹. ¹H-NMR (CDCl₃): δ = 2.31 (s, 3H), 3.67 (s, 2H), 6.45 (d, J=16 Hz, 1H), 7.15 (m, 3H), 7.22 (m, 3H), 7.34 (m, 3H), 7.46 (m, 4H), 7.56 (m, 2H), 7.68 (m, 2H), 8.01 (m, 1H), 8.51 (m, 1H), 10.51 (s, 1H). MS: m/z = 474 (100, M⁺), 342 (39).

### Beispiel 22: N-[3-Benzoyl-4-[2-(4-methylphenyl)acetyl]amino]phenyl]-4-nitrozimtsäureamid

### Stufen 1 und 2: s. Beispiel 2

### Stufe 3: N-[3-[3-Benzoyl-4- [2-(4-methylphenyl)acetyl]amino]phenylamino]zimtsäureamid

C₃₁H₂₅N₃O₅ (519.56 gmol⁻¹)

Gemäß Allgemeiner Vorschrift 3 aus 4-Nitro-zimtsäurechlorid (0.382 g, 1.5 mmol) und N-(4-Amino-2-benzoylphenyl)-2-(4-methylphenyl)acetamid (0.516 g, 1.5 mmol). Reinigung: Umkristallistion aus Toluol.

Ausbeute: 0.560 g (72%), gelber Feststoff, Fp.: 224°C.

Ber.: C 71.28, H, 4.59 N, 8.31; Gef.: C 71.36, H 4.73, N, 8.31.

IR (KBr): ν = 3430, 3070, 1685, 1665, 1505 cm⁻¹. ¹H-NMR (DMSO-d₆): δ = 2.22 (s, 3H), 3.35 (s, 2H), 6.90 (d, J=18 Hz, 1H), 7.00 (m, 4H), 7.48 (m, 3H), 7.61 (m, 3H), 7.66 (m, 2H), 7.75 (m, 1H), 7.82 (m, 2H), 8.22 (m, 2H), 10.00 (s, 1H), 10.37 (s, 1H). MS: m/z = 519 (68, M⁺), 387 (100), 212 (75).

### Beispiel 23: N-[3-[3-Benzoyl-4- [2-(4-methylphenyl)acetyl]amino]phenylamino]-4-phenylzimtsäureamid

### Stufen 1 und 2: s. Beispiel 2

### Stufe 3: N-[3-[3-Benzoyl-4- [2-(4-methylphenyl)acetyl]amino]phenylamino]-4-phenylzimtsäureamid

C₃₇H₃₀N₂O₃ (550.66 gmol⁻¹)

Gemäß Allgemeiner Vorschrift 3 aus 4-Phenylzimtsäurechlorid (0.282 g, 1.0 mmol) und N-(4-Amino-2-benzoylphenyl)-2-(4-methylphenyl)acetamid (0.270 g, 1.0 mmol). Reinigung: Umkristallistion aus Toluol.

Ausbeute: 0.456 g (83%), gelber Feststoff, Fp.: 200°C.

Ber.: C 80.70, H, 5.49 N, 5.09; Gef.: C 80.53, H 5.36, N, 5.12.

IR (KBr): ν = 3445, 1700, 1685, 1655, 1640, 1550, 1505 cm⁻¹. ¹H-NMR (CDCl₃): δ = 2.24 (s, 3H), 3.60 (s, 2H), 6.42 (d, J=16 Hz, 1H), 7.08 (m, 2H), 7.16 (m, 3H), 7.28 (m,1H), 7.38 (m, 5H), 7.48 (m, 6H), 7.61 (m, 3H), 7.96 (m, 1H), 8.43 (m, 1H), 10.46 (s, 1H). MS: m/z = 550 (98, M⁺), 207 (100).

### Beispiel 24: N-[2-[3-Benzoyl-4-[[2-(4-methylphenyl)acetyl]amino]phenyl]-4-benzyloxyzimtsäureamid

### Stufen 1 und 2: s. Beispiel 2

### Stufe 3: N-[2-[3-Benzoyl-4-[[2-(4-methylphenyl)acetyl]amino]phenyl]-4-benzyloxyzimtsäureamid

C₃₈H₃₂N₂O₄ (580.72 gmol⁻¹)

Gemäß Allgemeiner Vorschrift 3 aus 4-Benzyloxyzimtsäurechlorid (0.327 g, 1.2 mmol) und N-(4-Amino-2-benzoylphenyl)-2-(4-methylphenyl)acetamid (0.413 g, 1.2 mmol). Reinigung: Umkrist. aus n-Hexan/Aceton.

Ausbeute: 0.482 g (69%), gelber Feststoff, Fp.: 183°C.

Ber.:C 78.60, H 5.55, N 4.82. Gef.: C 78.85, H 5.59, N, 4.94.

IR (KBr): ν = 3285, 1675, 1600, 1540 cm⁻¹. ¹H-NMR (CDCl₃): δ = 2.31 (s, 3H), 3.67, (s, 2H), 5.06 (s, 2H), 6.33 (d, J=16 Hz, 1H), 6.91 (m, 2H), 7.15 (m, 2H), 7.24 (m, 2H), 7.34 (m, 9H), 7.55 (m, 2H), 7.60 (d, J=16 Hz, 1H), 7.70 (m, 2H), 7.78,(s, 1H), 8.03 (s, 1H), 8.49 (m, 1H), 10.52 (s, 1H). ¹³C-NMR (CDCl₃): δ = 21.1, 45.1, 70.1, 115.2, 118.0, 122.4, 124.4, 125.2, 128.3, 128.7, 129.3, 129.6, 129.6, 130.0, 131.1, 132.7, 132.9, 136.2, 136.5, 137.0, 138.1, 142.2, 160.4, 164.4, 170.4, 198.7. MS: m/z = 580 (0.3, M⁺), 581 (81), 345 (55), 237 (100).

### Beispiel 25: N-[2-[3-Benzoyl-4-[[2-(4-methylphenyl)acetyl]amino]phenylamino]-2-oxoethyl]-4-benzoesäureamid

### Stufen 1 und 2: s. Beispiel 2

### Stufe 3: N-[2-[3-Benzoyl-4-[[2-(4-methylphenyl)acetyl]amino]phenylamino]-2-oxoethyl]-4-benzoesäureamid

C₃₁H₂₇N₃O₄ (505.61 gmol⁻¹)

Gemäß Allgemeiner Vorschrift 6 aus N-(Benzoyl)glycin (0.18 g, 1.0 mmol) und N-(4-Amino-2-benzoylphenyl)-2-(4-methylphenyl)acetamid (0.344 g, 1.0 mmol). Reinigung: Säulenchromatographie an Kieselgel mit 1. Ethylacetat n-Hexan 3:2, 2. Ethylacetat.

Ausbeute: 0.298 g (59%), gelber Feststoff, Fp.: 196°C.

Ber.: C 73.64, H, 5.39 N, 8.31; Gef.: C 73.40, H 5.23, N, 8.26.

IR (KBr): ν = 3395, 1680, 1640, 1575, 1520 cm⁻¹. ¹H-NMR (DMSO-d₆): δ = 2.25 (s, 3H), 3.36 (s, 2H), 4.04 (m, 2H), 7.00 (m, 5H), 7.02 (m, 2H), 7.47 (m, 5H), 7.60 (m, 2H), 7.65 (m, 2H), 7.79 (m, 1H), 7.87 (m, 2H), 8.68 (m, 1H), 9.99 (s, 1H), 10.11 (s, 1H). ¹³C-NMR (DMSO-d₆): δ = 20.4, 42.1, 43.1, 120.3, 122.2, 124.0, 127.1, 127.9, 128.0, 128.7, 129.3, 130.6, 131.0, 131.6, 132.0, 132.3, 133.8, 134.8, 135.2, 137.0, 166.4, 167.7, 168.8, 194.9. MS: m/z = 505 (67, M⁺), 212 (100).

### Beispiel 26: N-[3-Benzoyl-4-[2-(4-methylphenyl)acetyl]amino]phenyl]naphthalin-1-carbonsäureamid

### Stufen 1 und 2: s. Beispiel 2

### Stufe 3: N-[3-Benzoyl-4-[2-(4-methylphenyl)acetyl]amino]phenyl]benzoesäureamid

C₃₃H₂₆N₂O₃ (498.61 gmol⁻¹)

Gemäß Allgemeiner Vorschrift 3 aus Naphthylin-1-carbonsäurechlorid (0.19 g, 1.0 mmol) und N-(4-Amino-2-benzoylphenyl)-2-(4-methylphenyl)acetamid (0.344 g, 1.0 mmol). Reinigung: Umkristallisation aus Toluol.

Ausbeute: 0.440 g (88%), gelber Feststoff, Fp.: 208°C.

Ber.: C 79.49, H, 5.27 N, 5.62; Gef.: C 79.00, H 5.38, N, 5.64.

¹H-NMR (DMSO-d₆): δ = 2.26 (s, 3H), 3.37 (s, 2H), 7.00 (m, 2H), 7.05 (m, 2H), 7.48 (m, 2H), 7.58 (m, 5H), 7.69 (m, 3H), 7.88 (m, 1H), 8.03 (m, 3H), 8.18 (m, 1H), 10.05 (s, 1H), 10.61 (s, 1H). ¹³C-NMR (DMSO-d₆): δ = 20.4, 42.1, 120.9, 122.8, 124.7, 124.9, 125.3, 126.1, 126.7, 128.0, 128.1, 128.6, 128.7, 129.4, 129.5, 130.0, 132.0, 132.4, 133.0, 134.2, 135.2, 135.3, 137.0, 167.1, 168.9, 194.9. MS: m/z = 498 (100, M⁺), 366 (54), 155 (79).

### Beispiel 27: N-[3-Benzoyl-4-[2-(4-methylphenyl)acetyl]amino]phenyl]anthracen-9-carbonsäureamid

### Stufen 1 und 2: s. Beispiel 2

### Stufe 3: N-[3-Benzoyl-4-[2-(4-methylphenyl)acetyl]amino]phenyl]benzoesäureamid

C₃₇H₂₈N₂O₃ (548.65 gmol⁻¹)

Gemäß Allgemeiner Vorschrift 3 aus Anthracen-9-carbonsäurechlorid (0.36 g, 1.5 mmol) und N-(4-Amino-2-benzoylphenyl)-2-(4-methylphenyl)acetamid (0.516 g, 1.5 mmol). Reinigung: Säulenchromatographie mit Ethylacetat n-Hexan 2:3.

Ausbeute: 0.345 g (42%), gelber Feststoff.

¹H-NMR (DMSO-d₆): δ = 2.28 (s, 3H), 3.60 (s, 2H), 7.10 (m, 2H), 7.17 (m, 3H), 7.42 (m, 5H), 7.48 (m, 1H), 7.66 (m, 1H), 7.70 (m, 3H), 7.93 (m, 2H), 7.98 (m, 3H), 8.42 (s, 1H), 8.52 (m, 1H), 10.46 (s, 1H).

### Beispiel 28: N-[3-Benzoyl-4-[2-(4-methylphenyl)acetyl]amino]phenyl]fluoren-9-carbonsäure-amid

### Stufen 1 und 2: s. Beispiel 2

### Stufe 3: N-[3-Benzoyl-4-[2-(4-methylphenyl)acetyl]amino]phenyl]benzoesäureamid

C₃₆H₂₈N₂O₃ (536.64 gmol⁻¹)

Gemäß Allgemeiner Vorschrift 3 aus Fluoren-9-carbonsäurechlorid (0.34 g, 1.5 mmol) und N-(4-Amino-2-benzoylphenyl)-2-(4-methylphenyl)acetamid (0.516 g, 1.5 mmol). Reinigung: Umkristallisation aus Toluol.

Ausbeute: 0.475 g (42%), gelber Feststoff.

¹H-NMR (DMSO-d₆): δ = 2.22 (s, 3H), 3.32 (s, 2H), 4.96 (s, 1H), 6.95 (m, 2H), 7.01 (m, 2H), 7.29 (m, 2H), 7.37-7.45 (m, 4H), 7.55 (m, 4H), 7.62 (m, 2H), 7.69 (m, 1H), 7.80 (m, 1H), 7.83 (m, 2H), 9.99 (s, I H), 10.67 (s, 1H).

### Beispiel 29: N-[2-[3-Benzoyl-4-[[2-(4-methylphenyl)acetyl]amino]phenyl]-benzylthioglykolsäureamid

### Stufen 1 und 2: s. Beispiel 2

### Stufe 3: N-[2-[3-Benzoyl-4-[[2-(4-methylphenyl)acetyl]amino]phenyl]benzylthioglykolsäureamid

C₃₁H₂₈N₂O₃S (508.68 gmol⁻¹)

Gemäß Allgemeiner Vorschrift 3 aus Benzylthioglykolsäurechlorid (0.241 g, 1.2 mmol) und N-(4-Amino-2-benzoylphenyl)-2-(4-methylphenyl)acetamid (0.413 g, 1.2 mmol). Reinigung: MPLC an Kieselgel mit Ethylacetat/n-Hexan 1:1. Umkrist. aus n-Hexan/Aceton.

Ausbeute: 0.341 g (67%), gelber Feststoff, Fp.: 120°C.

Ber.:C 73.19, H 5.56, N 5.51, S 6.30. Gef.: C 73.10, H 5.53, N, 5.48, S 6.58.

IR (KBr): ν = 3305, 1700, 1680, 1635, 1590, 1510 cm⁻¹. ¹H-NMR (CDCl₃): δ = 2.34 (s, 3H), 3.23 (s, 2H), 3.68, (s, 2H), 3.73 (s, 2H), 7.19 (m, 9H), 7.37 (m, 1H), 7.50 (m, 2H), 7.60 (m, 1H), 7.71 (m, 3H), 8.33 (s, 1H), 8.51 (m, 1H), 10.52 (s, 1H). ¹³C-NMR (CDCl₃): δ = 21.1, 36.4, 37.6, 45.0, 122.2, 124.1, 124.3, 125.1, 127.6, 128.3, 128.8, 128.8, 129.3, 129.6, 130.0, 131.1, 131.7, 132.8, 136.6, 137.0, 138.0, 166.6, 170.2, 198.5. MS: m/z = 508 (100, M⁺), 386 (52), 254 (84), 91 (54).

### Beispiel 30: N-[2-[3-Benzoyl-4-[[2-(4-methylphenyl)acetyl]amino]phenyl]-4-benzyloxyessigsäureamid

### Stufen 1 und 2: s. Beispiel 2

### Stufe 3: N-[2-[3-Benzoyl-4-[[2-(4-methylphenyl)acetyl]amino]phenyl]benzyloxyessigsäureamid

C₃₁H₂₈N₂O₄ (492.61 gmol⁻¹)

Gemäß Allgemeiner Vorschrift 3 aus Benzyloxyessigsäurechlorid (0.185 g, 1.0 mmol) und N-(4-Amino-2-benzoylphenyl)-2-(4-methylphenyl)acetamid (0.344 g, 1.0 mmol). Reinigung: MPLC an Kieselgel mit Ethylacetat/n-Hexan 1:1.

Ausbeute: 0.379 g (77%), braun-gelbes Harz. ,

Ber.: C 75.59, H, 5.73 N, 5.69; Gef.: C 75.40, H 5.85, N, 5.41.

IR (KBr): ν = 3305, 3030, 2920, 1685, 1595, 1510 cm⁻¹. ¹H-NMR (CDCl₃): δ = 2.34 (s, 3H), 3.69 (s, 2H), 4.04 (s, 2H), 4.62 (s, 2H), 7.17 (m, 2H), 7.24 (m, 2H), 7.35 (m, 5H), 7.49 (m, 2H), 7.54 (m, 1H), 7.60 (m, 1H), 7.71 (m, 2H), 7.89 (m 1H), 8.25 (s, 1H), 8.55 (m, 1H), 10.53 (s, 1H). ¹³C-NMR (CDCl₃): δ = 21.1, 45.1, 69.5, 73.9, 122.4, 124.2, 124.5, 125.3, 128.1, 128.4, 128.5, 128.8, 129.3, 129.6,130.1, 131.2, 131.4, 132.8, 136.3, 136.7, 137.0, 138.1, 167.6, 170.3, 198.5. MS: m/z = 492 (100, M⁺), 360 (65), 91(55).

### Beispiel 31: N-[3-Benzoyl-4-[2-(4-methylphenyl)acetyl]amino]phenyl]-4-(4-nitrophenyl)-buttersäureamid

### Stufen 1 und 2: s. Beispiel 2

### Stufe 3: N-[3-Benzoyl-4-[2-(4-methylphenyl)acetyl]amino]phenyl]-4-phenylbuttersäureamid

C₃₂H₃₉N₃O₅ (535.61 gmol⁻¹)

Gemäß Allgemeiner Vorschrift 3 aus 4-(4-Nitrophenyl)buttersäurechlorid (0.340 mg, 1.5 mmol) und N-(4-Amino-2-benzoylphenyl)-2-(4-methylphenyl)acetamid (0.512 g, 1.5 mmol). Reinigung: Umkristallisation aus Toluol.

Ausbeute: 0.330 g (41%), gelber Feststoff.

¹H-NMR (DMSO-d₆): δ = 1.90 (m, 2H), 2.23 (s, 3H), 2.30 (m, 2H), 2.73 (m, 2H), 3.32 (s, 2H), 6.95 (m, 2H), 7.01 (m, 2H), 7.48 (m, 5H), 7.62 (m, 4H), 7.75 (m, 1H), 8.11 (m, 2H), 9.91 (s, 1H), 9.96 (s, 1H).

### Beispiel 32: N-[2-[3-Benzoyl-4-[[2-(4-methylphenyl)acetyl]amino]phenyl]-(4-nitrobenzyl)-thioglykolsäureamid

### Stufen 1 und 2: s. Beispiel 2

### Stufe 3: N-[2-[3-Benzoyl-4-[[2-(4-methylphenyl)acetyl]amino]phenyl]-(4-nitrobenzyl)-thioglykolsäureamid

C₃₁H₂₇N₃O₅S (553.68 gmol⁻¹)

Gemäß Allgemeiner Vorschrift 3 aus 4-Nitrobenzylthioglykolsäurechlorid (0.295 g, 1.2 mmol) und N-(4-Amino-2-benzoylphenyl)-2-(4-methylphenyl)acetamid (0.413 g, 1.2 mmol).

Reinigung: MPLC an Kieselgel mit Ethylacetat/n-Hexan 1:1.

Ausbeute: 0.397 g (60%), gelber Feststoff, Fp.: 60°C.

Ber.: C 67.24, H 4.93, N 7.59, S 5.79. Gef.: C 67.04, H 5.08, N, 7.22, S 5.86.

IR (KBr): ν = 3295, 1665, 1600, 1555, 1515 cm⁻¹. ¹H-NMR (CDCl₃): δ = 2.34, (s, 3H), 3.16, (s, 2H), 3.68, (s, 2H), 3.82 (s, 2H), 7.15 (m, 2H), 7.22 (m, 2H), 7.47 (m, 5H), 7.61 (m, 1H), 7.70 (m, 2H), 7.82 (s, 1H), 8.10 (m, 2H), 8.31 (s, 1H), 8.50 (m, 1H), 10.47 (s, 1H). ¹³C-NMR (CDCl₃): δ = 21.1, 35.8, 36.2, 45.0, 122.5, 123.9, 124.1, 124.4, 125.0, 128.4, 129.3, 129.6, 129.8, 130.0, 131.1, 131.8, 132.8, 136.7, 137.1, 137.9, 144.4, 147.2, 166.4, 170.4, 198.3. MS: m/z = 553 (0.5, M⁺), 554 (85), 421 (100).

### Beispiel 33: N-[3-Benzoyl-4-[(4-methylphenyl)acetylamino]phenyl]-4-propyloxyzimtsäureamid

### Stufen 1 und 2: s. Beispiel 2

### 3. Stufe: N-[3-Benzoyl-4-[(4-methylphenyl)acetylamino]phenyl]-4-propyloxyzimtsäureamid

C₃₄H₃₂N₂O₄ (532.68 gmol⁻¹)

Gemäß Allgemeiner Vorschrift 3 aus 4-Propyloxyzimtsäurechlorid (0.270 g, 1.2 mmol) und N-(4-Amino-2-benzoylphenyl)-2-(4-methylphenyl)acetamid (0.413 g, 1.2 mmol). Reinigung:

MPLC EtOAc:n-Hexan 1:1.

Ausbeute: 0.384 g (60%), hellgelber Feststoff, Fp.: 175°C.

Ber.: C, 76.66; H, 6.07;N,5.26; Gef.: C, 76.26; H, 5.88; N,5.56.

IR (KBr): ν = 3230, 3035, 2965, 1665, 1605, 1550, 1515 cm⁻¹. ¹H-NMR (DMSO-D₆): δ = 0.96 (t, J = 7 Hz, 3H), 1.67-1.77 (m, 2H), 2.26 (s, 3H), 3.37 (s, 2H), 3.97 (t, J = 6 Hz, 2H), 6.60 (d, J = 16 Hz, 1H), 6.98-6.96 (m, 2H), 7.00-7.02 (m, 2H), 7.04-7.06 (m, 2H), 7.50-7.53 (m, 5H), 7.60-7.70 (m, 4H), 7.77 (s, 1H), 7.88 (m, 1H), 10.02 (s, 1H), 10.16 (s, 1H). MS: m/z = 532 (10) [M⁺], 344 (11), 189 (39), 98 (23), 83 (38), 73 (93), 69 (97), 55 (85), 43 (100).

### Beispiel 34: N-[3-Benzoyl-4-[(4-bromphenyl)acetylamino]phenyl]-4-propyloxyzimtsäureamid

### Stufen 1 und 2: s. Beispiel 36

### 3. Stufe: N-[3-Benzoyl-4-[(4-bromphenyl)acetylamino]phenyl]-4-propyloxyzimtsäureamid

C₃₃H₂₉BrN₂O₄ (597.51 gmol⁻¹)

Gemäß Allgemeiner Vorschrift 3 aus 4-Propyloxyzimtsäurechlorid (0.103 g, 0.5 mmol) und N-(4-Amino-2-benzoylphenyl)-2-(4-bromphenyl)acetamid (0.205 g, 0.5 mmol). Reinigung: Umkristallisation aus Ethanol.

Ausbeute: 0.193 g (65%), hellbraune nadelförmige Kristalle, Fp.: 178 °C.

Ber.: C, 66.34; H, 4.89; N, 4.69; Gef.: C, 66.31; H, 4.98; N, 4.77.

IR (KBr): ν = 3428, 3327, 2968, 2938, 1663, 1603, 1550, 1509, 1400, 1285, 1171, 977 cm⁻¹.

¹H-NMR (CDCl₃): δ = 0.96 (t, J=8 Hz, 3H); 1.73 (m, 2H); 3.86 (t, J=7 Hz, 2H); 6.23 (d, J=16 Hz, 1H); 6.80 (m, 2H); 7.16-7.19 (m, 2H); 7.32-7.43 (m, 6H); 7.56 (d, J=16 Hz, 1H); 7.58-7.66 (m, 4H); 7.97 (s, 1H); 8.47 (m , 1H); 10.61 (s, 1H).

MS: m/z = 598 (13, M⁺), 189 (100), 147 (44), 44 (32), 55 (26), 91 (23), 119 (23), 69 (19), 105 (14), 212 (14).

### Beispiel 35: N-[3-Benzoyl-4-[(4-methylphenyl)acetylamino]phenyl]-3-[5-(4-nitrophenyl)-2-furyl]acrylsäureamid

### Stufen 1 und 2: s. Beispiel 2

### 3. Stufe: N-[3-Benzoyl-4-[(4-methylphenyl)acetylamino]phenyl]-3-[5-(4-nitrophenyl)-2-furyl]acrylsäureamid

Gemäß Allgemeiner Vorschrift 3 aus 3-[5-(4-Nitrophenyl)-2-furyl]acrylsäurechlorid (0.333 g, 1.2 mmol) und N-(4-Amino-2-benzoylphenyl)-2-(4-methylphenyl)acetamid (0.413 g, 1.2 mmol). Reinigung: MPLC EtOAc:n-Hexan 2:1.

C₃₅H₂₇N₃O₆ (585.65 gmol⁻¹)

Gemäß Allgemeiner Vorschrift 3 aus 3-[5-(4-Nitrophenyl)-2-furyl]acrylsäure (g, mmol) und N-(4-Amino-2-benzoylphenyl)-2-(4-methylphenyl)acetamid (g, mmol). Reinigung: MPLC EtOAc:n-Hexan 1:1 und Umkristallisation aus n-Hexane:Aceton.

Ausbeute: 0. 311g (48%), gelber Feststoff, Fp.: 221°C.

Ber.: C, 71.77; H, 4.65; N, 7.18; Gef.: C, 71.40; H, 4.69; N, 7.05.

IR (KBr): ν = 3260, 2920, 1665, 1630, 1595, 1550, 1510 cm⁻¹. ¹H-NMR (CDCl₃): δ = 2.33, (s, 3H), 3.69, (s, 2H), 6.59 (d, J=15 Hz, 1H), 6.67-6.68 (m, 1H), 6.90-6.91 (m, 1H), 7.15-7.17 (m, 2H), 7.23-7.25 (m, 2H), 7.45-7.48 (m, 3H), 7.56-7.59 (m, 1H), 7.61-7.64 (m, 1H), 7.70-7.71 (m, 2H), 7.76 (m, 2H), 7.93 (s, br, 1H), 8.03 (s, br, 1H), 8.20 (m, 2H), 8.51 (m, 1H), 10.52 (s, br, 1H).. MS: m/z = 585 (52) [M⁺], 586 (20) [M⁺+1], 583 (15), 453 (18), 345 (17), 344 (71), 326 (18), 325 (20), 243 (15), 242 (100), 238 (15), 212 (80), 211 (29), 196 (22), 105 (39), 40 (11).

### Beispiel 36: N-[3-Benzoyl-4-[(4-bromphenyl)acetylamino]phenyl]-3-[5-(4-nitrophenyl)-2-furyl]acrylsäureamid

### 1. Stufe: N-(2-Benzoyl-4-nitrophenyl)-2-(4-bromphenyl)acetamid

C₂₁H₁₅BrN₂O₄ (439.27 gmol⁻¹)

Gemäß Allgemeiner Vorschrift 1 aus 2-Amino-4-nitrobenzophenon (1.2 g, 5.0 mmol) und 2-(4-Bromphenyl)acetylchlorid (1.167 g, 5.0 mmol). Reinigung: Umkristallisation aus Ethanol. Ausbeute: 1.91 g (87%), gelbe Kristalle, Fp.: 138°C.

Ber.: C, 57.42; H, 3.44; N, 6.38; Gef.: C, 57.71; H, 3.73; N, 6.42.

IR (KBr): ν = 3427, 1716, 1637, 1614, 1580, 1542, 1508 cm⁻¹. ¹H-NMR (CDCl₃): δ = 3.75 (s, 2H), 7.25 (m, 2H), 7.29 (m, 2H), 7.24 (m, 2H), 7.61 (m, 3H), 8.39 (m, 1H), 8.45 (m, 1H), 8.88 (m, 1H), 11.17 (s, 1H). MS: m/z= 59 (100), 43 (77), 72 (51), 45 (46), 41 (44), 269 (13), 198 (11), 81 (11), 408 (2), 438 (1) [M⁺], 440 (0.9) [M⁺+2].

### 2. Stufe: N-(4-Amino-2-benzoylphenyl)-2-(4-bromphenyl)acetamid

C₂₁H₁₇BrN₂O₂ (409.29 gmol⁻¹)

Gemäß Allgemeiner Vorschrift 2 aus *N*-(2-Benzoyl-4-nitrophenyl)-2-(4-bromphenyl)acetamid (1.490 g, 3.8 mmol)

Ausbeute: 1.124 g (82%), gelber Feststoff, Fp.: 172°C.

IR (KBr): ν = 3437, 3280, 2923, 1661, 1595, 1551, 1531, 1501 cm⁻¹. ¹H-NMR (CDCl₃): δ = 3.58 (s, 4H), 6.71 (m, 1H), 6.80 (m, 1H), 7.15 (m, 1H), 7.19 (m, 1H), 7.40 (m, 4H), 7.52 (m, 1H), 7.61 (m, 2H), 8.21 (m, 1H), 10.16 (s, 1H). MS: m/z = 212 (100), 408 (70) [M⁺], 410 (70) [M⁺+2], 211 (50), 409 (17), 41 (16), 239 (16), 213 (15), 105 (14), 169 (9), 171 (8), 210 (8).

### 3. Stufe: N-[3-Benzoyl-4-[(4-bromphenyl)acetylamino]phenyl]-3-[5-(4-nitrophenyl)-2-furyl]-acrylsäureamid

C₃₄H₂₄BrN₃O₆ (650.46 gmol⁻¹)

Gemäß Allgemeiner Vorschrift 3 aus 3-[5-(4-Nitrophenyl)-2-furyl]acrylsäurechlorid (0.277 g, 1.0 mmol) und *N*-(4-Amino-2-benzoylphenyl)-2-(4-bromphenyl)acetylamid (0.409 g, 1.0 mmol). Reinigung: Umkristallisation aus Toluol.

Ausbeute: 0.532 g (82%), gelber Feststoff, Fp.: 237°C.

Ber.: C, 62.78; H, 3.72; N, 6.46; Gef.: C, 63.08; H, 4.10; N, 6.44.

IR (KBr): ν = 3384, 3302, 1680, 1628, 1597, 1553, 1511 cm⁻¹. ¹H-NMR (DMSO-D₆): δ = 3.38 (s, 2H), 6.76 (d, J=16 Hz, 1H), 7.03-7.05 (m, 3H), 7.39-7.42 (m, 4H), 7.45-7.48 (m, 2H), 7.54-7.56 (m, 1H), 7.59-7.62 (m, 1H), 7.65-7.67 (m, 2H), 7.76-7.77 (m, 1H), 7.87-7.89 (m, 1H), 7.98-7.99 (m, 2H), 8.29-8.31 (m, 2H), 10.05 (s, 1H), 10.37 (s, 1H). MS: m/z= 212 (100), 242 (78), 408 (49), 211 (48), 410 (47), 238 (33), 239 (21), 196 (20), 169 (17), 171 (16), 105 (16), 183 (15) 650 (4).

### Beispiel 37: N-[3-Benzoyl-4-[(4-trifluormethylphenyl)acetylamino]phenyl]-3-[5-(4-nitrophenyl)-2-furyl]acrylsäureamid

### 1. Stufe: N-(2-Benzoyl-4-nitrophenyl)-trifluoracetamid

C₁₅H₉F₃N₂O₄ (338.25 gmol⁻¹)

Zu einer Lösung von 2-Amino-4-nitrobenzophenon (1.2 g, 5.0 mmol) in einer Mischung aus trockenem Dichlormethan (8.5 ml pro mmol Amin) und trockenem Pyridin (0.9 ml pro mmol Amin) wird bei 0°C Trifluoracetanhydrid (0.75 ml, 5.0 mmol; 0.15 ml pro mmol Amin) tropfenweise aus einer Spritze zugegeben. Die Reaktionsmischung wird zwei Stunden bei Raumtemperatur gerührt. Anschließend wird die Lösung mit Dichlormethan verdünnt und nacheinander mit. Wasser, gesättigter Natriumhydrogencarbonat-Lösung und gesättigter Natriumchlorid-Lösung gewaschen. Nach dem Trocknen über Natriumsulfat wird das Lösungsmittel am Rotationsverdampfer abdestilliert. Reinigung: Umkristallisation aus Ethanol.

Ausbeute: 1.420 g (83%), weiße Kristalle, Fp.: 135°C.

Ber.: C, 53.27; H, 2.68; N, 8.28; Gef.: C, 53.03; H, 2.91; N, 8.28.

IR (KBr): ν = 3432, 1735, 1643, 1619, 1587, 1558,1522 cm⁻¹. ¹H-NMR (CDCl₃): δ = 7.56 (m, 2H), 7.71 (m, 3H), 8.50 (m, 1H), 8.57 (m, 1H), 8.87 (m, 1H), 12.27 (s, 1H). MS: m/z = 105 (100), 77 (69), 338 (55) [M⁺], 269 (34), 191 (32), 145 (16), 51 (15), 69 (11), 339 (10), 139 (8), 106 (8), 241 (7).

### 2. Stufe: N-(4-Amino-2-benzoylphenyl)-trifluoracetamid

C₁₅H₁₁F₃N₂O₂ (308.26 gmol⁻¹)

Gemäß Allgemeiner Vorschrift 2 aus *N*-(2-Benzoyl-4-nitrophenyl)-trifluoracetamid (1.4 g, 4.1 mmol)

Ausbeute: 1.205 g (95%), gelber Feststoff, Fp.: 108°C.

IR (KBr): ν = 3445, 3389, 3058, 2975, 2874, 1714, 1653, 1595, 1539 cm⁻¹. ¹H-NMR (CDCl₃): δ = 6.83 (m, 1H), 6.87 (m, 1H), 7.43 (m, 2H), 7.55 (m, 1H), 7.65 (m, 2H), 8.32 (m, 1H), 11.47 (s, 1H). MS: m/z = 308 (100) [M⁺], 105 (91), 77 (54), 211 (29), 161 (21), 239 (18), 309 (17), 51 (13), 106 (12), 210 (10), 78 (10), 52 (9).

### 3. Stufe: N-[3-Benzoyl-4-(trifluoracetylamino)phenyl]-3-[5-(4-nitrophenyl)-2-furyl]acrylsäureamid

C₂₈H₁₈F₃N₃O₆ (549.47 gmol⁻¹)

Gemäß Allgemeiner Vorschrift 2 aus 3-[5-(4-Nitrophenyl)-2-furyl]acrylsäurechlorid (0.554 g, 2.0 mmol) und *N*-(4-Amino-2-benzoylphenyl)-trifluoracetamid (0.616 g, 2.0 mmol). Reinigung: Umkristallisation aus Toluol.

Ausbeute: 1.002 g (90%), gelber Feststoff, Fp.: 244°C.

Ber.: C, 61.21; H, 3.30; N, 7.65; Gef.: C, 61.09; H, 3.24; N, 7.31.

IR (KBr): ν = 3426, 1730, 1684, 1626, 1599, 1520 cm⁻¹. ¹H-NMR (DMSO-D₆): δ = 6.74 (d, J=16 Hz, 1H), 7.06 (m, 1H), 7.38 (m, 2H), 7.47 (m, 3H), 7.61 (m, 1H), 7.64 (m, 2H), 7.83 (m, 1H), 7.95 (m, 3H), 8.27 (m, 2H), 10.53 (s, 1H), 11.24 (s, 1H). MS: m/z = 242 (100), 549 (24) [M⁺], 243 (15), 196 (14), 550 (8), 308 (6), 139 (5), 212 (2), 105 (2), 197 (2), 244 (1), 77 (1).

### 4. Stufe: N-(4-Amino-3-benzoylphenyl)-3-[5-(4-nitrophenyl)-2-furyl]acrylsäureamid

C₂₆H₁₉N₃O₅ (453.46 gmol⁻¹)

Das *N*-[3-Benzoyl-4-(trifluoracetylamino)phenyl]-3-[5-(4-nitrophenyl)-2-furyl]acrylsäureamid (0.559 g, 1.0 mmol) wird in einer 1 : 1 Mischung (Vol:Vol) von Dioxan und gesättigter Kaliumcarbonat-Lösung (6 ml pro mmol des geschützten Amins) drei Stunden unter Rückfluß erhitzt. Anschließend wird mit der gleichen Menge Wasser verdünnt und die Mischung dreimal mit Ethylacetat extrahiert. Die vereinigten organischen Phasen werden mit Wasser und gesättigter Natriumchlorid-Lösung gewaschen, über Natriumsulfat getrocknet und das Lösungsmittel am Rotationsverdampfer abdestilliert. Reinigung: Umkristallisation aus Toluol.

Ausbeute: 0.385 g (85%), gelber Feststoff, Fp.: 257°C.

Ber.: C, 68.87; H, 4.22; N, 9.27; Gef.: C, 68.69; H, 4.44; N, 9.28.

IR (KBr): ν = 3487, 1700, 1684, 1622, 1598, 1550, 1511 cm⁻¹. ¹H-NMR (DMSO-D₆): δ = 6.70 (d, J=16 Hz, 1H), 6.86 (m, 2H), 6.95 (m, 1H), 7.32 (d, J=16 Hz, 1H), 7.37 (m, 1H), 7.51 (m, 2H), 7.58 (m, 3H), 7.67 (m, 1H), 7.71 (m, 1H), 7.96 (m, 2H), 8.29 (m, 2H), 9.95 (s, 1H).

MS: m/z = 453 (100) [M⁺], 212 (83), 242 (52), 454 (30), 211 (13), 213 (13), 243 (8), 455 (5), 423 (2), 214 (1), 196 (1), 238 (1).

### 5. Stufe: N-[3-Benzoyl-4-[(4-trifluormethylphenyl)acetylamino]phenyl]-3-[5-(4-nitrophenyl)-2-furyl]acrylsäureamid

C₃₅H₂₄F₃N₃O₆ (639.59 gmol⁻¹)

Gemäß Allgemeiner Vorschrift 1 aus *N*-(4-Amino-3-benzoylphenyl)-3-[5-(4-nitrophenyl)-2-furyl]acrylsäureamid (0.325 g, 0.75 mmol) und 4-Trifluormethylphenylacetylchlorid (0.166 g, 0.75 mmol). Reinigung: Umkristallisation aus Toluol.

Ausbeute: 0.190 g (40%), oranger Feststoff, Fp.: 235°C.

Ber.: C, 65.73; H, 3.78; N, 6.57; Gef.: C, 65.42; H, 4.09; N, 6.46.

IR (KBr): ν = 3426, 1684, 1626, 1598, 1553, 1511 cm⁻¹. ¹H-NMR (DMSO-D₆): δ = 3.52 (s, 2H), 6.77 (d, J=16 Hz, 1H), 7.03 (m, 1H), 7.32 (m, 2H), 7.41 (m, 2H), 7.44 (m, 2H), 7.58 (m, 4H), 7.66 (m, 2H), 7.78 (m, 1H), 7.90 (m, 1H), 7.99 (m, 2H), 8.32 (m, 2H), 10.12 (s, 1H), 10.39 (s, 1H). MS: m/z = 43 (100), 55 (86), 41 (84), 57 (77), 59 (69), 69 (56), 44 (54), 73 (50), 285 (38), 239 (35), 129 (32), 639 (1) [M⁺].

### Beispiel 38: N-[3-Benzoyl-4-[(4-methylphenyl)acetylamino]phenyl]-3-[5-(4-nitrophenyl)-4-thiazolyl]acrylsäureamid

### Stufen 1 und 2: s. Beispiel 2

### 3. Stufe: N-[3-Benzoyl-4-[(4-methylphenyl)acetylamino]phenyl]-3-[5-(4-nitrophenyl)-4-thiazolyl]acrylsäureamid

C₃₄H₂₆N₄O₅S (602.71 gmol⁻¹)

Gemäß Allgemeiner Vorschrift 3 aus 3-[5-(4-Nitrophenyl)-4-thiazolyl]acrylsäurechlorid (0.354 g, 1.2 mmol) und N-(4-Amino-2-benzoylphenyl)-2-(4-methylphenyl)acetamid (0.413 g, 1.2 mmol). Reinigung: MPLC EtOAc:n-Hexan 2:1.

Ausbeute:0.376 g (52%), gelber Feststoff, Fp.: 243°C.

Ber.: C, 67.75; H, 4.36; N, 9.30; S, 5.32; Gef.: C, 67.48; H, 4.46; N, 8.98; S, 5.37.

IR (KBr): ν = 3335, 3085, 2920, 1685, 1660, 1635, 1595, 1550, 1515 cm⁻¹. ¹H-NMR = (CDCl₃): δ = 2.34, (s, 3H), 3.71, (s, 2H), 7.01 (d, J=16 Hz, 1H), 7.17-7.18 (m, 2H), 7.24-7.28 (m, 4H), 7.44-7.54 (m, 3H), 7.59-7.68 (m, 2H), 7.72-7.74 (m, 2H), 8.00 (s, br, 1H), 8.12-8.14 (m, 2H), 8.28-8.32 (m, 2H), 8.57-8.59 (m, 1H), 10.54 (s, br, 1H). MS: m/z = 602 (5) [M⁺], 345 (17), 344 (79), 259 (14), 239 (20), 238 (13), 213 (22), 212 (100), 211 (47), 142 (13), 105 (42), 44 (11), 40 (24).

### Beispiel 39: N-[3-Benzoyl-4-[(4-methylphenyl)acetylamino]phenyl]-3-[5-(4-bromphenyl)-2-furyl]acrylsäureamid

### Stufen 1 und 2: s. Beispiel 2

### 3. Stufe: N-[3-Benzoyl-4-[(4-methylphenyl)acetylamino]phenyl]-3-[5-(4-bromphenyl)-2-furyl]acrylsäureamid

C₃₅H₂₇BrN₂O₄ (619.54 gmol⁻¹)

Gemäß Allgemeiner Vorschrift 3 aus 3-[5-(4-Bromphenyl)-2-furyl]acrylsäurechlorid (0.355 g, 1.2 mmol) und N-(4-Amino-2-benzoylphenyl)-2-(4-methylphenyl)acetamid (0.413 g, 1.2 mmol). Reinigung: MPLC EtOAc:n-Hexan 1:1.

Ausbeute: 0.468g (63%), gelber Feststoff, Fp.: 237°C.

Ber.: C, 67.85; H, 4.40; Br, 12.90; N, 4.52; Gef.: C, 67.79; H, 4.52; Br, 12.69; N, 4.58.

IR (KBr): ν = 3300, 3045, 2360, 1685, 1660, 1635, 1595, 1550, 1505 cm⁻¹. ¹H-NMR (CDCl₃): δ = 2.33, (s, 3H), 3.68, (s, 2H), 6.43 (d, J=15 Hz, 1H), 6.62-6.64 (m, 1H), 6.68-6.70 (m, 1H), 7.13-7.16 (m, 2H), 7.21-7.25 (m, 3H), 7.30 (s, br, 1H), 7.42-7.62 (m, 8H), 7.71-7.72 (m, 2H), 7.94 (s, br, 1H), 8.54 (d, J=9 Hz, 1H), 10.46 (s, br, 1H). MS: m/z = 619 (9) [M⁺], 620 (34) [M⁺+1], 618 (26), 607 (26), 344 (44), 277 (100), 275 (88), 265 (40), 212 (24), 105 (23), 40 (46).

### Beispiel 40: N-[3-Benzoyl-4-[(4-methylphenyl)acetylamino]phenyl]-3-[5-(4-chlorphenyl)-2-furyl]acrylsäureamid

### Stufen 1 und 2: s. Beispiel 2

### 3. Stufe: N-[3-Benzoyl-4-[(4-methylphenyl)acetylamino]phenyl]-3-[5-(4-chlorphenyl)-2-furyl]acrylsäureamid

C₃₅H₂₇ClN₂O₄ (575.09 gmol⁻¹)

Gemäß Allgemeiner Vorschrift 3 aus 3-[5-(4-Chlorphenyl)-2-furyl]acrylsäurechlorid (0.321 g, 1.2 mmol) und N-(4-Amino-2-benzoylphenyl)-2-(4-methylphenyl)acetamid (0.413 g, 1.2 mmol). Reinigung: MPLC EtOAc:n-Hexan 1:1.

Ausbeute: 0.493g (71%), gelber Feststoff, Fp.: 240°C.

Ber.: C, 73.10; H, 4.73; Cl, 6.16; N, 4.87; Gef.: 72.92; H, 4.82; Cl, 6.00; N, 4.96.

IR (KBr): ν = 3320, 1685, 1660, 1635, 1595, 1555, 1505 cm⁻¹. ¹H-NMR (DMSO-D₆): δ = 2.26, (s, 3H), 3.38, (s, 2H), 6.70 (d, J=15 Hz, 1H), 6.94-6.95 (m, 1H), 7.00-7.02 (m, 2H), 7.04-7.06 (m, 2H), 7.12-7.13 (m, 1H), 7.38 (d, J= 15 Hz, 1H), 7.48-7.55 (m, 4H), 7.62-7.65 (m, 2H), 7.69-7.70 (m, 2H), 7.77-7.79 (m, 3H), 7.88-7.90 (m, 1H), 10.03 (s, br, 1H), 10.30 (s, br, 1H). MS: m/z = 575 (29) [M⁺], 576 (29) [M⁺+1], 577 (10) [M⁺+2], 574 (72), 345 (12), 344 (48), 233 (33), 232 (15), 231 (100), 212 (16).

### Beispiel 41: N-[3-Benzoyl-4-[[2-(4-methyl-1-piperazinyl)-2-phenylacetyl]amino]phenyl]-3-[5-{4-nitrophenyl)-2-furyl]acrylsäureamid

### 1. Stufe: N-(2-Benzoyl-4-nitrophenyl)-2-chlor-2-phenylacetamid

C₂₁H₁₅ClN₂O₄ (394.82 gmol⁻¹)

Gemäß Allgemeiner Vorschrift 1 aus 2-Amino-4-nitrobenzophenon (1.2 g, 5.0 mmol) und 2-Chlor-2-phenylacetylchlorid (0.945 g, 5.0 mmol). Reinigung: Umkristallisation aus Ethanol.

Ausbeute: 1.658 g (84%), gelbe Kristalle, Fp.: 124°C.

Ber.: C, 63.89; H, 3.83; N, 7.10; Gef.: C, 63.80; H, 3.89; N, 7.45.

IR (KBr): ν = 3421, 1691, 1653, 1644, 1616, 1579, 1539, 1507 cm⁻¹. ¹H-NMR (CDCl₃): δ = 5.44 (s, 1H), 7.32 (m, 3H), 7.49 (m, 4H), 7.63 (m, 1H), 7.66 (m, 2H), 8.35 (m, 1H), 8.44 (m, 1H), 8.81 (m, 1H), 12.01 (s, 1H). MS: m/z= 269 (100), 270 (18), 191 (13), 105 (4), 118 (2), 271 (2), 192 (1), 394 (1) [M⁺], 223 (1), 125 (1), 145 (1), 226 (1).

### 2. Stufe: N-(2-Benzoyl-4-nitrophenyl)-2-(4-methyl-1-piperazinyl)-2-phenylacetamid

C₂₆H₂₆N₄O₄ (458.52 gmol⁻¹)

*N*-(2-Benzoyl-4-nitrophenyl)-2-chlor-2-phenylacetamid (0.945 g, 2.4 mmol) wird in 40 ml Acetonitril gelöst und nach Zugabe von drei Äquivalenten Methylpiperazin (80 ml, 7.2 mmol) wird die Reaktionsmischung 20 Stunden zum Sieden erhitzt. Nach dem Entfernen vom Acetonitril am Rotationsverdampfer wird der Feststoff in Dichlormethan aufgenommen und mit einer Kaliumcarbonat-Lösung gewaschen, anschließend über Natriumsulfat getrocknet und am Rotationsverdampfer vom Lösungsmittel befreit. Reinigung: Umkristallisation aus Ethanol. [*J. Med. Chem*. **23**, (1980), 721]

Ausbeute: 0.693 g (63%), rötliche Feststoff, Fp.: 78°C.

Ber.: C, 68.11; H, 5.72; N, 12.22; Gef.: C, 67.93; H, 6.09; N, 12.19.

IR (KBr): ν = 3425, 1698, 1641, 1612, 1597, 1578, 1525, 1502 cm⁻¹. ¹H-NMR (CDCl₃): δ = 2.26 (s, 3H), 2.53 (s, 8H), 4.01 (s, 1H), 7.29 (m, 3H), 7.35 (m, 2H), 7.55 (m, 2H), 7.68 (m, 1H), 7.77 (m, 2H), 8.34 (m, 1H), 8.43 (m, 1H), 8.85 (m, 1H), 12.09 (s, 1H). MS: m/z = 189 (100), 40 (52), 59(20), 91 (20), 44 (17), 105 (16), 70(15), 190 (14), 57 (13), 41 (11), 77 (9), 458 (5) [M⁺].

### 3. Stufe: N-(4-Amino-2-benzoylphenyl)-2-(4-methyl-1-piperazinyl)-2-phenylacetamid C₂₆H₂₈N₄O₂ (428.54 gmol⁻¹)

Gemäß Allgemeiner Vorschrift 2 aus *N*-(2-Benzoyl-4-nitrophenyl)-2-(4-methyl-1-piperazinyl)-2-phenylacetamid (0.553 g, 1.2 mmol).

Ausbeute: 0.439 g (85%), rötlich ölige Substanz.

IR (KBr): ν = 3369, 2945, 2833, 1712, 1634, 1616, 1597, 1512 cm⁻¹. ¹H-NMR (CDCl₃): δ = 2.00 (s, 8H), 2.65 (s, 3H), 3.98 (s, 1H), 6.75 (m, 1H), 6.82 (m, 1H), 7.24 (m, 3H), 7.29 (m, 2H), 7.45 (m, 2H), 7.56 (m, 1H), 7.67 (m, 1H), 8.26 (m, 1H), 11.39 (s, 1H). MS: m/z = 189 (100), 91 (27), 190 (25), 70 (22), 44 (19), 59 (19), 43 (17), 105 (16), 57 (14), 55 (13), 43 (13), 428 (10) [M⁺].

### 4. Stufe: N-[3-Benzoyl-4-[[2-(4-methyl-1-piperazinyl)-2-phenylacetyl]amino]phenyl]-3-[5-(4-nitrophenyl)-2-furyl]acrylsäureamid

C₃₉H₃₅N₅O₆ (669.74 gmol⁻¹)

Gemäß Allgemeiner Vorschrift 3 aus 3-[5-(4-Nitrophenyl)-2-furyl]acrylsäurechlorid (0.277 g, 1.0 mmol) und *N*-(4-Amino-2-benzoylphenyl)-2-(4-methyl-1-piperazinyl)-2-phenylacetamid (0.428 g, 1.0 mmol). Reinigung: Umkristallisation aus Toluol.

Ausbeute: 0.152 g (23%), gelber Feststoff, Fp.: 160°C.

Ber.: C, 69.94; H, 5.27; N, 10.46; Gef.: C, 70.07; H, 5.60; N, 10.43.

IR (KBr): ν = 3426, 1682, 1630, 1598, 1547, 1510 cm⁻¹. ¹H-NMR (DMSO-D₆): δ = 2.28 (s, 8H), 2.71 (s, 3 H), 4.19 (s, 1H), 6.79 (d, J=16 Hz, 1H), 7.02-7.03 (m, 1H), 7.11-7.17 (m, 2H), 7.21-7.25 (m, 4H), 7.31-7.33 (m, 4H), 7.40-7.41 (m, 2H), 7.53-7.56 (m, 2H), 7.75-7.77 (m, 2H), 7.98-8.00 (m, 2H), 8.30-8.32 (m, 2H), 10.51 (s, 1H), 10.88 (s, 1H). MS: m/z = 189 (100), 190 (15), 105 (8), 91 (7), 77 (4), 70 (3), 212 (3), 42 (3), 44 (2), 43 (2), 428 (2), 58 (2).

Die Aktivität der Substanzen wird in einem Versuchssystem bestimmt. Dieses System beruht auf der Inhibition des Wachstums von Parasiten, Bakterien, Viren und Pilzen in vitro.

Zum Beispiel wird zur Bestimmung der Antimalaria-Aktivität die Inhibition des Wachstums von Malaria-Parasiten in Blutkulturen bestimmt.

Einige der Mikroorganismen, die untersucht werden sollen, können nur in Tiermodellen untersucht werden. Hier werden die entsprechenden Modelle angewendet.

Substanzen, die eine Wirksamkeit in den in vitro Meßsystemen zeigen, werden in in vivo Modellen weiter untersucht. Die antiparasitäre, antivirale, fungizide oder antibakterielle Aktivität wird in den entsprechenden Tiermodelle weiter evaluiert.

### Beispiel 42 zur Wirksamkeit

Die Antimalaria-Wirksamkeit der Verbindungen 1 bis 32 wurde an in-vitro-Kulturen des Malaria-Erregers Plasmodium falciparum bestimmt. Die Vertiefungen einer 96-well- Mikrotiterplatte wurden mit je 200 µl einer asynchronen Plasmodium falciparum-Kultur bei 0.4 % Parasitämie und 2 % Hämatokrit beschickt. Dann wurde eine serielle Verdünnungsreihe der Verbindungen bei Konzentrationen von 100, 10 und 1 µmol l⁻¹ hergestellt. Die Platten werden bei 37°C, 3 % CO₂ und 5 % O₂ über einen Zeitraum von 48 Stunden inkubiert. Dann wurden zu jedem well 30 µl Medium supplementiert mit 27 µCi ml⁻¹ [³H]-Hypoxanthin zugefügt. Nach 24-stündiger Inkubation wurden die Parasiten durch Filtration auf Glasfaserfilter geemtet und die incorporierte Radioaktivität gemessen. Die Inhibition des Parasitenwachstums wurde als prozentuale Inhibition der Tritiumcorporation bezogen auf einen Vergleich ohne Substanz ausgedrückt. Die Tabelle gibt die prozentuale Inhibition in Abhängigkeit der Konzentrationen an.

**Tabelle I:**

| | | % Hemmung bei | | |
|---|---|---|---|---|
| | | 100 µM | 10 µM | 1 µM |
| Beispiel 1 | 2-[N-[3-[3-Benzoyl-4-(2-phenylpropionyl)amino]-phenylamino]carbamoyl]essigsäuremethylester | 89 | 19 | -- |
| Beispiel 2 | 2-[N-[3-[3-Benzoyl-4-[[2-(4-methylphenyl)acetyl]-amino]phenylamino]carbamoyl]essigsäure-methylester | 95 | 19 | -- |
| Beispiel 3 | 3-[N-[3-[3-Benzoyl-4-[[2-(4-methylphenyl)acetyl]-amino]phenylamino]carbamoyl]propionsäure | 38 | 0 | -- |
| Beispiel 4 | 3-[N-[3-[3-Benzoyl-4-[[2-(4-methylphenyl)acetyl]-amino]phenylamino]carbamoyl]propionsäure-methylester | 29 | 0 | -- |
| Beispiel 5 | 4-[N-[3-[3-Benzoyl-4-[[2-(4-methylphenyl)acetyl]-amino]phenylamino]carbamoyl]buttersäure | 59 | 0 | -- |
| Beispiel 6 | 4-[N-[3-[3-Benzoyl-4-[[2-(4-methylphenyl)acetyl]-amino]phenylamino]carbamoyl]buttersäure-methylester | 98 | 0 | -- |
| Beispiel 7 | N-[2-[3-Benzoyl-4-[[2-(4-methylphenyl)acetyl]-amino]phenylamino]-2-oxoethyl]heptadecan-säureamid | 83 | 0 | -- |
| Beispiel 8 | N-[4-[3-Benzoyl-4-[[2-(4-methylphenyl)acetyl]-amino]phenylamino]-4-oxobutyl]pentadecan-säureamid | 93 | 0 | -- |
| Beispiel 9 | 4-[N-[3-[3-Benzoyl-4-[[2-(4-methylphenyl)acetyl]-amino]phenylamino]carbamoyl]buttersäure-N-tetradecylamid | 96 | 0 | -- |
| Beispiel 10 | N-[3-Benzoyl-4-[[2-(4-methylphenyl)acetyl]-amino]phenyl]eicosansäureamid | 99 | 0 | -- |
| Beispiel 11 | N-[3-[3-Benzoyl-4-[(2-phenylacetyl)amino]-phenylamino]-3-oxopropyl]hexadecansäureamid | 98 | 0 | -- |
| Beispiel 12 | N-[2-[3-Benzoyl-4-[[2-(4-methylphenyl)acetyl]-amino]phenylamino]-2-oxoethyl]-4-phenylzimt-säureamid | 89 | 67 | -- |
| Beispiel 13 | N-[3-[3-Benzoyl-4-[(2-phenylacetyl)amino]-phenylamino]-3-oxopropyl]-4-benzyloxyzimt-säureamid | 95 | 97 | -- |
| Beispiel 14 | N-[3-Benzoyl-4-[2-(4-methylphenyl)acetyl]amino]-phenyl]nicotinsäureamid | 34 | 0 | -- |
| Beispiel 15 | N-[3-Benzoyl-4-[2-(4-methylphenyl)acetyl]amino]-phenyl]benzoesäureamid | 96 | 77 | 0 |
| Beispiel 16 | N-[3-Benzoyl-4-[2-(4-methylphenyl)acetyl]amino]-phenyl]-2-phenylessigsäuresäureamid | 94 | 0 | 0 |
| Beispiel 17 | N-[3-Benzoyl-4-[2-(4-methylphenyl)acetyl]amino]-phenyl]-3-phenylpropionsäureamid | 94 | 91 | 0 |
| Beispiel 18 | N-[3-Benzoyl-4-[2-(4-methylphenyl)acetyl]amino]-phenyl]-3-cyclohexylpropionsäureamid | 98 | 98 | 9 |
| Beispiel 19 | N-[3-Benzoyl-4-[2-(4-methylphenyl)acetyl]amino]-phenyl]-4-phenylbuttersäureamid | 97 | 95 | 0 |
| Beispiel 20 | N-[3-Benzoyl-4-[2-(4-methylphenyl)acetyl]amino]-phenyl]-5-phenylvaleriansäureamid | 97 | 94 | 0 |
| Beispiel 21 | N-[3-Benzoyl-4-[2-(4-methylphenyl)acetyl]amino]-phenyl]zimtsäureamid | 94 | 95 | 0 |
| Beispiel 22 | N-[3-Benzoyl-4-[2-(4-methylphenyl)acetyl]amino]-phenyl]-4-nitrozimtsäureamid | 88 | 82 | 0 |
| Beispiel 23 | N-[3-[3-Benzoyl-4-[2-(4-methylphenyl)acetyl]amino]-phenylamino]-4-phenylzimtsäureamid | 94 | 94 | 82 |
| Beispiel 24 | N-[2-[3-Benzoyl-4-[[2-(4-methylphenyl)acetyl]-amino]phenyl]- 4-benzyloxyzimtsäureamid | 97 | 96 | 85 |
| Beispiel 25 | N-[2-[3-Benzoyl-4-[[2-(4-methylphenyl)acetyl]-amino]phenylamino]-2-oxoethyl]-4-benzoesäure-amid | 86 | 0 | 0 |
| Beispiel 26 | N-[3-Benzoyl-4-[2-(4-methylphenyl)acetyl]amino]-phenyl]naphthalin-1-carbonsäureamid | 93 | 65 | 0 |
| Beispiel 27 | N-[3-Benzoyl-4-[2-(4-methylphenyl)acetyl]amino]-phenyl]anthracen-9-carbonsäureamid | | | |
| Beispiel 28 | N-[3-Benzoyl-4-[2-(4-methylphenyl)acetyl]amino]-phenyl]fluoren-9-carbonsäureamid | 98 | 0 | 0 |
| Beispiel 29 | N-[2-[3-Benzoyl-4-[[2-(4-methylphenyl)acetyl]-amino]phenyl]benzylthioglykolsäureamid | 97 | 91 | 0 |
| Beispiel 30 | N-[2-[3-Benzoyl-4-[[2-(4-methylphenyl)acetyl]-amino]phenyl]-4-benzyloxyessigsäureamid | 97 | 93 | 0 |
| Beispiel 31 | N-[3-Benzoyl-4-[2-(4-methylphenyl)acetyl]amino]-phenyl]-4-(4-nitrophenyl)buttersäureamid | 96 | 93 | 0 |
| Beispiel 32 | N-[2-[3-Benzoyl-4-[[2-(4-methylphenyl)acetyl]-amino]phenyl]-(4-nitrobenzyl)thioglykolsäureamid | 96 | 0 | 0 |

## Patentansprüche

1. Verwendung mindestens eines 2-Phenylendiaminderivats der allgemeinen Formel (I): 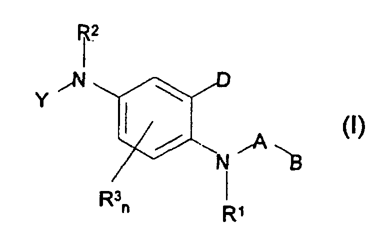 worin
n = 0 - 3;
R¹, R² = H, C₁₋₂₆-Alkyl, Aryl, Heteroaryl, C₁₋₂₆-Acyl;
R³ = H, Halogen, C₁₋₂₆-Alkyl, Aryl, Heteroaryl, Ar- C₁₋₂₆-alkyl, C₁₋₂₆-Acyl, CN, NO₂, R⁴-X-;
mit R⁴ = H, C₁₋₂₆-Alkyl, Aryl, Heteroaryl, Ar- C₁₋₂₆-alkyl, C₁₋₂₆-Acyl;
X = NH, O, S, SO₂, NHSO₂, OSO₂,
A = CH₂, CHR⁵, CR⁵R⁶, CO, CS, CONR⁴, CSNR⁴, SO₂, PO₂,
R⁵, R⁶ = unabhängig voneinander C₁₋₂₆-Alkyl, Aryl, Heteroaryl, Ar- C₁₋₂₆-alkyl, CN, NO₂, COR⁷,
R⁷ = H, C₁₋₂₆-Alkyl, Aryl, Ar- C₁₋₂₆-alkyl, C₁₋₂₆-Alkoxy, Aryloxy, Ar- C₁₋₂₆-alkoxy, NR⁸R⁹,
R⁸, R⁹ = unabhängig voneinander H, C₁₋₂₆-Alkyl, Aryl, Ar- C₁₋₂₆-alkyl, Heteroaryl,
B = C₁₋₂₆-Alkyl, Aryl, Heteroaryl, Arylcarbonyl, gesättigte Heterocyclen, substituiertes Alkyl mit 1-4 Kettenglieder, wobei als Substituenten C₁₋₉-Alkyl, Aryl, Heteroaryl, Halogen, =O, OH, NH₂, NH-CO-R¹⁰, NH-SO₂-R¹⁰, COOR¹¹, CO-NR¹²R¹³, CS-NR¹⁴R¹⁵, SO₂OR¹⁶, SO₂NR¹⁷R¹⁸, NH-CO-OR¹⁹, NH-CO-NR²⁰R²¹, NHCSNR²²R²³ auftreten können,
R¹⁰ - R²³ = unabhängig voneinander H, C₁₋₂₆-Alkyl, Aryl, Ar- C₁₋₂₆-alkyl, Heteroaryl,
D = H, C₁₋₂₆-Alkyl, Aryl, Heteroaryl, Ar- C₁₋₂₆-alkyl, -Y-R²⁴, Halogen, NO₂, CN, NH-CO-R²⁵, NH-SO₂-R²⁶, NH-CO-OR²⁷, NH-CO-NR²⁸R²⁹, NH-CS-NR³⁰R³¹,
Y= O, NH, S, CO, CS, SO₂, COO, CONR³¹, CSNR³², SO₂NR³³,
R²⁴ - R³³ = unabhängig voneinander H, C₁₋₂₆-Alkyl, Aryl, Heteroaryl, Ar- C₁₋₂₆-alkyl bedeuten, und
Y = eine Gruppe ausgewählt aus 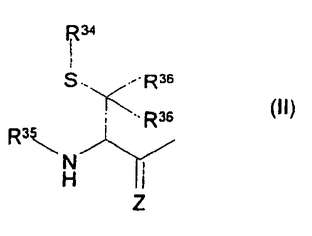 worin
Z = O, S oder zwei Wasserstoffatome,
R³⁴ = H, C₁₋₂₆-Alkyl, Aryl, Ar- C₁₋₂₆-alkyl, COOR³⁷, Arylsulfonyl,
R³⁵ = H, C₁₋₂₆-Acyl, COOR³⁸,
R³⁶ = unabhängig voneinander H, C₁₋₂₆-Alkyl,
R³⁷, R³⁸ = unabhängig voneinander C₁₋₂₆-Alkyl, Aryl, Ar- C₁₋₂₆-alkyl bedeuten,
oder 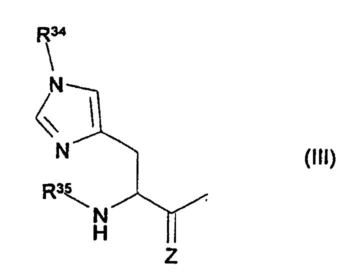 worin
Z = O, S oder zwei Wasserstoffatome,
R³⁴ = H, C₁₋₂₆-Alkyl, Aryl, Ar- C₁₋₂₆-alkyl, COOR³⁷, Arylsulfonyl,
R³⁵ = H, C₁₋₂₆-Acyl, COOR³⁸
R³⁷, R³⁸ = unabhängig voneinander C₁₋₂₆-Alkyl, Aryl, Ar- C₁₋₂₆-alkyl bedeuten, oder 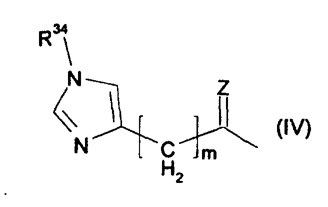 worin
Z = O, S oder zwei Wasserstoffatome,
m=0-3
R³⁴ = H, C₁₋₂₆-Alkyl, Aryl, Ar- C₁₋₂₆-alkyl, COOR³⁷, Arylsulfonyl,
R³⁷ = C₁₋₂₆-Alkyl, Aryl, Ar- C₁₋₂₆-alkyl bedeuten,
oder 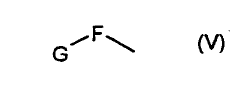 worin
F = CH₂, CO, CS, SO₂,
G = COOR³⁹, CONHOH, CONR⁴⁰R⁴¹, CSNR⁴²R⁴³, C₁₋₂₆-alkyl- oder arylsubstituiertes Alkyl mit 1-3 Kettengliedern oder Alkyl mit 1-3 Kettengliedern, das am terminalen C-Atom einen Substituenten ausgewählt aus COOR⁴⁴, CONHOH, CONR⁴⁵R⁴⁶, CSNR⁴⁷R⁴⁸, SR⁴⁹, SOR⁵⁰, SO₂R⁵¹, SO₂NR⁵²R⁵³, PO(OR⁵⁴)OR⁵⁵, PO(OR⁵⁶)NR⁵⁷₂, OSO₂R⁵⁸, O(PO)OR⁵⁹, NHSO₂R⁶⁰, NHPO₂R⁶¹, NHCOR⁶², NHCSR⁶³, NHCONR⁶⁴R⁶⁵, NHCSNR⁶⁶R⁶⁷, -S(NH)₂-R⁶⁸ oder NH(C=NR⁷⁰)NHR⁶⁹ trägt, ferner Aryl oder Heteroaryl, R³⁹ - R⁶⁹ = unabhängig voneinander H, C₁₋₂₆-Alkyl, Aryl,
R⁷⁰ = CONH₂, SO₂NH₂ bedeuten,
oder 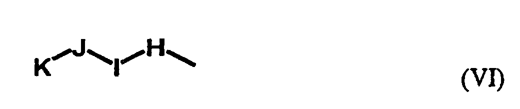 ist, worin
H = CH₂, CO, CS, CHR⁷¹, CR⁷²R⁷³, SO₂, SO, PO₂,
I = C₁₋₂₆-Alkylen, C₁₋₂₆-Alkylen, bei dem eine Methylengruppe durch O, S, oder NR⁷⁷ ersetzt ist, oder C₂₋₂₆-Alkenylen, wobei diese Alkylen- bzw. Alkenylenreste unsubstituiert oder durch Aryl, Heteroaryl, Halogen, OH, CN, C₁₋₉-Alkyloxy, Aryloxy, COOR⁷⁴, CONR⁷⁵R⁷⁶, NR⁷⁷R⁷⁸, NH(C=NR⁷⁰)NHR⁶⁹, SR⁷⁹, SO₂R⁸⁰ substituiert sind, ferner C₃₋₈-Cycloalkylen, unsubstituiert oder durch Aryl, Heteroaryl, Halogen, OH, CN, C₁₋₉-Alkyloxy, Aryloxy, COOR⁷⁴, CONR⁷⁵R⁷⁶, NR⁷⁷R⁷⁸, SR⁷⁹, SO₂R⁸⁰ substituiert, C₃₋₈-Cycloalkylen, worin die Alkylenkette durch O, S oder NR⁷⁷ unterbrochen ist, Arylen, unsubstituiert oder durch Aryl, Heteroaryl, Halogen, OH, CN, C₁₋₂₆-Alkoxy, Aryloxy, COOR⁷⁴, CONR⁷⁵R⁷⁶, NR⁷⁷R⁷⁸, SR⁷⁹, SO₂R⁸⁰ substituiert,
R⁷¹ - R⁸⁰ unabhängig voneinander H, C₁₋₂₆-Alkyl, Aryl, Ar- C₁₋₂₆-alkyl, Heteroaryl,
J = eine Bindung oder CO, COO, CONR⁸¹, CS, CSNR⁸², SO₂, S(NH)₂, SO(NH), SO₂O, SO₂NR⁸³, PO(OR⁸⁴), PO(OR⁸⁵)NR⁸⁶, NR⁸⁷CO, NR⁸⁸CS, NR⁸⁹SO₂, OSO₂, NR⁹⁰PO(OR⁸⁴), OPO(OR⁹¹), PO(OR⁸⁴)O, NR⁹²CONR⁹³, NR⁹⁴CSNR⁹⁵, NR⁹⁶SO₂NR⁹⁷, NR⁹⁸C(NR⁹⁹)NR¹⁰⁰,
R⁸¹-R¹⁰⁰ = unabhängig voneinander H, C₁₋₂₆-Alkyl, Aryl, Ar- C₁₋₂₆-alkyl, Heteroaryl,
R⁹⁹ = H, C₁₋₂₆-Alkyl, Aryl, Ar- C₁₋₂₆-alkyl, Heteroaryl, CONR¹⁰¹R¹⁰², CSNR¹⁰³R¹⁰⁴, SO₂NR¹⁰⁵R¹⁰⁶
R¹⁰¹ - R¹⁰⁶ = unabhängig voneinander H, C₁₋₂₆-Alkyl, Aryl, Ar- C₁₋₂₆-alkyl, Heteroaryl,
K = verzweigtes oder unverzweigtes C₁₁₋₂₃-Alkyl, verzweigtes oder unverzweigtes C₁₁₋₂₃-Alkenyl, welches unsubstituiert oder durch Aryl oder Heteroaryl substituiert ist, C₁₁₋₂₃-Alkinyl, Aryl, Heteroaryl, Ar- C₁₋₂₆-alkyl, wobei Aryl, Heteroaryl und Ar- C₁₋₂₆-alkyl mit weiteren Aryl-, Heteroaryl- und/oder Ar- C₁₋₂₆-alkylresten substituiert sein können,
oder eines seiner Salze zur Herstellung von Arzneimitteln für die therapeutische und prophylaktische Behandlung von Infektionen.

2. Verwendung nach Anspruch 1, **dadurch gekennzeichnet, daß** für die Verbindungen der Formel (I) gilt:
n = 0 - 3,
R¹, R² und R³ = H,
A = CO, SO₂,
B = Phenyl, Benzyl, Phenethyl, 4-Chlorphenylmethyl, 4-Bromphenylmexhyl, 4-Nitrophenylmethyl, 4-Trifluormethylphenylmethyl, 4-Methylphenylmethyl, 1-(4-Methyl-1-piperazinyl)-1-(4-trifluormethylphenyl)methyl, 1-(4-Methyl-1-piperazinyl)-1-phenylmethyl, 4-Phenylphenyl-methyl, 1-Naphthylmethyl, 2-Naphthylmethyl, Benzoyl, 2,4,4-Trimethylpentyl, 2-Carboxyethyl, 3-Carboxypropyl,
D = Benzoyl,
Y =
ein Rest der Formel (II),
worin
Z = O,
R³⁴ = H, Benzyloxycarbonyl, Trityl,
R³⁵ = H, Benzyloxycarbonyl, tert.-Butyloxycarbonyl,
R³⁶ = H,
oder ein Rest der Formel (III),
worin
Z = O,
R³⁴ = H, Tosyl, Benzyl, 4-Nitrobenzyl, 4-Cyanobenzyl, Benzyloxycarbonyl,
R³⁵ = H, Benzyloxycarbonyl, tert.-Butyloxycarbonyl,
oder ein Rest der Formel (IV),
worin
Z = O,
m = 0 - 3,
R³⁴ = H, Tosyl, Benzyl, 4-Nitrobenzyl, 4-Cyanobenzyl, Benzyloxycarbonyl,
oder ein Rest der Formel (V),
worin
F = CO, CH₂, SO₂,
G = COOH, COOMe, CONHOH, CH₂-COOH, CH₂COOMe, CH₂CONHOH, CH₂CONH-(C₁₄-C₂₀)-Alkyl, CH₂CH₂COOH, CH₂CH₂COOMe, CH₂CH₂CONHOH, CH₂CH₂CONH-(C₁₄-C₂₀)-Alkyl, CH₂CH₂CH₂COOH, CH₂CH₂CH₂COOMe, CH₂CH₂CH₂CONHOH, CH₂CH₂CH₂CONH-(C₁₄-C₂₀)-Alkyl, Phenyl, Naphthyl, Pyridyl, Fluorenyl, Anthracenyl,
oder ein Rest der Formel (VI),
worin
H = CO, SO₂,
I = Methylen, 1,2-Ethylen, 1,3-Trimethylen, 1,4-Tetramethylen, -CH₂-S-CH₂-, -CH₂-O-CH₂-, -CH₂-NH-CH₂-, 1,2-Ethenylen, 1,1-Ethylen, Prop-1-en-1,3-ylen, Prop-2-en-1,3-ylen, Benzylen, 2-Phenyl-1,1-ethylen, Carboxymethylen, Aminocarbonylmethylen, 2-Carboxy-1,1-ethylen, 2-Aminocarbonyl-1,1-ethylen, 3-Carboxy-1,1-propylen, 3-Aminocarbonyl-1,1-propylen, 2-Methyl-1,1-propylen, 3-Methyl-1,1-butylen, 2-Methyl-1,1-butylen, über N1 und C2 verknüpftes Pyrrolidin, 1,2-Phenylen, 1,3-Phenylen, 1,2-Naphthylen, 1,3-Naphthylen, 1,1-Cyclopentylen, 1,1-Cyclohexylen, 1,2-Cyclohexylen,
J = eine Bindung, CO, CS, SO₂, PO(OMe), PO(OH), CONH, CSNH, SO₂NH, PO(OH)O, PO(OH)NH, PO(OMe)O, PO(OMe)NH,
K = (C₁₃-C₁₉)-Alkyl, (C₁₃-C₁₉)-Alkenyl, 4-Benzyloxystyryl, 4-Styrylstyryl, 4-Phenylstyryl, 4-Cyanostyryl, 4-Nitrostyryl, Phenyl, 4-Biphenylyl, 4-Nitrophenyl, 4-Cyanophenyl, 4-Methylsulfonylphenyl, 4-Methoxyphenyl, 4-Bromphenyl, 4-Chlorphenyl, 4-Trifluormethylphenyl, 4-Formylphenyl, 4-Methoxycarbonylphenyl, 4-(1,1-Dicyano-2-vinyl)phenyl, 4-Aminophenyl, 4-Ethylphenyl, 4-Isopropylphenyl, 4-tert.-Butylphenyl, 4-Ethoxyphenyl, 4-Propoxyphenyl, 4-Butoxyphenyl, 4-Benzyloxyphenyl, 3,4-Bis-(benzyloxy)phenyl 3-Phenoxyphenyl, 4-Styrylphenyl 1-Naphthyl, 2-Naphthyl, 2-Fluorenyl, 2-(2-Phenylthiazol-4-yl), 5-(4-Nitrophenyl)thiazo-4-yl) 5-(4-Nitrophenyl)-furan-2-yl), 5-(3-Nitrophenyl)furan-2-yl), 5-(2-Nitrophenyl)furan-2-yl), 5-(4-Bromphenyl)furan-2-yl), 5-(4-Chlorphenyl)furan-2-yl), 5-(3-Trifluormethylphenyl)-furan-2-yl), 5-(4-Trifluormethylphenyl)furan-2-yl), 5-(2-Chlor-3-trifluormethylphenyl)-furan-2-yl) 3,4-Methylendioxyphenyl, 1-Acetylindol-3-yl, 4'-Nitrobiphenylyl, 5-(4-Bromphenyl)thiophen-2-yl), 5-(4-Methylphenyl)furan-2-yl), 5-(4-Methoxyphenyl)furan-2-yl), 5-Bromthiophen-2-yl, 5-Bromfuran-2-yl, 4-Pyridyl, 3-Pyridyl, 2-Pyridyl, Chinolin-2-yl, 1-Naphthylvinyl, 2-Naphthylvinyl, 2-Fluorenyl-vinyl, 2-(2-Phenylthiazol-4-yl)vinyl, 2-[5-(4-Nitrophenyl)furan-2-yl)vinyl, 2-[5-(4-Acetoxy-methylphenyl)furan-2-yl)vinyl, 2-[5-(3-Trifluormethylphenyl)furan-2-yl)vinyl, 4-Benzyloxy-styryl, 3,4-Dibenzyloxystyryl, 3-Methoxy-4-(4-nitrobenzyloxy)styryl, 2-Methylindol-3-ylvinyl, 1-Acetylindol-3-ylvinyl, 3,4-Methylendioxystyryl, 4-(1,1 Dicyano-2-vinyl)styryl, bedeuten, und Salze davon.

3. Verwendung nach Anspruch 1, **dadurch gekennzeichnet, daß** für die Verbindungen der Formel (I) gilt:
n = 0 - 3,
R¹, R² = H
R³ = H,
A = CO, SO₂,
B = Phenyl, Benzyl, Phenethyl, 4-Chlorphenylmethyl, 4-Methylphenylmethyl, 1-(4-Methyl-1-piperazinyl)-1-(4-trifluormethylphenyl)methyl, 1-(4-Methyl-1-piperazinyl)-1-phenylmethyl, 4-Bromphenylmethyl, 4-Nitrophenylmethyl, 4-Trifluormethylphenylmethyl, 4-Phenylphenyl-methyl, 1-Naphthylmethyl, 2-Naphthylmethyl, Benzoyl, 2,4,4-Trimethylpentyl, 2-Carboxyethyl, 3-Carboxypropyl,
D = Benzoyl,
Y =
ein Rest der Formel (IV)
worin
Z = O,
m = 0 - 3,
R³⁴ = H, Benzyl, 4-Nitrobenzyl, 4-Cyanobenzyl,
oder ein Rest der Formel (V)
worin
F = CO, CH₂, SO₂,
G = CH₂CH₂CONH-(C₁₄-C₁₈)-Alkyl, CH₂CH₂CH₂CONH-(C₁₄-C₁₈)-Alkyl, Phenyl, Naphthyl, Pyridyl, Fluorenyl, Anthracenyl
oder ein Rest der Formel (VI)
H = CO,
I = Methylen, 1,2-Ethylen, 1,3-Trimethylen, 1,4-Tetramethylen, -CH₂-S-CH₂-, -CH₂-O-CH₂-, -CH₂-NH-CH₂-, 1,2-Ethenylen, 1,1-Ethylen, Prop-1-en-1,3-ylen, Prop-2-en-1,3-ylen, Benzylen, 2-Phenyl-1,1-ethylen, 2-Methyl-1,1-propylen, 3-Methyl-1,1-butylen, 2-Methyl-1,1-butylen, über N1 und C2 verknüpftes Pyrrolidin,
J = PO(OMe), PO(OH), CONH, CSNH, SO₂NH, PO(OH)O, PO(OH)NH, PO(OMe)O, PO(OMe)NH,
K = (C₁₃-C₁₉)-Alkyl, (C₁₃-C₁₉)-Alkenyl, 4-Benzyloxystyryl, 4-Phenylstyryl, 4-Cyanostyryl, 4-Nitrostyryl, Phenyl, 4-Biphenylyl, 4-Nitrophenyl, 4-Cyanophenyl, 4-Methoxyphenyl, 1-Naphthylvinyl, 2-Naphthylvinyl, 2-Fluorenylvinyl, 2-(2-Phenylthiazol-4-yl)vinyl, 2-[5-(4-Nitrophenyl)furan-2-yl)vinyl, 2-[5-(4-Acetoxymethylphenyl)furan-2-yl)vinyl, 2-[5-(3-Trifluormethylphenyl)furan-2-yl)vinyl, 3,4-Dibenzyloxystyryl, 3-Methoxy-4-(4-nitrobenzyloxy)styryl, 3,4-Methylendioxystyryl, 4-(1,1 Dicyano-2-vinyl)styryl bedeuten, und Salze davon.

4. Verwendung nach einem der Ansprüche 1 bis 4, **dadurch gekennzeichnet, daß** A für CO und B für Benzyl oder 4-Methylphenylmethyl und D für Benzoyl steht.

5. Verwendung nach Anspruch 4, **dadurch gekennzeichnet, daß** die Verbindungen der Formel (I) aus der Gruppe ausgewählt sind, die aus 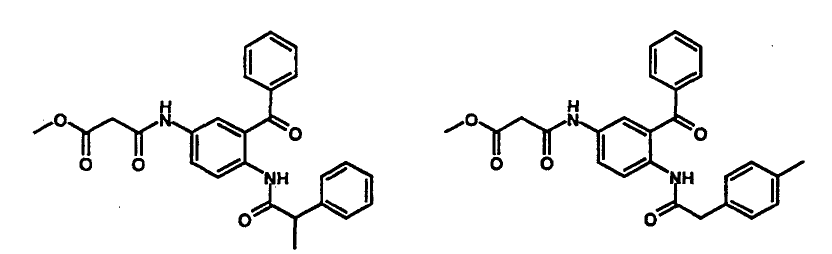 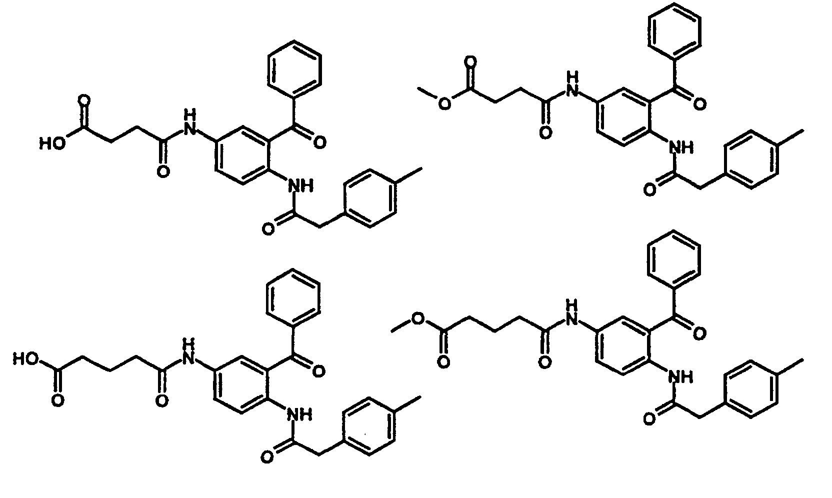 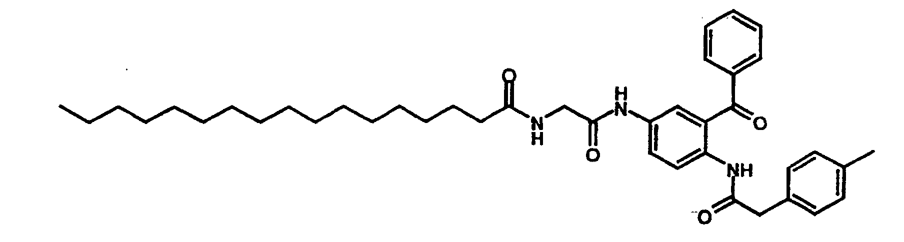 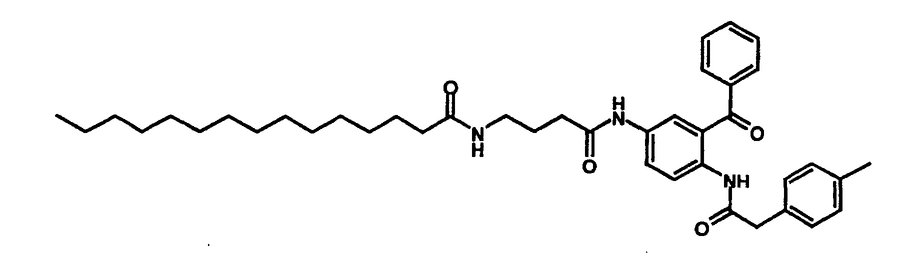 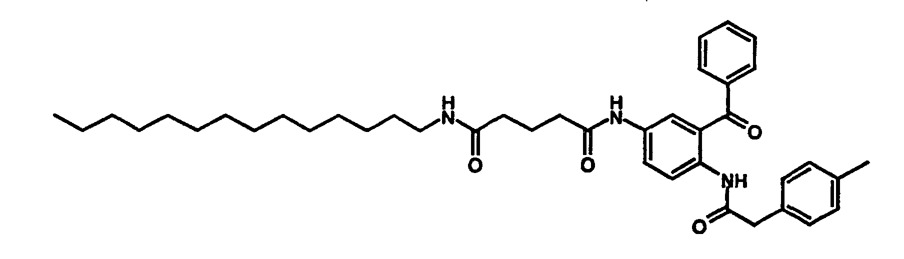 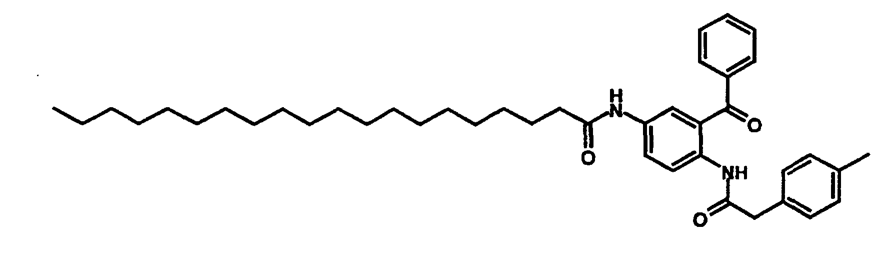 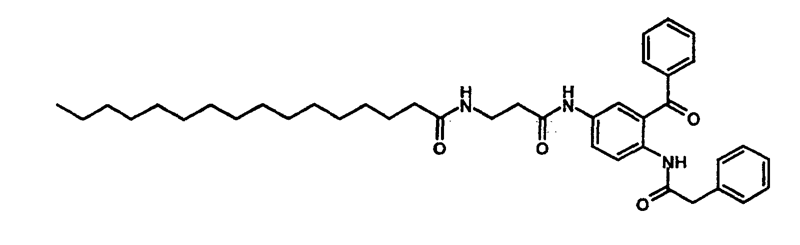 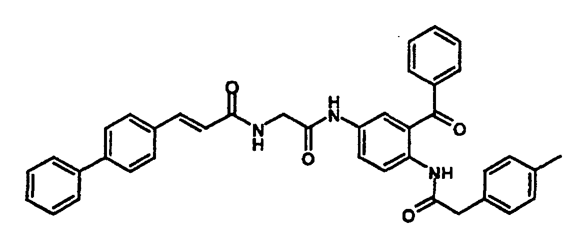 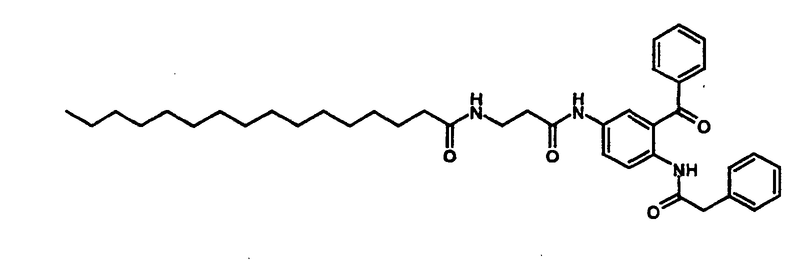 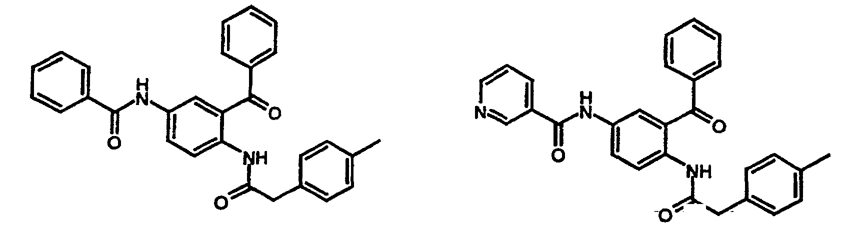 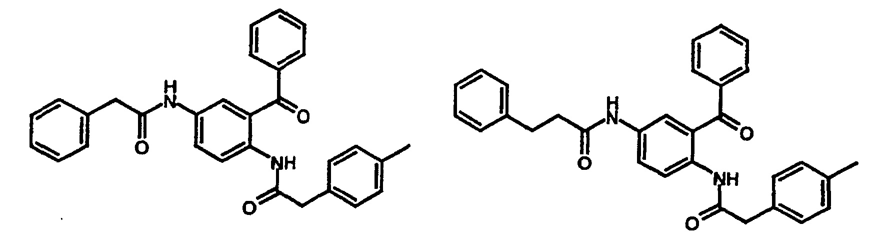 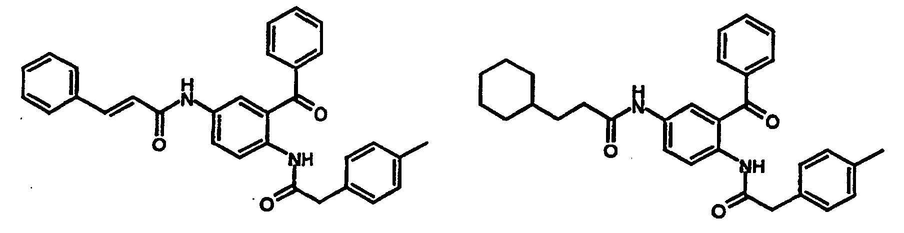 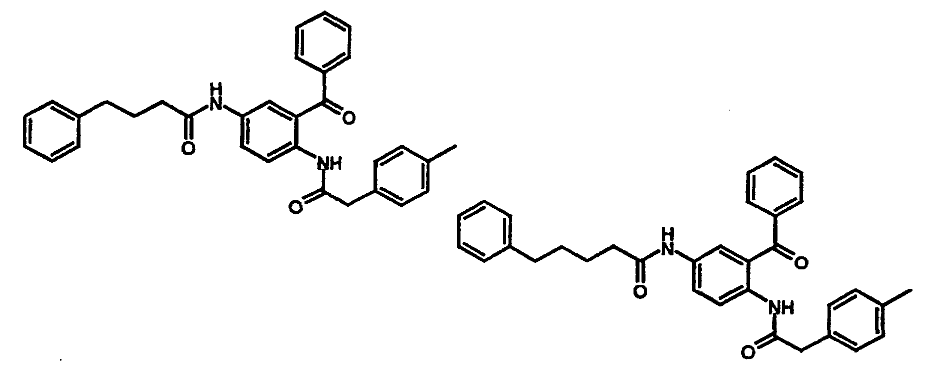 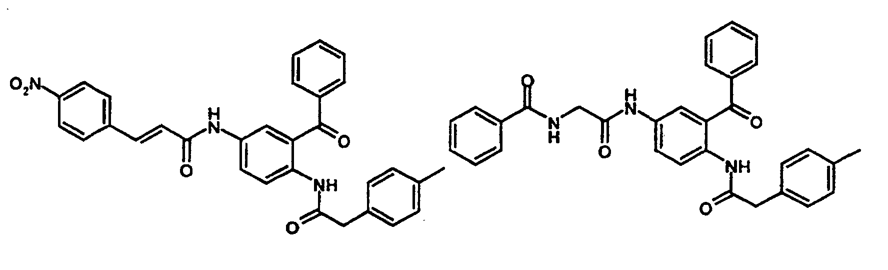 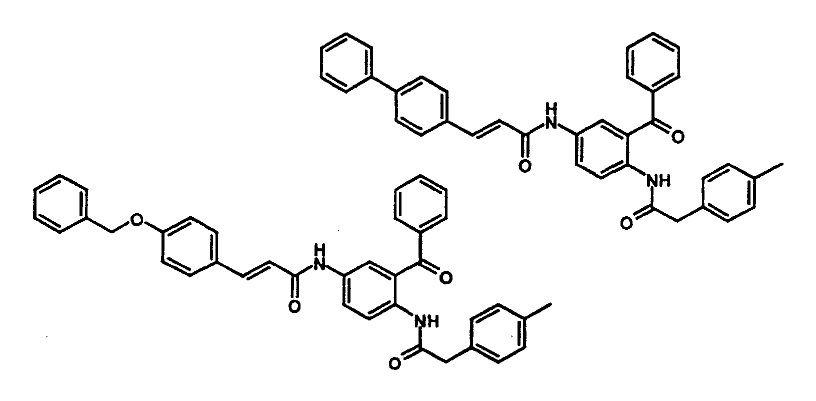 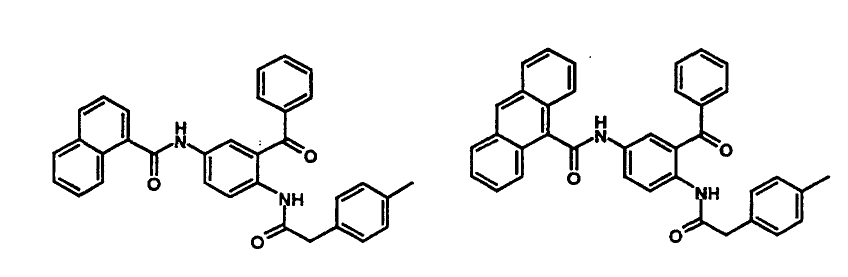 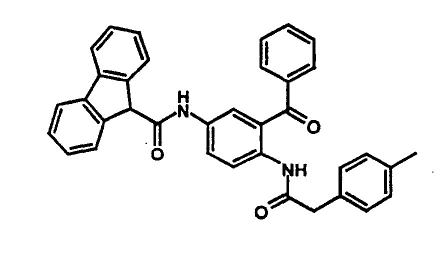 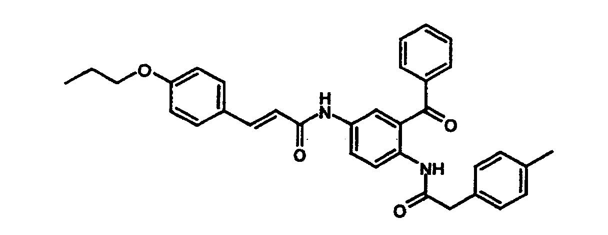 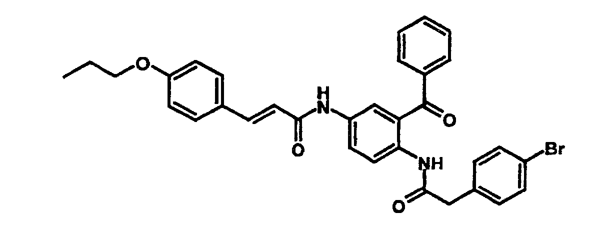 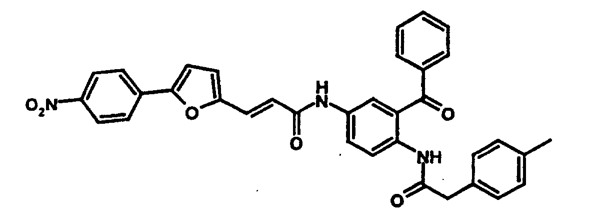 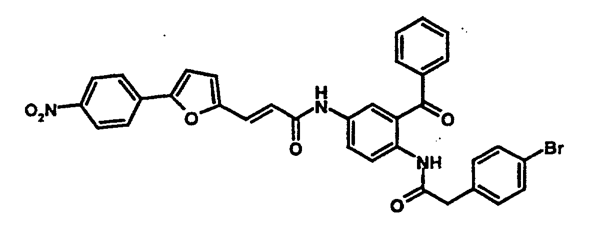 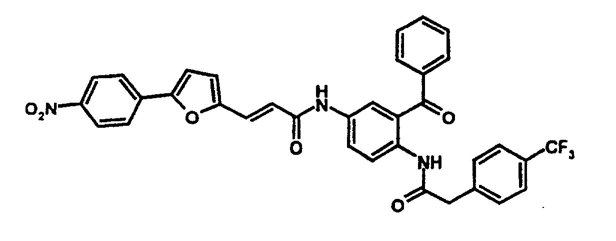 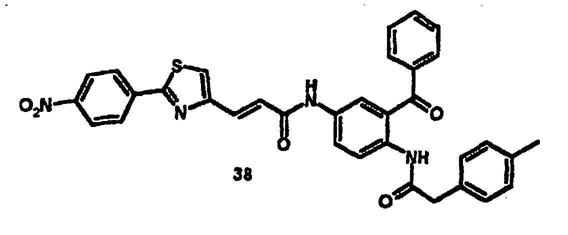 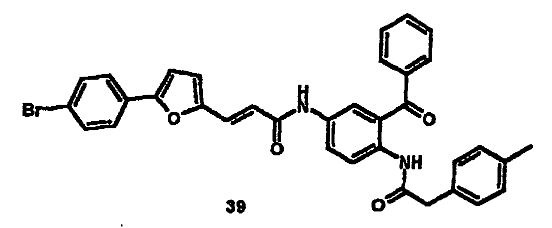 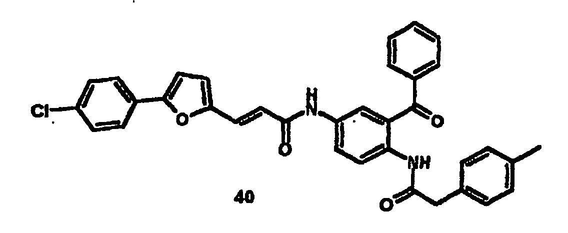 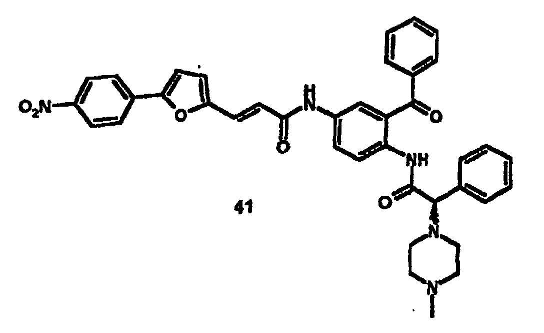 besteht.

6. Verwendung nach Anspruch 1, **dadurch gekennzeichnet, daß** die Verbindungen der Formel (I) aus der Gruppe ausgewählt sind, die aus N-[3-[3-Benzoyl-4-[2-(4-methylphenyl)-acetyl]amino]-phenylamino]-4-phenylzimtsäureamid und N-[2-[3-Benzoyl-4-[[2-(4-methylphenyl)acetyl]-amino]phenyl]-4-benzyloxyzimtsäureamid besteht.

7. Verwendung nach einem der Ansprüche 1 bis 6 zur Herstellung von Arzneimitteln zur Vorbeugung und Behandlung von Infektionen verursacht durch Parasiten, insbesondere einzellige Parasiten, nämlich Erreger der Malaria, der Schlafkrankheit, der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akantha-möbose, der Naeglerose, der Kokzidiose, der Giardiose und der Lambliose.

8. Arzneimittel zur therapeutischen und prophylaktischen Behandlung infektiöser Prozesse, **dadurch gekennzeichnet, daß** das Präparat einen wirksamen Gehalt an zumindest einer Verbindung nach einem der Ansprüche 1 bis 6 zusammen mit einem pharmazeutisch akzeptablen Träger enthält.

9. Arzneimittel nach Anspruch 8, **dadurch gekennzeichnet, daß** das Präparat einen weiteren pharmazeutischen Wirkstoff enthält.

## Claims

1. Use of at least one 2-phenylene diamine derivative of general formula (I): 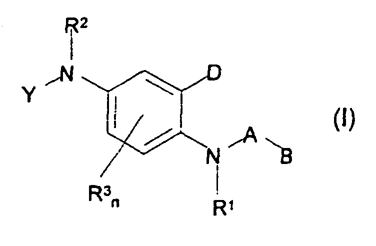 wherein
n =0-3;
R¹, R² = H, C₁₋₂₆-alkyl, aryl, heteroaryl, C₁₋₂₆-acyl;
R³ = H, halogen, C₁₋₂₆-alkyl, aryl, heteroaryl, ar- C₁₋₂₆-alkyl, C₁₋₂₆-acyl, CN, NO₂, R⁴-X-; with R⁴ = H, C₁₋₂₆-alkyl, aryl, heteroaryl, ar- C₁₋₂₆-alkyl, C₁₋₂₆-acyl;
X = NH, O, S, SO₂, NHSO₂, OSO₂,
A = CH₂, CHR⁵, CR⁵R⁶, CO, CS, CONR⁴, CSNR⁴, SO₂, PO₂,
R⁵, R⁶ = independently of each other C₁₋₂₆-alkyl, aryl, heteroaryl, ar- C₁₋₂₆-alkyl, CN, NO₂, COR⁷,
R⁷ = H, C₁₋₂₆-alkyl, aryl, ar- C₁₋₂₆-alkyl, C₁₋₂₆-alkoxy, aryloxy, ar- C₁₋₂₆-alkoxy, NR⁸R⁹, R⁸, R⁹ = independently of each other H, C₁₋₂₆-alkyl, aryl, ar- C₁₋₂₆-alkyl, heteroaryl,
B = C₁₋₂₆-alkyl, aryl, heteroaryl, arylcarbonyl, saturated heterocycles, substituted alkyl having 1-4 chain members, wherein the substituents may be C₁₋₉-alkyl, aryl, heteroaryl, halogen, =O, OH, NH₂, NH-CO-R¹⁰, NH-SO₂-R¹⁰, COOR¹¹, CO-NR¹²R¹³, CS-NR¹⁴R¹⁵, SO₂OR¹⁶, SO₂NR¹⁷R¹⁸, NH-CO-OR¹⁹, NH-CO-NR²⁰R²¹, NHCSNR²²R²³,
R¹⁰ - R²³ = independently of each other H, C₁₋₂₆-alkyl, aryl, ar- C₁₋₂₆-alkyl, heteroaryl,
D = H, C₁₋₂₆-alkyl, aryl, heteroaryl, ar- C₁₋₂₆-alkyl, -Y-R²⁴, halogen, NO₂, CN, NH-CO-R²⁵, NH-SO₂-R²⁶, NH-CO-OR²⁷, NH-CO-NR²⁸R²⁹, NH-CS-NR³⁰R³¹,
Y= O, NH, S, CO, CS, SO₂, COO, CONR³¹, CSNR³², SO₂NR³³,
R²⁴ - R³³ = independently of each other H, C₁₋₂₆-alkyl, aryl, heteroaryl, ar- C₁₋₂₆-alkyl,
and
Y = a group selected from 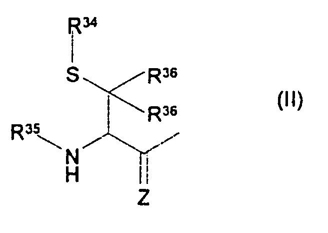 wherein
Z = O, S or two hydrogen atoms,
R³⁴ = H, C₁₋₂₆-alkyl, aryl, ar- C₁₋₂₆-alkyl, COOR³⁷, arylsulfonyl,
R³⁵ = H, C₁₋₂₆-acyl, COOR³⁸,
R³⁶ = independently of each other H, C₁₋₂₆-alkyl,
R³⁷, R³⁸ = independently of each other C₁₋₂₆-alkyl, aryl, ar- C₁₋₂₆-alkyl,
or 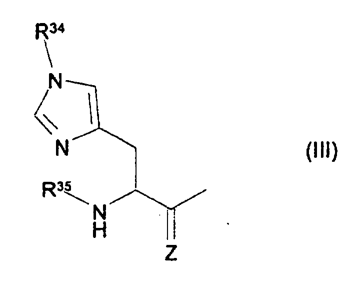 wherein
Z = O, S or two hydrogen atoms,
R³⁴ = H, C₁₋₂₆-alkyl, aryl, ar- C₁₋₂₆-alkyl, COOR³⁷, arylsulfonyl,
R³⁵ = H, C₁₋₂₆-acyl, COOR³⁸
R³⁷, R³⁸ = independently of each other C₁₋₂₆-alkyl, aryl, ar- C₁₋₂₆-alkyl,
or 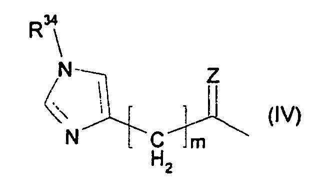 wherein
Z = O, S or two hydrogen atoms,
m=0-3
R³⁴ = H, C₁₋₂₆-alkyl, aryl, ar- C₁₋₂₆-alkyl, COOR³⁷, aryl sulfonyl,
R³⁷ = C₁₋₂₆-alkyl, aryl, ar- C₁₋₂₆-alkyl,
or 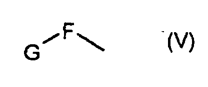 wherein
F = CH₂, CO, CS, SO₂,
G = COOR³⁹, CONHOH, CONR⁴⁰R⁴¹, CSNR⁴²R⁴³, C₁₋₂₆-alkyl- or alkyl substituted by aryl and having 1-3 chain members or alkyl having 1-3 chain members, which supports a substituent at the terminal C-atom the substituent selected from COOR⁴⁴, CONHOH, CONR⁴⁵R⁴⁶, CSNR⁴⁷R⁴⁸, SR⁴⁹, SOR⁵⁰, SO₂R⁵¹, SO₂NR⁵²R⁵³, PO(OR⁵⁴)OR⁵⁵, PO(OR⁵⁶)NR⁵⁷₂, OSO₂R⁵⁸, O(PO)OR⁵⁹, NHSO₂R⁶⁰, NHPO₂R⁶¹, NHCOR⁶², NHCSR⁶³, NHCONR⁶⁴R⁶⁵, NHCSNR⁶⁶R⁶⁷, -S(NH)₂-R⁶⁸ or NH(C=NR⁷⁰)NHR⁶⁹, furthermore aryl or heteroaryl,
R³⁹ - R⁶⁹ = independently of each other H, C₁₋₂₆-alkyl, aryl,
R⁷⁰ = CONH₂, SO₂NH₂,
or is 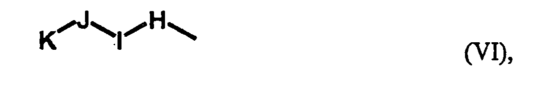 wherein
H = CH₂, CO, CS, CHR⁷¹, CR⁷²R⁷³, SO₂, SO, PO_{2,}
I = C₁₋₂₆-alkylene, C₁₋₂₆-alkylene, in which one methylene group is replaced by O, S, or NR⁷⁷, or C₂₋₂₆-alkenylene, these alkylene or alkenylene radicals being unsubstituted or substituted by aryl, heteroaryl, halogen, OH, CN, C₁₋₉-alkyloxy, aryloxy, COOR⁷⁴, CONR⁷⁵R⁷⁶, NR⁷⁷R⁷⁸, NH(C=NR⁷⁰)NHR⁶⁹, SR⁷⁹, SO₂R⁸⁰; furthermore C₃₋₈-cycloalkylene, unsubstituted or substituted by aryl, heteroaryl, halogen, OH, CN, C₁₋₉-alkyloxy, aryloxy, COOR⁷⁴, CONR⁷⁵R⁷⁶, NR⁷⁷R⁷⁸, SR⁷⁹, SO₂R⁸⁰; C₃₋₈-cycloalkylene,
wherein the alkylene chain is interrupted by O, S or NR⁷⁷, arylene, unsubstituted or substituted by aryl, heteroaryl, halogen, OH, CN, C₁₋₂₆-alkoxy, aryloxy, COOR⁷⁴, CONR⁷⁵R⁷⁶, NR⁷⁷R⁷⁸, SR⁷⁹, SO₂R⁸⁰,
R⁷¹ - R⁸⁰ = independently of each other H, C₁₋₂₆-alkyl, aryl, ar- C₁₋₂₆-alkyl, heteroaryl,
J = a bonding or CO, COO, CONR⁸¹, CS, CSNR⁸², SO₂, S(NH)₂, SO(NH), SO₂O, SO₂NR⁸³, PO(OR⁸⁴), PO(OR⁸⁵)NR⁸⁶, NR⁸⁷CO, NR⁸⁸CS, NR⁸⁹SO₂, OSO₂, NR⁹⁰PO(OR⁸⁴), OPO(OR⁹¹), PO(OR⁸⁴)O, NR⁹²CONR⁹³, NR⁹⁴CSNR⁹⁵, NR⁹⁶SO₂NR⁹⁷, NR⁹⁸C(NR⁹⁹)NR¹⁰⁰,
R⁸¹-R¹⁰⁰ = independently of each other H, C₁₋₂₆-alkyl, aryl, ar- C₁₋₂₆-alkyl, heteroaryl,
R⁹⁹ = H, C₁₋₂₆-alkyl, aryl, ar- C₁₋₂₆-alkyl, heteroaryl, CONR¹⁰¹R¹⁰², CSNR¹⁰³R¹⁰⁴, SO₂NR¹⁰⁵R¹⁰⁶
R¹⁰¹ - R¹⁰⁶ = independently of each other H, C₁₋₂₆-alkyl, aryl, ar- C₁₋₂₆-alkyl, heteroaryl,
K = branched or unbranched C₁₁₋₂₃-alkyl, branched or unbranched C₁₁₋₂₃-alkenyl being unsubstituted or substituted by aryl or heteroaryl, C₁₁₋₂₃-alkinyl, aryl, heteroaryl, ar- C₁₋₂₆-alkyl, wherein aryl, heteroaryl and ar- C₁₋₂₆-alkyl are substituted by further aryl-, hetero-aryl- and/or ar- C₁₋₂₆-alkyl radicals, or one of its salts for preparing pharmaceutical compositions for the therapeutic and prophylactic treatment of infections.

2. Use according to claim 1, **characterized in that** the following is valid for the compounds of formula (I) gilt:
n = 0 - 3,
R¹, R² and R³ = H,
A = CO, SO₂,
B = phenyl, benzyl, phenethyl, 4-chlorophenylmethyl, 4-bromophenylmethyl, 4-nitrophenylmethyl, 4-trifluoromethylphenylmethyl, 4-methylphenylmethyl, 1-(4-methyl-1-piperazinyl)-1-(4-trifluoromethylphenyl)methyl, 1-(4-methyl-1-piperazinyl)-1-phenylmethyl, 4-phenylphenyl-methyl, 1-naphthylmethyl, 2-naphthylmethyl, benzoyl, 2,4,4-trimethylpentyl, 2-carboxyethyl, 3-carboxypropyl,
D = benzoyl,
Y =
a radical of formula (II),
wherein
Z = O,
R³⁴ = H, benzyloxycarbonyl, trityl,
R³⁵ = H, benzyloxycarbonyl, tert.-butyloxycarbonyl,
R³⁶ = H,
or a radical of formula (III),
wherein
Z = O,
R³⁴ = H, tosyl, benzyl, 4-nitrobenzyl, 4-cyanobenzyl, benzyloxycarbonyl,
R³⁵ = H, benzyloxycarbonyl, tert.-butyloxycarbonyl,
or a radical of formula (IV),
wherein
Z = O,
m = 0 - 3,
R³⁴ = H, tosyl, benzyl, 4-nitrobenzyl, 4-cyanobenzyl, benzyloxycarbonyl,
or a radical of formula (V),
wherein
F = CO, CH₂, SO₂,
G = COOH, COOMe, CONHOH, CH₂-COOH, CH₂COOMe, CH₂CONHOH, CH₂CONH-(C₁₄-C₂₀)-alkyl, CH₂CH₂COOH, CH₂CH₂COOMe, CH₂CH₂CONHOH, CH₂CH₂CONH-(C₁₄-C₂₀)-alkyl, CH₂CH₂CH₂COOH, CH₂CH₂CH₂COOMe, CH₂CH₂CH₂CONHOH, CH₂CH₂CH₂CONH-(C₁₄-C₂₀)-alkyl, phenyl, naphthyl, pyrridyl, fluorenyl, anthracenyl,
or a radical of formula (VI),
wherein
H = CO, SO₂,
I = methylene, 1,2-ethylene, 1,3-trimethylene, 1,4-tetramethylene, -CH₂-S-CH₂-, -CH₂-O-CH₂-, -CH₂-NH-CH₂-, 1,2-ethenylene, 1,1-ethylene, prop-1-en-1,3-ylene, prop-2-en-1,3-ylene, benzylene, 2-phenyl-1,1-ethylene, carboxymethylene, amino carbonyl methylene, 2-Carboxy-1,1-ethylene, 2-aminocarbonyl-1,1-ethylene, 3-carboxy-1,1-propylene, 3-aminocarbonyl-1,1-propylene, 2-methyl-1,1-propylene, 3-methyl-1,1-butylene, 2-methyl-1,1-butylene, pyrrolidine connected via N1 and C2,, 1,2-phenylene, 1,3-phenylene, 1,2-naphthylene, 1,3-naphthylene, 1,1-cyclopentylene, 1,1-cyclohexylene, 1,2-cyclohexylene, J = a bonding, CO, CS, SO₂, PO(OMe), PO(OH), CONH, CSNH, SO₂NH, PO(OH)O, PO(OH)NH, PO(OMe)O, PO(OMe)NH,
K = (C₁₃-C₁₉)-alkyl, (C₁₃-C₁₉)-alkenyl, 4-benzyloxystyryl, 4-styrylstyryl, 4-phenylstyryl, 4-cyanostyryl, 4-nitrostyryl, phenyl, 4-biphenylyl, 4-nitrophenyl, 4-cyanophenyl, 4-methylsulfonylphenyl, 4-methoxyphenyl, 4-bromophenyl, 4-chlorophenyl, 4-trifluoromethylphenyl, 4-Formylphenyl, 4-methoxycarbonylphenyl, 4-(1,1-dicyano-2-vinyl)phenyl, 4-aminophenyl, 4-ethylphenyl, 4-Isopropylphenyl, 4-tert.-butylphenyl, 4-ethoxyphenyl, 4-propoxyphenyl, 4-butoxyphenyl, 4-benzyloxyphenyl, 3,4-bis-(benzyloxy)phenyl 3-phenoxyphenyl, 4-styrylphenyl 1-naphthyl, 2-naphthyl, 2-fluorenyl, 2-(2-phenylthiazol-4-yl), 5-(4-nitrophenyl)thiazo-4-yl) 5-(4-nitrophenyl)-furan-2-yl), 5-(3-nitrophenyl)furan-2-yl), 5-(2-nitrophenyl)furan-2-yl), 5-(4-bromophenyl)furan-2-yl), 5-(4-chlorophenyl)furan-2-yl), 5-(3-trifluoromethylphenyl)-furan-2-yl), 5-(4-trifluoromethylphenyl)furan-2-yl), 5-(2-chloro-3-trifluoromethylphenyl)-furan-2-yl) 3,4-methylenedioxyphenyl, 1-acetylindol-3-yl, 4'-nitrobiphenylyl, 5-(4-bromophenyl)thiophen-2-yl), 5-(4-methylphenyl)furan-2-yl), 5-(4-methoxyphenyl)furan-2-yl), 5-bromothiophen-2-yl, 5-bromofuran-2-yl, 4-pyridyl, 3-pyridyl, 2-pyridyl, Chinolin-2-yl, 1-naphthylvinyl, 2-naphthylvinyl, 2-fluorenyl-vinyl, 2-(2-phenylthiazol-4-yl)vinyl, 2-[5-(4-nitrophenyl)furan-2-yl)vinyl, 2-[5-(4-acetoxy-methylphenyl)furan-2-yl)vinyl, 2-[5-(3-trifluoromethylphenyl)furan-2-yl)vinyl, 4-benzyloxy-styryl, 3,4-dibenzyloxystyryl, 3-methoxy-4-(4-nitrobenzyloxy)styryl, 2-methylindol-3-ylvinyl, 1-acetylindol-3-ylvinyl, 3,4-methylenedioxystyryl, 4-(1,1 dicyano-2-vinyl)styryl and the salts thereof.

3. Use according to claim 1, **characterized in that** the following is valid for the compounds of formula (I):
n = 0 - 3,
R¹, R² = H
R³ = H,
A = CO, SO₂,
B = phenyl, benzyl, phenethyl, 4-chlorophenylmethyl, 4-methylphenylmethyl, 1-(4-methyl-1-piperazinyl)-1-(4-trifluoromethylphenyl)methyl, 1-(4-methyl-1-piperazinyl)-1-phenylmethyl, 4-bromophenylmethyl, 4-nitrophenylmethyl, 4-trifluoromethylphenylmethyl, 4-phenylphenyl-methyl, 1-naphthylmethyl, 2-naphthylmethyl, benzoyl, 2,4,4-trimethylpentyl, 2-Carboxyethyl, 3-Carboxypropyl, D = benzoyl,
Y =
a radical of formula (IV)
wherein
Z = O,
m = 0 - 3,
R³⁴ = H, benzyl, 4-nitrobenzyl, 4-cyanobenzyl,
or a radical of formula (V)
wherein
F = CO, CH₂, SO₂,
G = CH₂CH₂CONH-(C₁₄-C₁₈)-alkyl, CH₂CH₂CH₂CONH-(C₁₄-C₁₈)-alkyl, phenyl, naphthyl, pyridyl, fluorenyl, anthracenyl
or a radical of formula (VI)
H=CO,
I = methylene, 1,2-ethylene, 1,3-trimethylene, 1,4-tetramethylene, -CH₂-S-CH₂-, -CH₂-O-CH₂-, -CH₂-NH-CH₂-, 1,2-ethenylene, 1,1-ethylene, prop-1-en-1,3-ylene, prop-2-en-1,3-ylene, benzylene, 2-phenyl-1,1-ethylene, 2-methyl-1,1-propylene, 3-methyl-1,1-butylene, 2-methyl-1,1-butylene, pyrrolidine connected via N1 and C2,
J = PO(OMe), PO(OH), CONH, CSNH, SO₂NH, PO(OH)O, PO(OH)NH, PO(OMe)O, PO(OMe)NH,
K = (C₁₃-C₁₉)-alkyl, (C₁₃-C₁₉)-alkenyl, 4-benzyloxystyryl, 4-phenylstyryl, 4-cyanostyryl, 4-nitrostyryl, phenyl, 4-biphenylyl, 4-nitrophenyl, 4-cyanophenyl, 4-methoxyphenyl, 1-naphthylvinyl, 2-naphthylvinyl, 2-fluorenylvinyl, 2-(2-phenylthiazol-4-yl)vinyl, 2-[5-(4-nitrophenyl)furan-2-yl)vinyl, 2-[5-(4-acetoxymethylphenyl)furan-2-yl)vinyl, 2-[5-(3-trifluoromethylphenyl)furan-2-yl)vinyl, 3,4-dibenzyloxystyryl, 3-methoxy-4-(4-nitrobenzyloxy)styryl, 3,4-methylenedioxystyryl, 4-(1,1 dicyano-2-vinyl)styryl and the salts thereof.

4. Use according to one of claims 1 to 4, **characterized in that** A represents CO and B represents benzyl or 4-methylphenylmethyl and D represents benzoyl.

5. Use according to claim 4, **characterized in that** die compounds of formula (I) is selected from the group consisting of

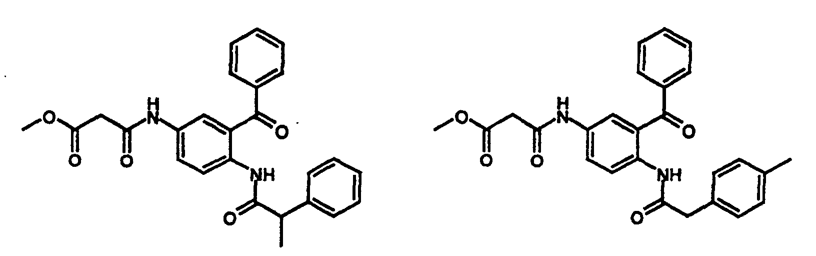

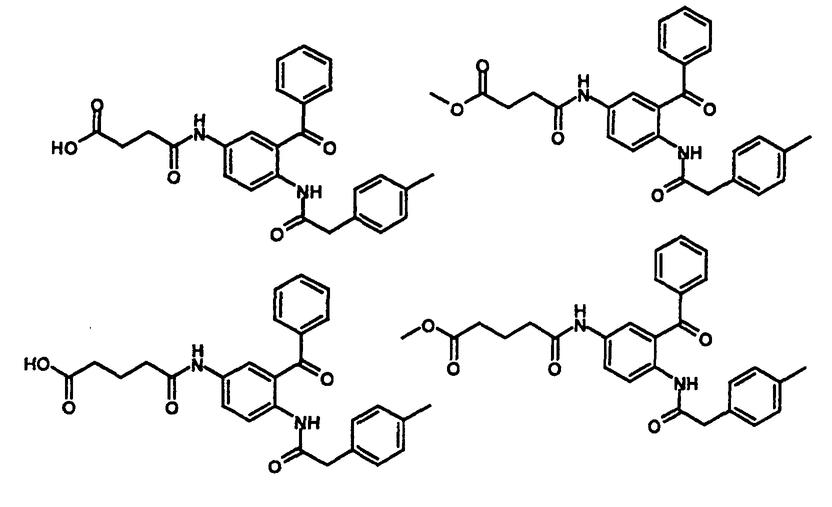

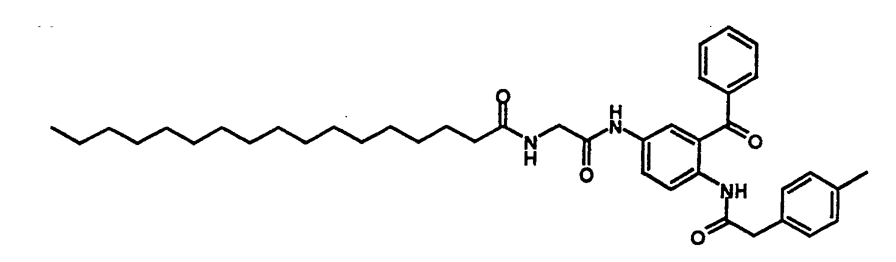

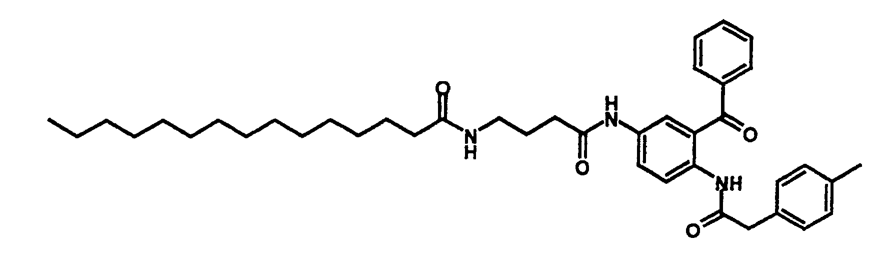

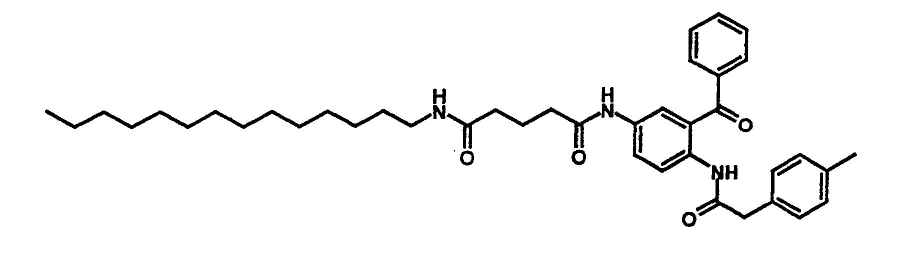

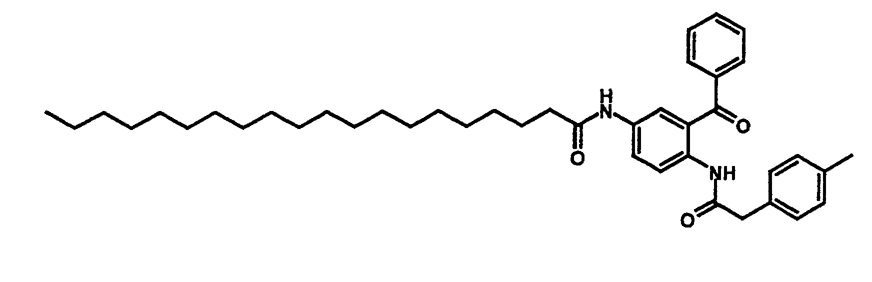

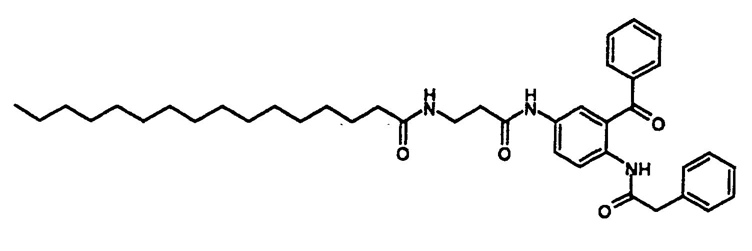

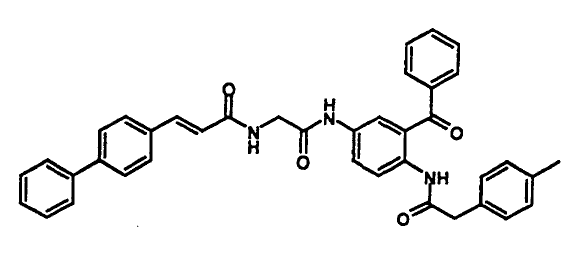

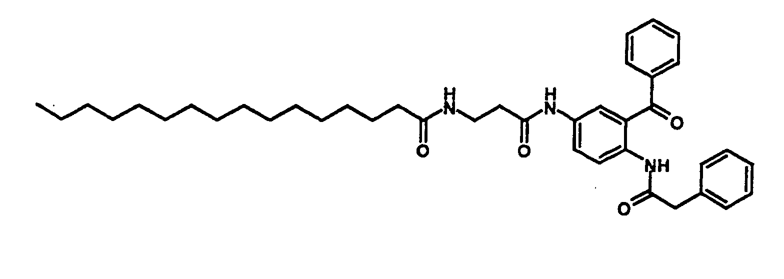

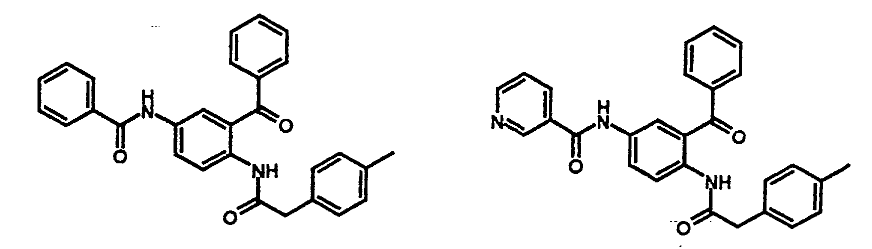

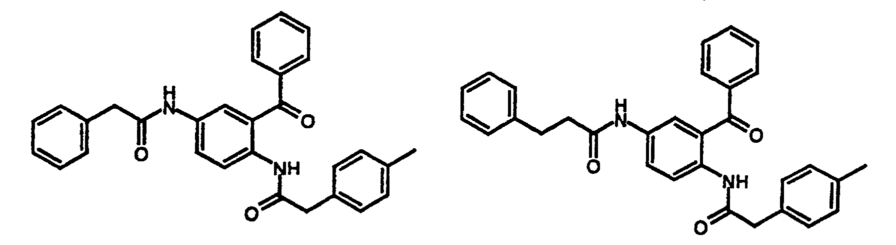

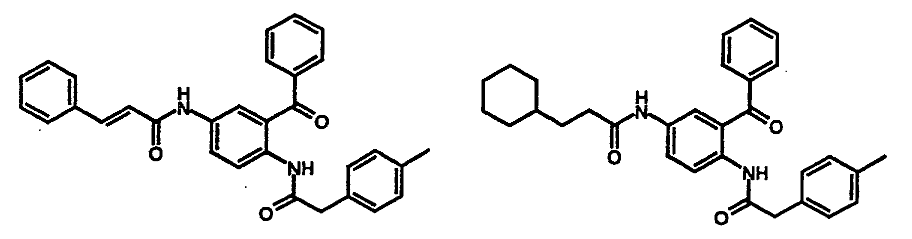

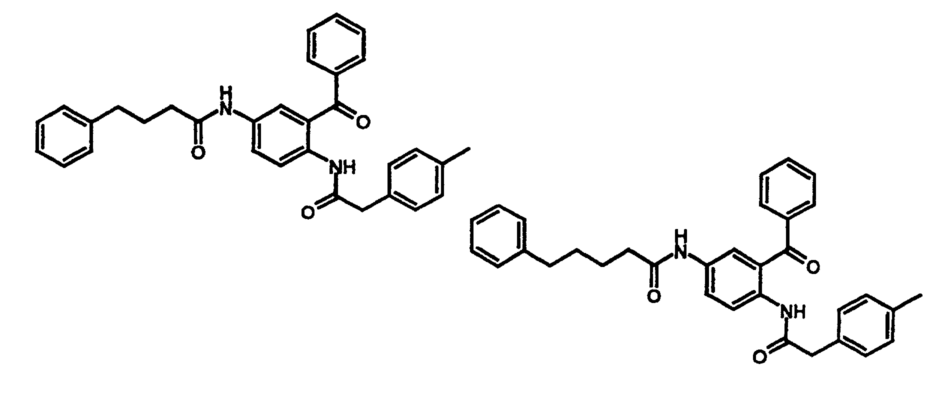

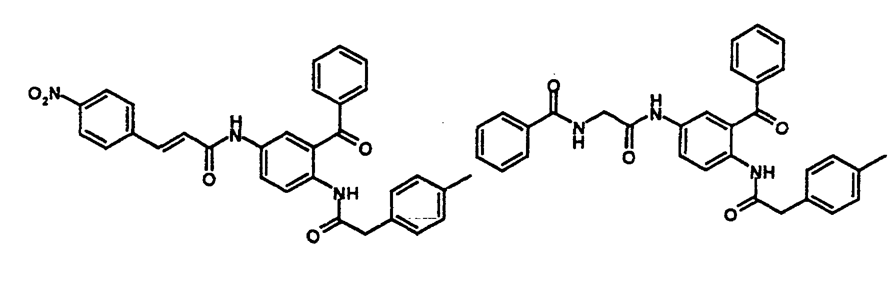

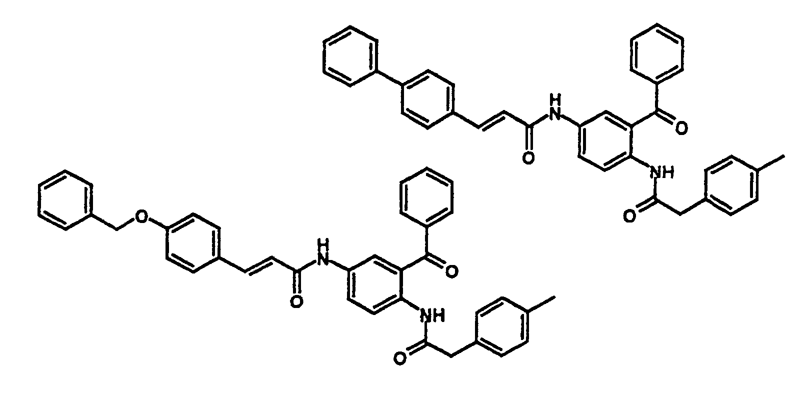

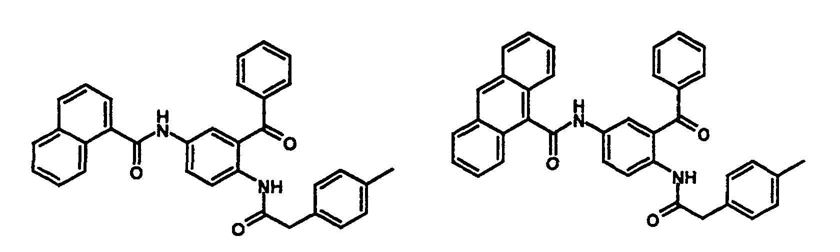

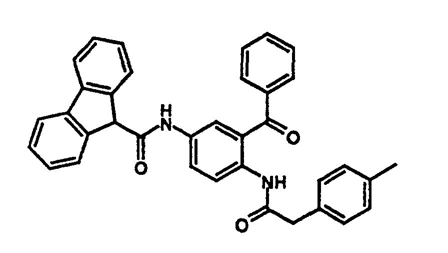

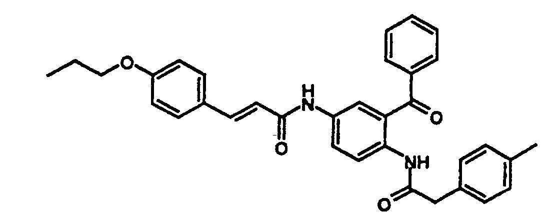

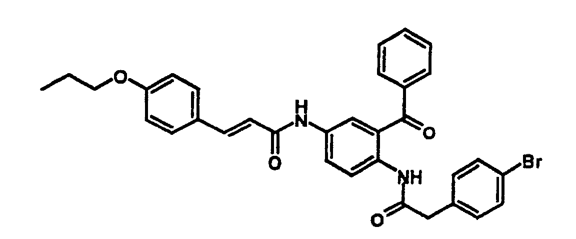

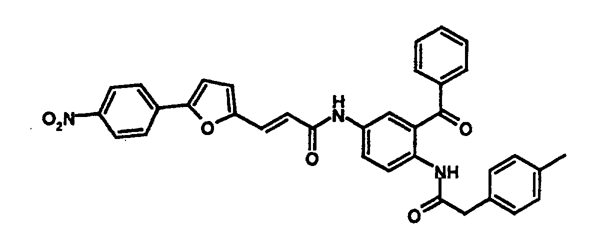

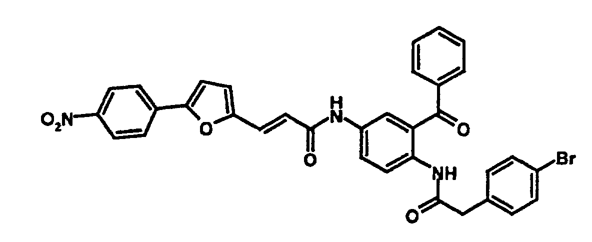

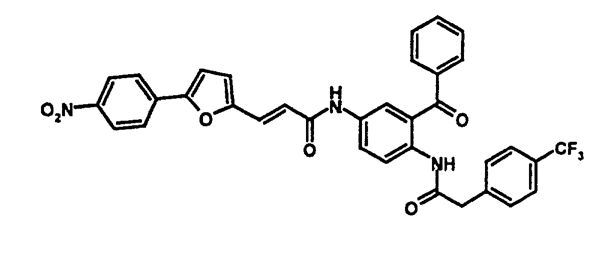

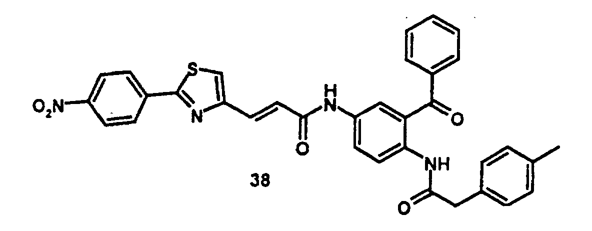

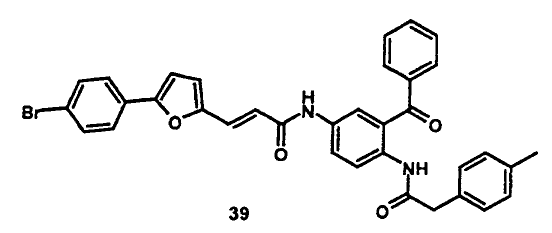

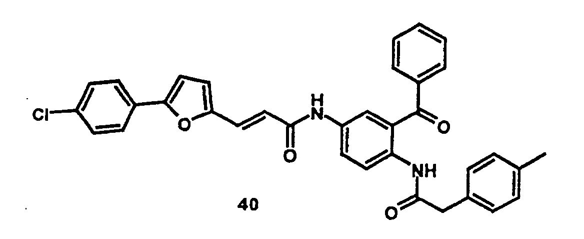

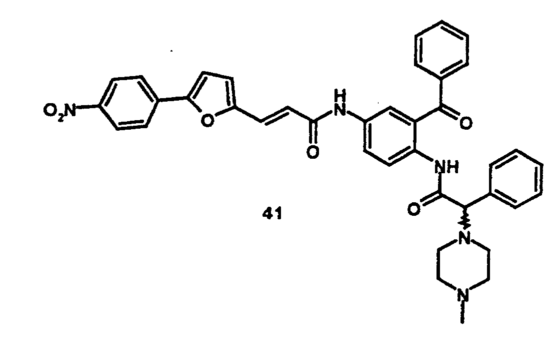

6. Use according to claim 1, **characterized in that** die compounds of formula (I) selected from the group consisting of N-[3-[3-benzoyl-4-[2-(4-methylphenyl)-acetyl]amino]-phenylamino]-4-phenyl cinnamic acid amide and N-[2-[3-benzoyl-4-[[2-(4-methylphenyl)acetyl]-amino]phenyl]-4-benzyloxy cinnamic acid amide.

7. Use according to one of claims 1 to 6 for preparing pharmaceutical compositions for the prophylactics and treatment of infections caused by parasites, especially unicellular parasites, namely pathogens of malaria, the sleeping sickness, the Chagas' disease, the toxoplasmosis, amoebic dysentery, leishmania sis, trichomoniasis, pnemacystosis, balantidiosis, cryptosporidiosis, sarcocystosis, acanthamebiasis, naegleriasis, coccidiosis, giardiasis and lambliosis..

8. Pharmaceutical preparation for the therapeutic and prophylactic treatment of infectious processes, **characterized in that** the preparation contains an effective amount of at least one compound according to one of claims 1 to 6 together with a pharmaceutically acceptable carrier.

9. Pharmaceutical preparation according to claim 8, **characterized in that** the preparation contains a further pharmaceutical active agent.

## Revendications

1. Utilisation d'au moins un dérivé de 2-phénylènediamine de formule générale (I) : 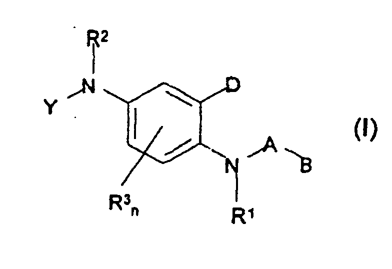 dans laquelle
n = 0 à 3 ;
R¹, R² = H, un groupe alkyle en C₁ à C₂₆, aryle, hétéroaryle, acyle en C₁ à C₂₆ ;
R³ = H, un atome d'halogène, un groupe alkyle en C₁ à C₂₆, aryle, hétéroaryle, ar-(alkyle en C₁ à C₂₆), acyle en C₁ à C₂₆, CN, NO₂, R⁴-X- ;
avec R⁴ = H, un groupe alkyle en C₁ à C₂₆, aryle, hétéroaryle, ar-(alkyle en C₁ à C₂₆), acyle en C₁ à C₂₆ ;
X = NH, O, S, SO₂, NHSO₂, OSO₂,
A = CH₂, CHR⁵, CR⁵R⁶, CO, CS, CONR⁴, CSNR⁴, SO₂, PO₂,
R⁵, R⁶ = indépendamment l'un de l'autre, un groupe alkyle en C₁ à C₂₆, aryle, hétéroaryle, ar-(alkyle en C₁ à C₂₆), CN, NO₂, COR⁷,
R⁷ = H, un groupe alkyle en C₁ à C₂₆, aryle, ar-(alkyle en C₁ à C₂₆), alcoxy en C₁ à C₂₆, aryloxy, ar-(alcoxy en C₁ à C₂₆), NR⁸R⁹,
R⁸, R⁹ = indépendamment l'un de l'autre, H, un groupe alkyle en C₁ à C₂₆, aryle, ar-(alkyle en C₁ à C₂₆), hétéroaryle,
B = un groupe alkyle en C₁ à C₂₆, aryle, hétéroaryle, arylcarbonyle, des hétérocycles saturés, un groupe alkyle substitué présentant 1 à 4 éléments dans la chaîne, les substituants pouvant être un groupe alkyle en C₁ à C₉, aryle, hétéroaryle, un atome d'halogène, =O, OH, NH₂, NH-CO-R¹⁰, NH-SO₂-R¹⁰, COOR¹¹, CO-NR¹²R¹³, CS-NR¹⁴R¹⁵, SO₂OR¹⁶, SO₂NR¹⁷R¹⁸, NH-CO-OR¹⁹, NH-CO-NR²⁰R²¹, NHCSNR²²R²³,
R¹⁰ à R²³ = indépendamment les uns des autres, H, un groupe alkyle en C₁ à C₂₆, aryle, ar-(alkyle en C₁ à C₂₆), hétéroaryle,
D = H, un groupe alkyle en C₁ à C₂₆, aryle, hétéroaryle, ar-(alkyle en C₁ à C₂₆), -Y-R²⁴, un atome d'halogène, NO₂, CN, NH-CO-R²⁵, NH-SO₂-R²⁶, NH-CO-OR²⁷, NH-CO- NR²⁸R²⁹, NH-CS-NR³⁰R³¹,
Y = O, NH, S, CO, CS, SO₂, COO, CONR³¹, CSNR³², SO₂NR³³,
R²⁴ à R³³ = indépendamment les uns des autres, H, un groupe alkyle en C₁ à C₂₆, aryle, hétéroaryle, ar-(alkyle en C₁ à 26), et
Y = un groupe choisi parmi 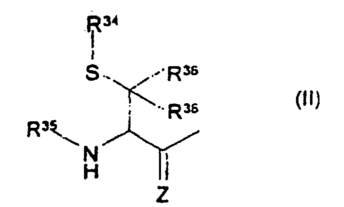
dans lequel
Z = O, S ou deux atomes d'hydrogène,
R³⁴ = H, un groupe alkyle en C₁ à C₂₆, aryle, ar-(alkyle en C₁ à C₂₆), COOR³⁷, arylsulfonyle,
R³⁵ = H, un groupe acyle en C₁ à C₂₆, COOR³⁸,
R³⁶ = indépendamment l'un de l'autre, H, un groupe alkyle en C₁ à C₂₆,
R³⁷, R³⁸ = indépendamment l'un de l'autre, un groupe alkyle en C₁ à C₂₆, aryle, ar-(alkyle en C₁ à C₂₆), ou 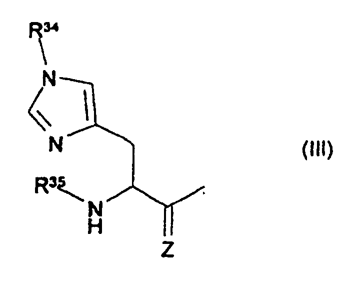
dans lequel
Z = O, S ou deux atomes d'hydrogène,
R³⁴ = H, un groupe alkyle en C₁ à C₂₆, aryle, ar-(alkyle en C₁ à C₂₆), COOR³⁷, arylsulfonyle,
R³⁵ = H, un groupe acyle en C₁ à C₂₆, COOR³⁸
R³⁷, R³⁸ = indépendamment l'un de l'autre, un groupe alkyle en C₁ à C₂₆, aryle, ar-(alkyle en C₁ à C₂₆),
ou 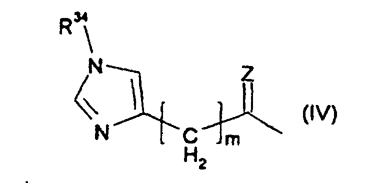 dans lequel
Z = O, S ou deux atomes d'hydrogène,
m = 0 à 3
R³⁴ = H, un groupe alkyle en C₁ à C₂₆, aryle, ar-(alkyle en C₁ à C₂₆), COOR³⁷, arylsulfonyle,
R³⁷ = un groupe alkyle en C₁ à C₂₆, aryle, ar-(alkyle en C₁ à C₂₆),
ou 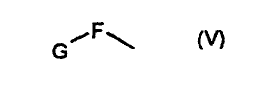 dans lequel
F = CH₂, CO, CS, SO₂,
G = COOR³⁹, CONHOH, CONR⁴⁰R⁴¹, CSNR⁴²R⁴³, un groupe alkyle substitué par un groupe alkyle ou aryle en C₁ à C₂₆ présentant 1 à 3 éléments dans la chaîne ou un groupe alkyle présentant 1 à 3 éléments dans la chaîne qui porte, sur l'atome de C terminal, un substituant choisi parmi COOR⁴⁴, CONHOH, CONR⁴⁵R⁴⁶, CSNR⁴⁷R⁴⁸, SR⁴⁹, SOR⁵⁰, SO₂R⁵¹, SO₂NR⁵²R⁵³, PO(OR⁵⁴)OR⁵⁵, PO(OR⁵⁶)NR⁵⁷², OSO₂R⁵⁸, O(PO)OR⁵⁹, NHSO₂R⁶⁰, NHPO₂R⁶¹, NHCOR⁶², NHCSR⁶³, NHCONR⁶⁴R⁶⁵, NHCSNR⁶⁶R⁶⁷, -S(NH)₂-R⁶⁸ ou NH (C=NR⁷⁰)NHR⁶⁹, ou encore un groupe aryle ou hétéroaryle,
R³⁹ à R⁶⁹ = indépendamment les uns des autres, H, un groupe alkyle en C₁ à C₂₆, aryle,
R⁷⁰ = CONH₂, SO₂NH₂, ou 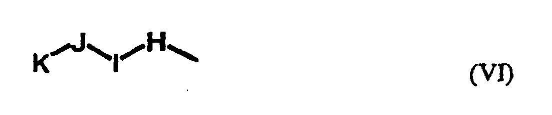
dans laquelle
H = CH₂, CO, CS, CHR⁷¹, CR⁷²R⁷³, SO₂, SO, PO₂,
I = un groupe alkylène en C₁ à C₂₆, un groupe alkylène en C₁ à C₂₆, dans lequel un groupe méthylène est remplacé par O, S, ou NR⁷⁷, ou un groupe alcénylène en C₂ à 26, ces groupes alkylène ou alcénylène n'étant pas substitués ou étant substitués par un groupe aryle, hétéroaryle, un atome d'halogène, OH, CN, un groupe alkyloxy en C₁ à C₉, aryloxy, COOR⁷⁴, CONR⁷⁵R⁷⁶, NR⁷⁷R⁷⁸, NH(C=NR⁷⁰)NHR⁶⁹, SR⁷⁹, SO₂R⁸⁰, ou encore un groupe cycloalkylène en C₃ à C₈, non substitué ou substitué par un groupe aryle, hétéroaryle, un atome d'halogène, OH, CN, alkyloxy en C₁ à C₉, aryloxy, COOR⁷⁴, CONR⁷⁵R⁷⁶, NR⁷⁷R⁷⁸, SR⁷⁹, SO₂R⁸⁰, cycloalkylène en C₃ à C₈, dans lequel la chaîne alkylène est interrompue par O, S ou NR⁷⁷, arylène, non substitué ou substitué par un groupe aryle, hétéroaryle, un atome d'halogène, OH, CN, alcoxy en C₁ à C₂₆, aryloxy, COOR⁷⁴, CONR⁷⁵R⁷⁶, NR⁷⁷R⁷⁸, SR⁷⁹, SO₂R⁸⁰,
R⁷¹ à R⁸⁰ = indépendamment les uns des autres, H, un groupe alkyle en C₁ à C₂₆, aryle, ar-(alkyle en C₁ à C₂₆), hétéroaryle,
J = une liaison ou CO, COO, CONR⁸¹, CS, CSNR⁸², SO, S(NH)₂, SO(NH), SO₂O, SO₂NR⁸³, PO(OR⁸⁴), PO(OR⁸⁵)NR⁸⁶, NR⁸⁷CO, NR⁸⁸CS, NR⁸⁹SO₂, OSO₂, NR⁹⁰PO(OR⁸⁴), OPO (OR⁹¹), PO(OR⁸⁴)O, NR⁴²CONR⁹³, NR94CSNR⁹⁵, NR⁹⁶SO₂NR⁹⁷, NR⁹⁸C(NR⁹⁹)NR¹⁰⁰
R⁸¹ à R¹⁰⁰ = indépendamment les uns des autres, H, un groupe alkyle en C₁ à C₂₆, aryle, ar-(alkyle en C₁ à C₂₆), hétéroaryle, R⁹⁹ = H, un groupe alkyle en C₁ à C₂₆, aryle, ar-(alkyle en C₁ à C₂₆), hétéroaryle, CONR¹⁰¹R¹⁰², CSNR¹⁰³R¹⁰⁴, SO₂NR¹⁰⁵R¹⁰⁶
R¹⁰¹ à R¹⁰⁶ = indépendamment les uns des autres, H, un groupe alkyle en C₁ à C₂₆, aryle, ar-(alkyle en C₁ à C₂₆), hétéroaryle,
K = un groupe alkyle en C₁₁ à C₂₃ ramifié ou non ramifié, un groupe alcényle en C₁₁ à C₂₃ ramifié ou non ramifié, lequel est non substitué ou substitué par un groupe aryle ou hétéroaryle, un groupe alcynyle en C₁₁ à C₂₃, aryle, hétéroaryle, ar-(alkyle en C₁ à C₂₆), les groupes aryle, hétéroaryle et ar-(alkyle en C₁ à C₂₆) pouvant être substitués par d'autres groupes aryle, hétéroaryle et/ou ar-(alkyle en C₁ à C₂₆), ou l'un de ses sels, pour la préparation de médicaments destinés au traitement thérapeutique et prophylactique d'infections.

2. Utilisation selon la revendication 1, **caractérisée en ce que**, pour les composés de formule (I), les variables ont la signification suivante :
n = 0 à 3,
R¹, R² et R³ = H,
A = CO, SO₂,
B = un groupe phényle, benzyle, phénéthyle, 4-chlorophénylméthyle, 4-bromophénylméthyle, 4-nitrophénylméthyle, 4-trifluorméthylphénylméthyle, 4-méthylphénylméthyle, 1-(4-méthyl-1-pipérazinyl)-1-(4-trifluorméthylphényl)méthyle, 1-(4-méthyl-1-pipérazinyl)-1-phénylméthyle, 4-phénylphénylméthyle, 1-naphtylméthyle, 2-naphtylméthyle, benzoyle, 2,4,4-triméthylpentyle, 2-carboxyéthyle, 3-carboxypropyle,
D = un groupe benzoyle,
Y = un groupe de formule (II),
dans laquelle
Z = O,
R³⁴ = H, un groupe benzyloxycarbonyle, trytile,
R³⁵ = H, un groupe benzyloxycarbonyle, tert-butyloxycarbonyle,
R³⁶ = H,
ou un groupe de formule (III),
dans laquelle
Z = O,
R³⁴ = H, un groupe tosyle, benzyle, 4-nitrobenzyle, 4-cyanobenzyle, benzyloxycarbonyle,
R³⁵ = H, un groupe benzyloxycarbonyle, tert-butyloxycarbonyle,
ou un groupe de formule (IV),
dans laquelle
Z = O,
m = 0 à 3,
R³⁴ = H, un groupe tosyle, benzyle, 4-nitrobenzyle, 4-cyanobenzyle, benzyloxycarbonyle,
ou un groupe de formule (V),
dans laquelle
F = CO, CH₂, SO₂,
G = un groupe COOH, COOMe, CONHOH, CH₂-COOH, CH₂COOMe, CH₂CONHOH, CH₂CONH-(alkyle en C₁₄ à C₂₀), CH₂CH₂COOH, CH₂CH₂COOMe, CH₂CH₂CONHOH, CH₂CH₂CONH-(alkyle en C₁₄ à C₂₀), CH₂CH₂CH₂COOH, CH₂CH₂CH₂COOMe, CH₂CH₂CH₂CONHOH, CH₂CH₂CH₂CONH-(alkyle en C₁₄ à C₂₀), phényle, naphtyle, pyridyle, fluorényle, anthracényle,
ou un groupe de formule (VI),
dans laquelle
H = CO, SO₂,
I = un groupe méthylène, 1,2-éthylène, 1,3-triméthylène, 1,4-tétraméthylène, -CH₂-S-CH₂-, -CH₂-O-CH₂-, -CH₂-NH-CH₂-, 1,2-éthénylène, 1,1-éthylène, prop-1-én-1,3-ylène, prop-2-én-1,3-ylène, benzylène, 2-phényl-1,1-éthylène, carboxyméthylène, aminocarbonylméthylène, 2-carboxy-1,1-éthylène, 2-aminocarbonyl-1,1-éthylène, 3-carboxy-1,1-propylène, 3-aminocarbonyl-1,1-propylène, 2-méthyl-1,1-propylène, 3-méthyl-1,1-butylène, 2-méthyl-1,1-butylène, pyrrolidine liée par l'intermédiaire de N₁ et C₂, 1,2-phénylène, 1,3-phénylène, 1,2-naphtylène, 1,3-naphtylène, 1,1-Cyclopentylène, 1,1-cyclohexylène, 1,2-cyclohexylène,
J = une liaison, CO, CS, SO₂, PO(OMe), PO(OH), CONH, CSNH, SO₂NH, PO (OH) O, PO (OH) NH, PO(OMe)O, PO(OMe)NH,
K = un groupe alkyle en C₁₃ à C₁₉, alcényle en C₁₃ à C₁₉, 4-benzyloxystyryle, 4-styrylstyryle, 4-phénylstyryle, 4-cyanostyryle, 4-nitrostyryle, phényle, 4-biphénylyle, 4-nitrophényle, 4-cyanophényle, 4-méthylsulfonylphényle, 4-méthoxyphényle, 4-bromophényle, 4-chlorophényle, 4-trifluorméthylphényle, 4-formylphényle, 4-méthoxycarbonylphényle, 4-(1,1-dicyano-2-vinyl)phényle, 4-aminophényle, 4-éthylphényle, 4-isopropylphényle, 4-tert-butylphényle, 4-éthoxyphényle, 4-propoxyphényle, 4-butoxyphényle, 4-benzyloxyphényle, 3,4-bis-(benzyloxy)phényle, 3-phénoxyphényle, 4-styrylphényle, 1-naphtyle, 2-naphtyle, 2-fluorényle, 2-(2-phénylthiazol-4-yle), 5-(4-nitrophényl)thiazo-4-yle), 5-(4-nitrophényl)-furan-2-yle), 5-(3-nitrophényl)furan-2-yle), 5-(2-nitrophényl)furan-2-yle), 5-(4-bromophényl)furan-2-yle), 5-(4-chlorophényl)furan-2-yle), 5-(3-trifluorméthylphényl)-furan-2-yle), 5-(4-trifluorméthylphényl)furan-2-yle), 5-(2-chloro-3-trifluorméthylphényl)-furan-2-yle) 3,4-méthylènedioxyphényle, 1-acétylindol-3-yle, 4'-nitrobiphénylyle, 5-(4-bromophényl)thiophén-2-yle), 5-(4-méthylphényl)furan-2-yle), 5-(4-méthoxyphényl)furan-2-yle), 5-bromothiophén-2-yle, 5-bromofuran-2-yle, 4-pyridyle, 3-pyridyle, 2-pyridyle, quinoléin-2-yle, 1-naphtylvinyle, 2-naphtylvinyle, 2-fluorényl-vinyle, 2-(2-phénylthiazol-4-yl)vinyle, 2-[5-(4-nitrophényl)furan-2-yl)vinyle, 2-[5-(4-acétoxy-méthylphényl)furan-2-yl)vinyle, 2-[5-(3-trifluorméthylphényl)furan-2-yl)vinyle, 4-benzyloxy-styryle, 3,4-dibenzyloxystyryle, 3-méthoxy-4-(4-nitrobenzyloxy)styryle, 2-méthylindol-3-ylvinyle, 1-acétylindol-3-ylvinyle, 3,4-méthylènedioxystyryle, 4-(1,1-dicyano-2-vinyl)styryle, et leurs sels.

3. Utilisation selon la revendication 1, **caractérisée en ce que**, pour les composés de formule (I), les variables ont la signification suivante :
n = 0 à 3,
R¹,R² = H,
R³ = H,
A = CO, SO₂,
B = un groupe phényle, benzyle, phénéthyle, 4-chlorophénylméthyle, 4-méthylphénylméthyle, 1-(4-méthyl-1-pipérazinyl)-1-(4-trifluorméthylphényl)méthyle, 1-(4-méthyl-1-pipérazinyl)-1-phénylméthyle, 4-bromophénylméthyle, 4-nitrophénylméthyle, 4-trifluorméthylphénylméthyle, 4-phénylphényl-méthyle, 1-naphtylméthyle, 2-naphtylméthyle, benzoyle, 2,4,4-triméthylpentyle, 2-carboxyéthyle, 3-carboxypropyle,
D = un groupe benzoyle,
Y = un groupe de formule (IV),
dans laquelle
Z = O,
m = 0 à 3,
R³⁴ = H, un groupe benzyle, 4-nitrobenzyle, 4-cyanobenzyle,
ou un groupe de formule (V),
dans laquelle
F = CO, CH₂, SO₂,
G = un groupe CH₂CH₂CONH-(alkyle en C₁₄ à C₁₈), CH₂CH₂CH₂CONH- (alkyle en C₁₄ à C₁₈), phényle, naphtyle, pyridyle, fluorényle, anthracényle
ou un groupe de formule (VI),
H = CO,
I = méthylène, 1,2-éthylène, 1,3-triméthylène, 1,4-tétraméthylène, -CH₂-S-CH₂-, -CH₂-O-CH₂-, -CH₂-NH-CH₂-, 1,2-éthénylène, 1,1-éthylène, prop-1-én-1,3-ylène, prop-2-én-1,3-ylène, benzylène, 2-phényl-1,1-éthylène, 2-méthyl-1,1-propylène, 3-méthyl-1,1-butylène, 2-méthyl-1,1-butylène, pyrrolidine liée par l'intermédiaire de N₁ et C₂,
J = PO(OMe), PO(OH), CONH, CSNH, SO₂NH, PO(OH)O, PO(OH)NH, PO (OMe) O, PO (OMe) NH,
K = un groupe alkyle en C₁₃ à C₁₉, alcényle en C₁₃ à C₁₉, 4-benzyloxystyryle, 4-phénylstyryle, 4-cyanostyryle, 4-nitrostyryle, phényle, 4-biphénylyle, 4-nitrophényle, 4-cyanophényle, 4-méthoxyphényle, 1-naphtylvinyle, 2-naphtylvinyle, 2-fluorénylvinyle, 2-(2-phénylthiazol-4-yl)vinyle, 2-[5-(4-nitrophényl)furan-2-yl)vinyle, 2-[5-(4-acétoxyméthylphényl)furan-2-yl)vinyle, 2-[5-(3-trifluorméthylphényl)furan-2-yl)vinyle, 3,4-dibenzyloxystyryle, 3-méthoxy-4-(4-nitrobenzyloxy)styryle, 3,4-méthylènedioxystyryle, 4-(1,1-dicyano-2-vinyl)styryle, et leurs sels.

4. Utilisation selon l'une quelconque des revendications 1 à 4, **caractérisée en ce que** A représente CO et B représente un groupe benzyle ou 4-méthylphénylméthyle et D représente un groupe benzoyle.

5. Utilisation selon la revendication 4, **caractérisée en ce que** les composés de formule (I) sont choisis dans le groupe formé par

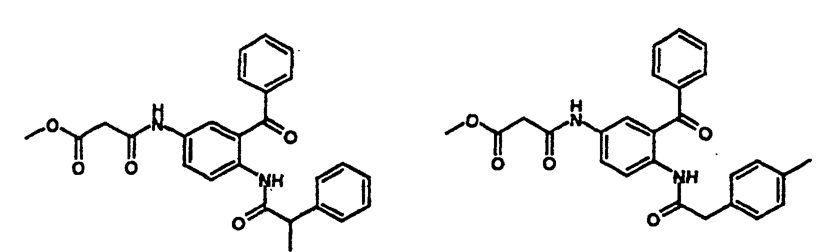

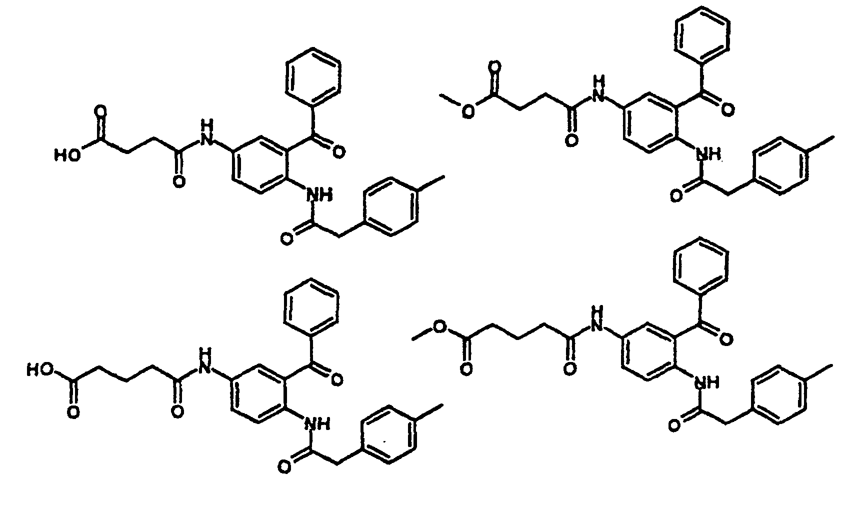

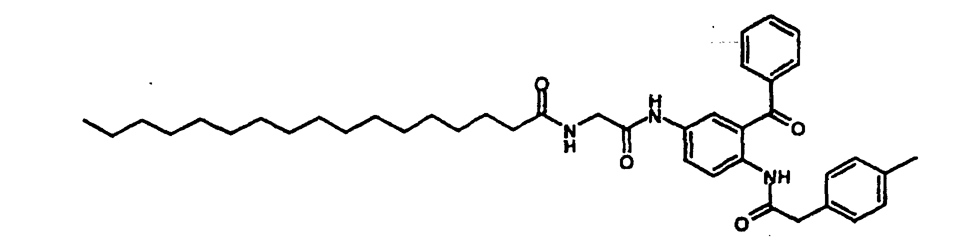

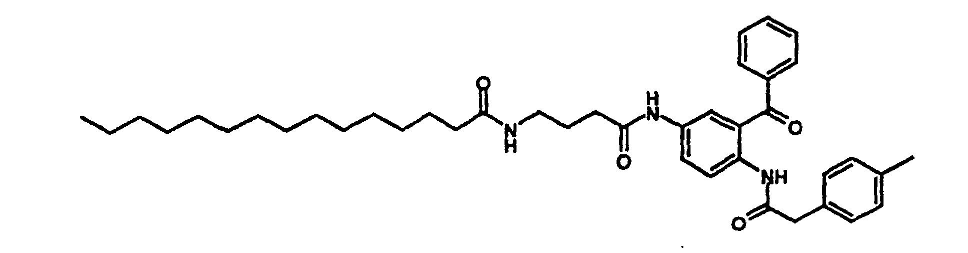

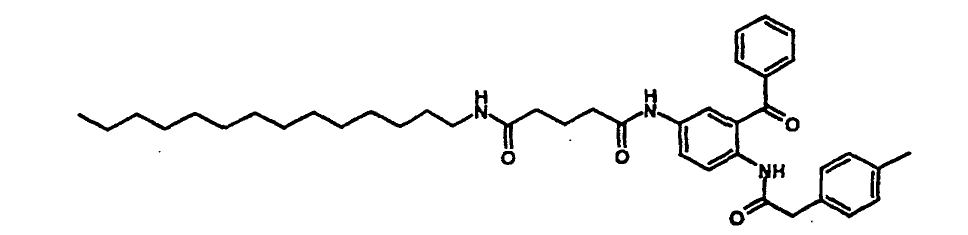

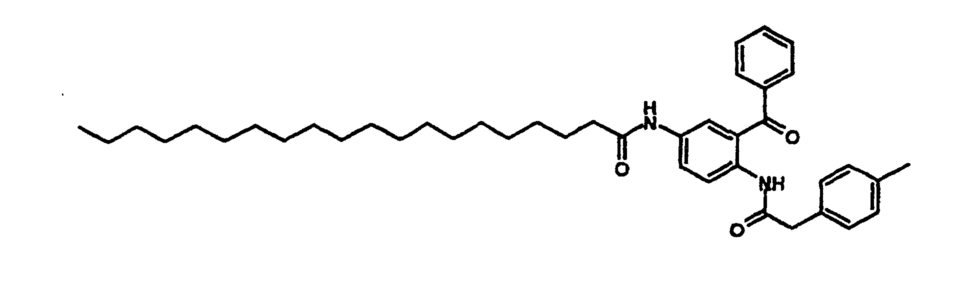

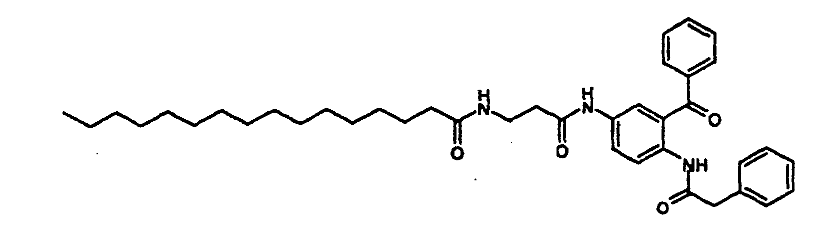

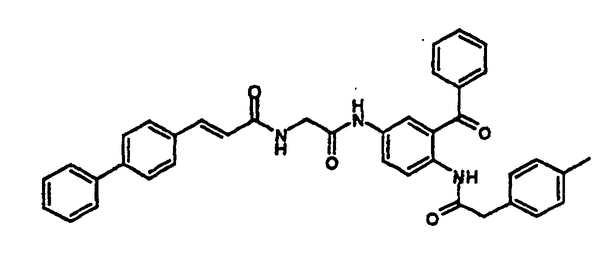

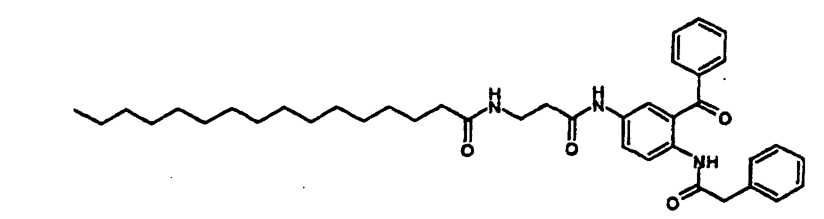

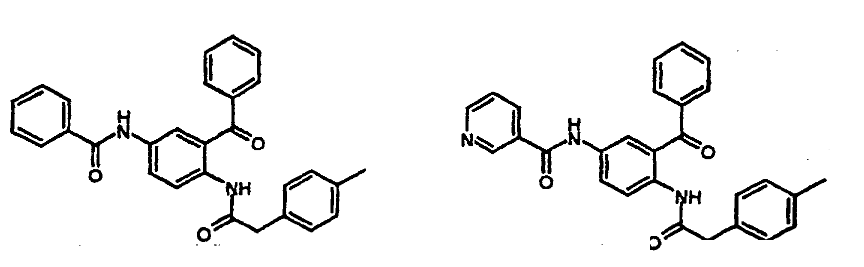

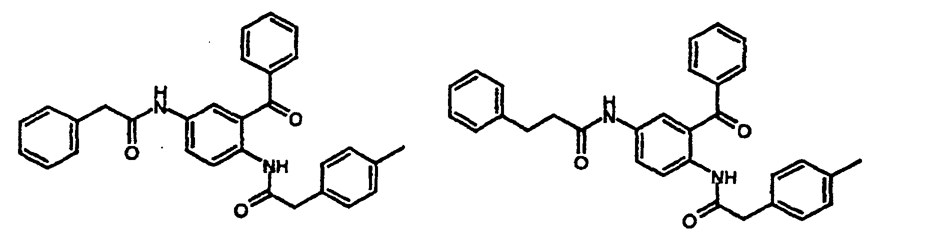

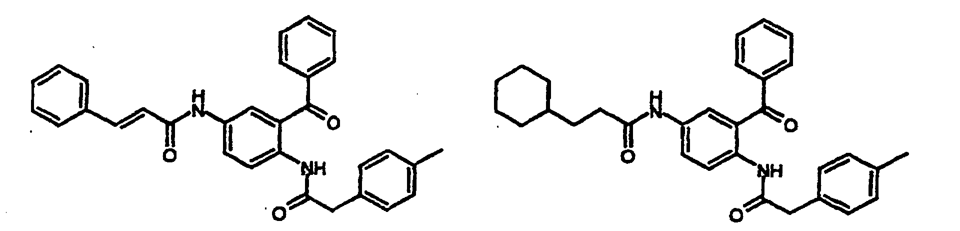

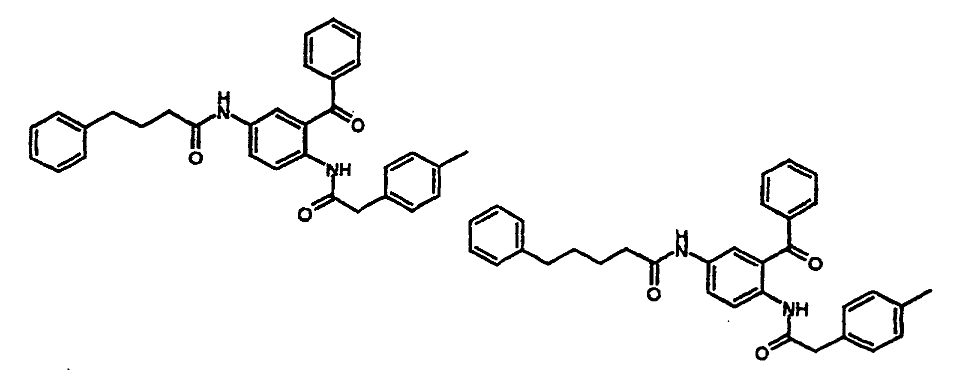

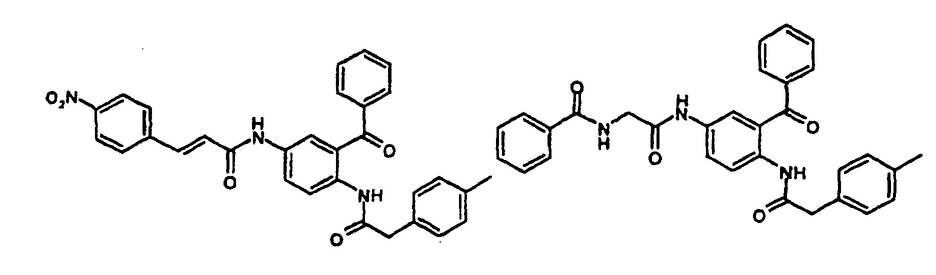

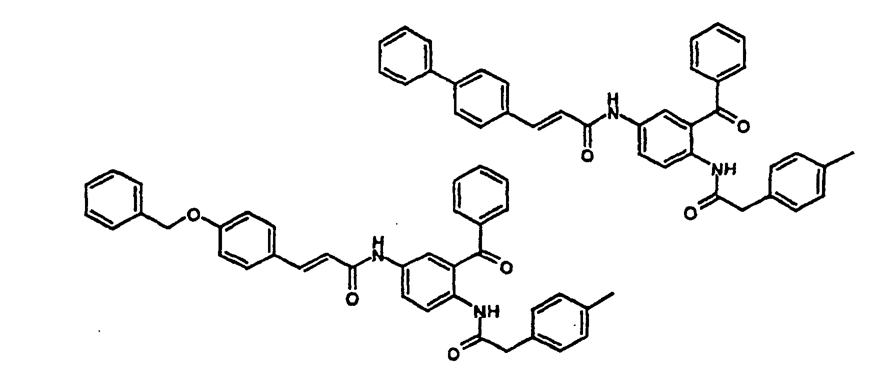

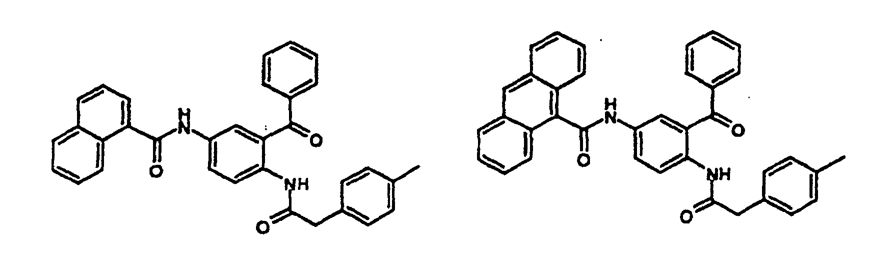

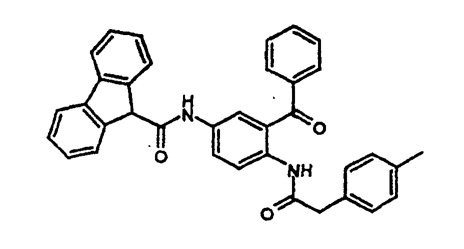

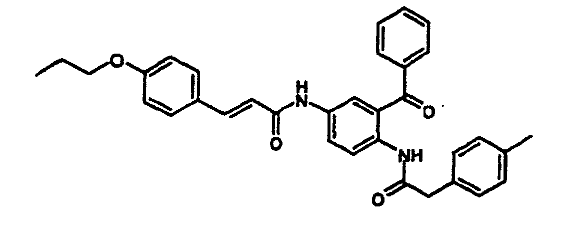

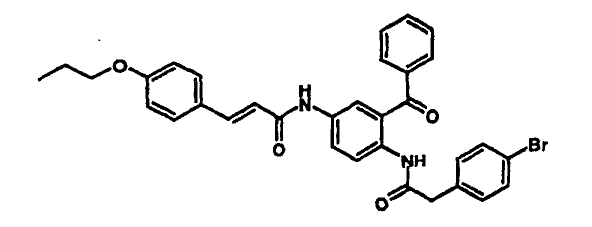

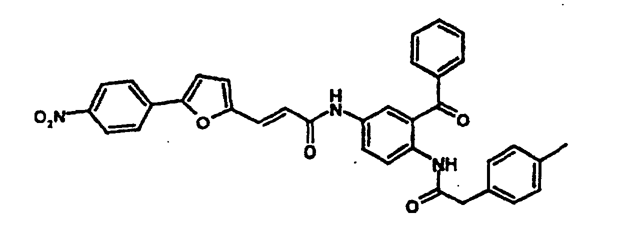

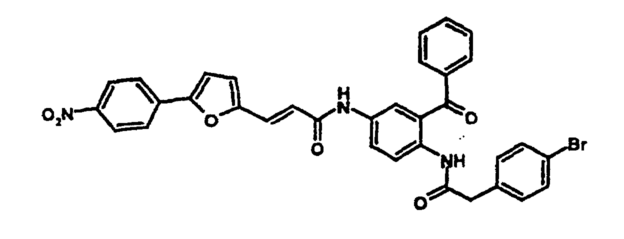

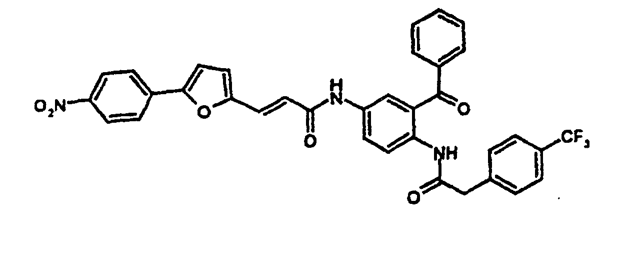

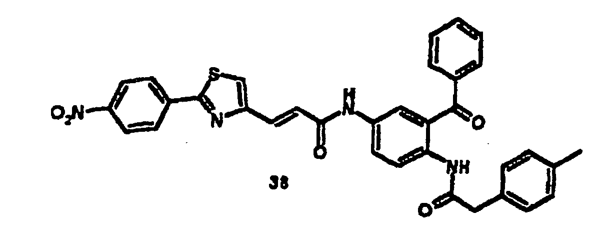

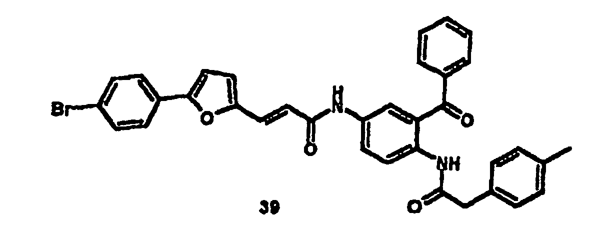

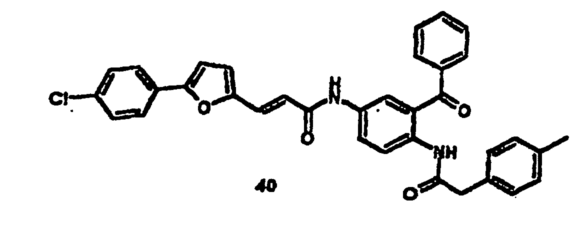

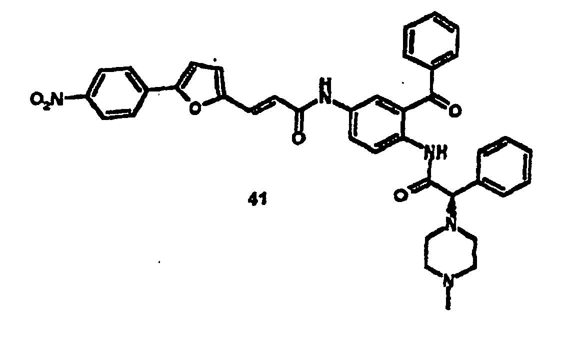

6. Utilisation selon la revendication 1, **caractérisée en ce que** les composés de formule (I) sont choisis dans le groupe formé par l'amide de l'acide N-[3-[3-benzoyl-4-[2-(4-méthylphényl)-acétyl]amino]-phénylamino]-4-phénylcinnamique et l'amide de l'acide N-[2-[3-benzoyl-4-[[2-(4-méthylphényl)acétyl]-amino]phényl]-4-benzyloxycinnamique.

7. Utilisation selon l'une quelconque des revendications 1 à 6 pour la préparation de médicaments destinés à la prophylaxie et au traitement d'infections causées par des parasites, en particulier par des parasites unicellulaires, à savoir les agents pathogènes de la malaria, de l'encéphalite épidémique, de la maladie de Chagas, de la toxoplasmose, de l'amibiase, de la leishmaniose, de la trichomonase, de la pneumocystose, de la balantidiase, de la cryptosporidiose, de la sarcocystose, de l'acanthamoebose, de la naegleriase, de la coccidiose, de la giardiose et de la lambliase.

8. Médicament destiné au traitement thérapeutique et prophylactique de processus infectieux, **caractérisé en ce que** la préparation contient une teneur efficace en au moins un composé selon l'une quelconque des revendications 1 bis 6 conjointement avec un véhicule acceptable sur le plan pharmaceutique.

9. Médicament selon la revendication 8, **caractérisé en ce que** la préparation contient un autre principe actif pharmaceutique.
